# EUROPEAN PATENT APPLICATION

(11) **EP 1 736 539 A1**
(43) Date of publication of application: **27.12.2006**
(21) Application number: 05013727.2
(22) Date of filing: 24.06.2005
(51) Int. Cl.: C12N 7/04, A61K 39/215, A61P 11/00

(54) **Attenuated SARS-CoV vaccines**

(71) Applicant: CONSEJO SUPERIOR DE INVESTIGACIONES CIENTIFICAS, 28006 Madrid (ES)
(72) Inventor: Álvarez Gómez, Enrique, 28035 Madrid (ES); López de Diego, Marta, 28700 San Sebastián de los Reyes, Madrid (ES); Enjuanes Sanches, Luis, 28109 Madrid (ES); Almazán Toral, Fernando, 28041 Madrid (ES)
(74) Representative: UEXKÜLL & STOLBERG

(57) **Abstract**

The present invention relates to nucleic acids encoding attenuated SARS-CoV viruses which are capable of producing a maximum viral titer in cell culture that is reduced at least by a factor of 2 when compared to the maximum viral titer of wild-type SARS-CoV virus in the same cell culture.

According to a further aspect of the present invention, the nucleic acids encoding an attenuated SARS-CoV virus, are obtainable by a method comprising steps, wherein the genome of a SARS-CoV virus is modified by amending the sequence of the gene encoding the SARS-CoV E protein so that the nucleic acid cannot express a functional E protein.

The present invention further relates to the viruses encoded by these nucleic acids as well as the medical use of the nucleic acids and of the viruses.

## Description

The present invention is directed to nucleic acids encoding an attenuated SARS-CoV virus capable of producing a maximum viral titer in cell culture that is reduced when compared to the maximum viral titer of wild-type SARS-CoV virus in the same cell culture.

### TECHNICAL BACKRGOUND

Therapy approaches that involve the insertion of a functional gene into a cell to achieve a therapeutic effect are also referred to as gene therapy approaches, as the gene serves as a drug. Gene therapy is a technique primarily for correcting defective genes responsible for disease development.

A carrier molecule also referred to as a vector is used to deliver the therapeutic gene to the patient's target cells. Currently, the most common vector is a virus that has been genetically altered to carry human or animal genes. Viruses naturally have evolved in a way of encapsulating and delivering their genes to human and animal cells in a pathogenic manner. However, in the meantime scientists have taken advantage of this capability and manipulated the virus genome to remove disease-causing genes and insert therapeutic genes.

Target cells such as the patient's liver or lung cells are infected with the viral vector. The vector then unloads its genetic material containing the therapeutic gene into the target cell. The generation of a functional protein product from the therapeutic gene restores the target cell to a normal state.

In an alternative approach, these viral vectors were used for expressing heterologous genes that cause an immunogenic response in the subject receiving the vector and thus immunize that subject. In that case the viral vector serves as a vaccine.

Since the etiologic pathogen causing SARS was identified as a new coronavirus (SARS-CoV; see Drosten et al., 2003; Holmes and Enjuanes, 2003; Marra et al., 2003; Rota et al., 2003), the study of coronavirus molecular biology was given a very high priority in order to develop effective strategies to prevent and control coronavirus infections.

Coronaviruses are ssRNA(+) viruses which have the largest genome so far found in RNA viruses with a length between 25 and 31 kilobases (kb; see Siddell S.G. 1995, The Coronaviridae). When a coronavirus infects a cell, the genomic RNA (gRNA) replicates in the cytoplasm and a set of subgenomic RNAs (sgRNA) of positive and negative polarity is produced (Sethna et al., 1989; Sawicki & Sawicki, 1990; and Van der Most and Spaan, The Coronaviridae).

Due to the fact that the coronaviruses replicate in the cytoplasm, use of coronaviruses as a vector for gene therapy and vaccination has been suggested. Specifically, defective interfering (DI) genomes of coronaviruses were produced. These DI genomes are deletion mutants which require the presence of a complementing or helper virus for replication and/or transcription (see Chang et al., 1994; WO97/34008; Spanish patent application P9600620; Izeta et al., 1999; and Sánchez et al., 1999).

A respective system was used in the art to generate immune responses in an animal which received a composition containing a DI genome which amongst others contained sequences encoding a heterologous reporter gene or a gene derived from a different infectious agent (porcine reproductive and respiratory disease virus, PRRSV; see Alonso et al., 2002a, 2002b).

The construction of coronavirus full-length cDNA clones (Almazán et al., 2000; Casais et al., 2001; Thiel et al., 2001; Yount et al., 2000; Yount et al., 2002; Yount et al., 2003) provided the opportunity for the genetic manipulation of coronavirus genomes to study fundamental viral processes and to develop expression vectors.

Although SARS-CoV emerged only in 2002, genome sequences of SARS-CoV isolates have been published recently and provide important information on the organization, phylogeny and variability of the SARS-CoV genome (Marra et al., 2003; Rota et al., 2003). About two-thirds of the 29.7-kb Urbani strain genome encode the replicase gene that comprises open reading frames Rep 1a and Rep 1b, the latter one being expressed by ribosomal frameshifting (Thiel et al., 2003). Translation of both ORFs results in the synthesis of two large polyproteins that are processed by viral proteinases to yield the replicase-transcriptase complex (Ziebuhr et al., 2000).

The 3' one-third of the genome includes the genes encoding the structural and non-structural proteins, in the order 5'-S-3a-3b-E-M-6-7a-7b-8a-8b-9b-N-3'. These proteins are expressed by a discontinuous transcription process that most probably takes place during the synthesis of the negative strand, leading to the generation of a 3' coterminal nested set of subgenomic mRNAs, each of which has at its 5' end a capped leader sequence derived from the 5' end of the genome (Sawicki and Sawicki, 1998; Zúñiga et al., 2004). Synthesis of subgenomic negative sense RNA species is regulated by the transcription-regulating sequences (TRSs), that include a highly conserved core sequence that is found preceding each gene and at the 3' end of the leader sequence (Thiel et al., 2003).

The preparation of a full-length cDNA of the SARS-CoV urbani strain has been described previously (Yount et al., 2003) where it was shown that the recombinant virus replicated as efficiently as wild-type virus. The production of SARS-CoV-like particles, by expression of the viral structural proteins M, E and S in the baculovirus system was also described (Mortola & Roy, 2004). It was shown that the simultaneous high level expression of S, E and M by a single recombinant virus allowed the assembly and release of VLPs. WO 04/092360 discloses nucleic acids and proteins from the SARS coronavirus which can be used in the preparation and manufacture of vaccine formulations, diagnostic reagents and kits.

SARS-CoV first broke out in humans in late 2002 and spread within a few months from its origin in Guangdong (China) to more than 30 countries. The rapid transmission and high mortality rate made SARS-CoV a global threat for which no efficacious therapy is available. The problem underlying the present invention thus resides in providing vaccines for protection against SARS-CoV with good safety and immunogenicity.

### SUMMARY OF THE INVENTION

This problem is now solved by nucleic acids encoding attenuated SARS-CoV viruses. According to one aspect of the present invention, the attenuated virus is capable of producing a maximum viral titer in cell culture that is reduced at least by a factor of 2 when compared to the maximum viral titer of wild-type SARS-CoV virus in the same cell culture.

According to a further aspect of the present invention, the nucleic acids encoding an attenuated SARS-CoV virus, are obtainable by a method comprising steps, wherein the genome of a SARS-CoV virus is modified by amending the sequence of the gene encoding the SARS-CoV E protein so that the nucleic acid cannot express a functional E protein. In the context of the present invention a functional E protein is a protein that:
- is encoded by SEQ ID NO: 6 or encoded by a gene having a homology of at least 70%, preferably at least 85% or at least 95% to the sequence of SEQ ID NO: 6; and
- is capable to associate with other SARS-CoV proteins into a viral envelope.

A nucleic acid encoding a modified E protein as described above will generate a virus particle that does not contain an E protein in its envelope.

The present invention further is directed to nucleic acids encoding an attenuated SARS-CoV virus, wherein the nucleic acid does not encode a sequence of a functional protein E of the SARS-CoV virus as described above. In accordance with this embodiment, the nucleic acids may encode an attenuated SARS-CoV virus, and comprise the sequences encoding the viral proteins replicase and S and M and N. The nucleic acid may contain other SARS derived proteins, such as the 3a protein.

In one aspect of the present invention, the nucleic acids as defined above, do not contain any other SARS derived proteins. The nucleic acids preferably are not capable of expressing a protein, which protein is encoded by:
- any of SEQ ID NOs: 13, 14, 15, 16, 17, 18, 19; or
- or any sequence having a homology of at least 70%, preferably at least 85% or at least 95% to the sequence of SEQ ID NOs: 13, 14, 15, 16, 17, 18, 19.
   In an alternative embodiment, the nucleic acids preferably are not capable of expressing a protein, which protein is encoded by:

- any of SEQ ID NOs: 12, 13, 14, 15, 16, 17, 18, 19; or
- or any sequence having a homology of at least 70%, preferably at least 85% or at least 95% to the sequence of SEQ ID NOs: 12, 13, 14, 15, 16, 17, 18, 19.

According to a further aspect, the nucleic acids of the present invention encode an attenuated SARS-CoV virus, wherein the nucleic acid comprises the sequences of the viral proteins replicase and S and M and N and/or E. Again, the nucleic acid may contain other SARS derived proteins but preferably does not contain any other SARS derived proteins as defined above.

The nucleic acid may be RNA or DNA.

The present invention further relates to host cells and SARS-CoV virus particles comprising one of the above nucleic acids. The SARS-CoV virus particle may for example be obtainable by introducing one of the above nucleic acids into a host cell.

The present invention further relates to vaccines comprising a respective SARS-CoV virus particle or a respective nucleic acid or a respective host cell. In the vaccine the virus particle may be present in an attenuated form, i.e. replication competent and infectious, but may also present in an inactivated form. Inactivating the attenuated virus using heat or chemicals according to well known methods for viral inactivation will further increase the safety of the use of the vaccine. The vaccine may further comprise a pharmaceutically acceptable adjuvans, carrier or excipient. The vaccine is preferably used vaccinate a mammal, preferably a human, and gives rise to an immune response that reduces or eliminates the disease symptoms caused by infection with the SARS-CoV virus.

In a further aspect the present invention relates to methods for preparing a SARS-CoV vaccine, comprising modifying a nucleic acid comprising the genome of a SARS-CoV virus by amending the sequence of the gene encoding the SARS-CoV E protein so that the gene cannot express a functional E protein.

Further preferred embodiments of the present invention are described in the following detailed description, in the examples and in the claims.

### DETAILED DESCRIPTION OF THE INVENTION

The present invention relates to nucleic acids encoding attenuated SARS-CoV viruses and respective viruses as well as their medical use. The attenuated virus is preferably capable of producing a maximum viral titer in cell culture that is reduced at least by a factor of 2 when compared to the maximum viral titer of wild-type SARS-CoV virus in the same cell culture.

According to the most preferred embodiment of the present invention, the nucleic acids do not encode a sequence of a functional protein E of the SARS-CoV virus. The nucleic acids are for example obtainable by a method comprising steps, wherein the genome of a SARS-CoV virus is modified by amending the sequence of the gene encoding the SARS-CoV E protein so that the nucleic acid cannot express a functional E protein. The modifications may include a deletion and/or a substitution of the coding sequences or of the regulatory sequences. It is for example possible but may not be necessary to remove the entire coding sequence of the E protein.

According to a preferred aspect of the present invention, the modification of the E gene comprises the preparation of a SARS-CoV replicon or a full length cDNA clone of SARS-CoV in a BAC and the subsequent modification of that BAC. This has the specific advantage that the modifications of the SARS-CoV genome can be carried out very conveniently.

The present inventors have surprisingly found that a vaccine comprising a nucleic acid of the present invention is safe and can initiate an immune response against SARS-CoV viruses. The vaccine comprises a nucleic acid or an attenuated virus particle, which contains all elements necessary to infect human or animal cells and which may have been inactivated or not.

Using the attenuated virus as a vaccine, infection of a cell, will lead to replication of the virus and production of further infective virus particles. However, the grwoth rate will be significantly reduced when compared to the wild-type SARS virus. In other words, the vaccine strain will only be able to grow slowly in a vaccinated host. The immune system of the host will produce an immune response directed against the virus which reduces or eliminates the disease symptoms caused by infection with a wild-type SARS-CoV.

In an alternative embodiment, the attenuated virus is obtained by expression of the nucleic acids of the present invention and subsequently inactivated using well known methods for virus inactivation, such as chemical or heat treatment. This embodiment has the specific advantage that the virus is disarmed twice and thus safer than the vaccines previously suggested in this field.

In the present application the term "attenuated" is used to refer to replication competent and infectious viruses, which viruses produce a reduced maximum viral titer in a cell culture when compared to the maximum viral titer produced by the wild-type SARS-CoV virus in a culture of the same cells under the same conditions (temperature, medium, incubation titer, incubation time, etc.). An attenuated virus may thus have an increased generation time or be otherwise inhibited in growth when compared to a wild-type virus.

The term "replication competent viral nucleic acid" is used to refer to a nucleic acid comprising sequences encoding a viral replicase and sequences which represent an origin of replication, i.e. sequences which will trigger replication of the nucleic acid by the replicase.

The term "infectious" is used to refer to a virus which is cable to grow by infection of cells, replication of the viral nucleic acid, expression of viral proteins, assembly of the viral nucleic acid with the viral proteins to a virus particle and infection of further cells by the newly produced virus particles.

The term "nucleic acid which encodes an attenuated virus" is used to refer to a nucleic acid which encodes all sequences of an attenuated virus. Respective nucleic acids can be used to obtain virus particles by transfecting a cell culture which will lead to growth of the attenuated virus in the culture. The nucleic acid can also be used as a vaccine.

SARS-CoV coat proteins are proteins, such as the SARS-CoV E, M or S (spike) protein or proteins. The proteins may have a sequence as observed in a SARS-CoV isolate or may be derived therefrom by methods known in the art. Using methods of recombinant expression it is for example possible to modify protein sequences by addition, deletion or substitution of one or several amino acids. The present invention thus covers viruses comprising or encoding coat proteins with a sequence homology of at least 60%, preferably at least 75% and most preferably at least 95% to the wild type SARS-CoV proteins.

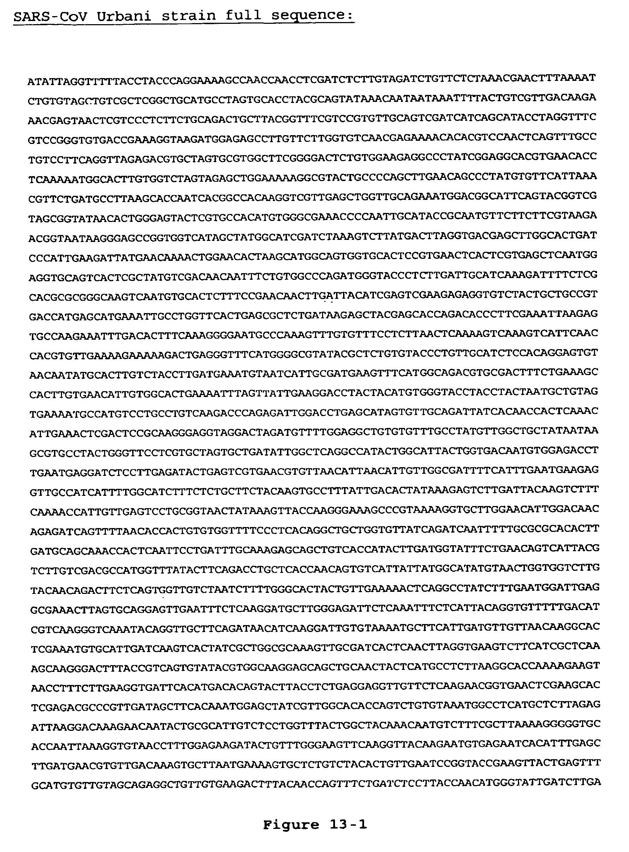

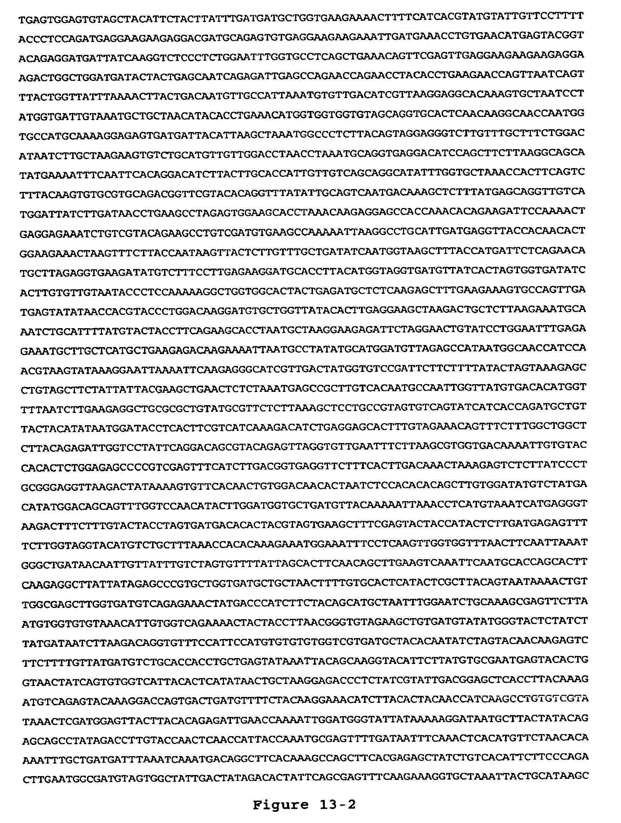

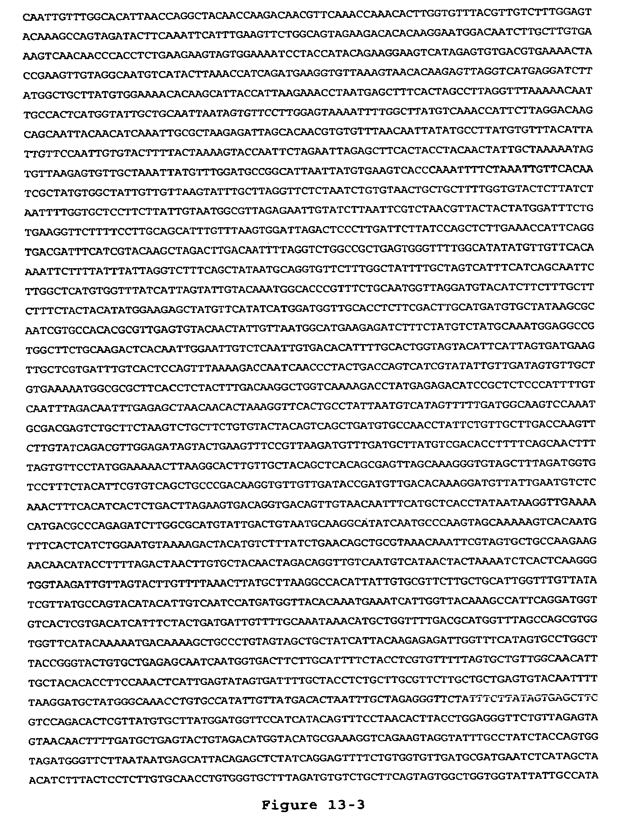

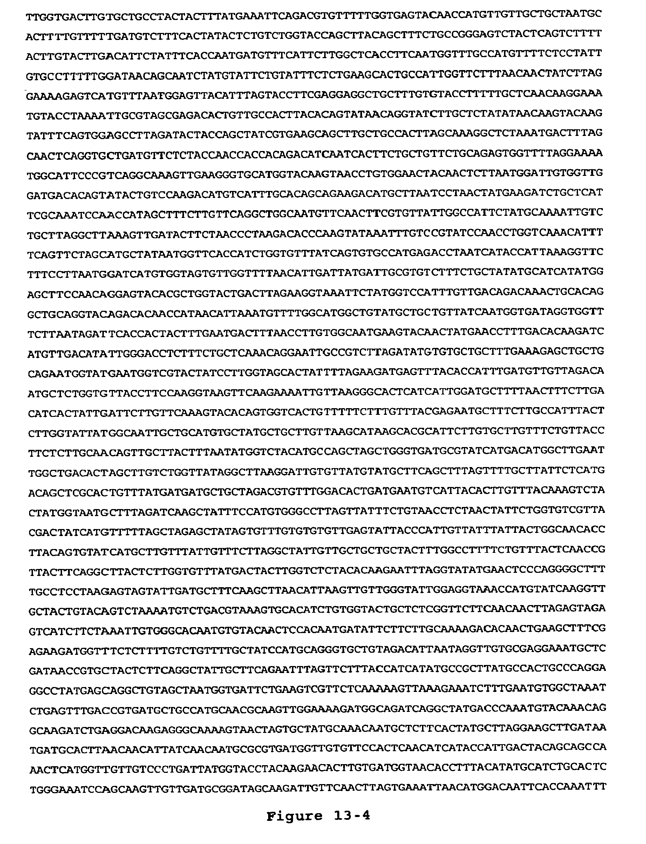

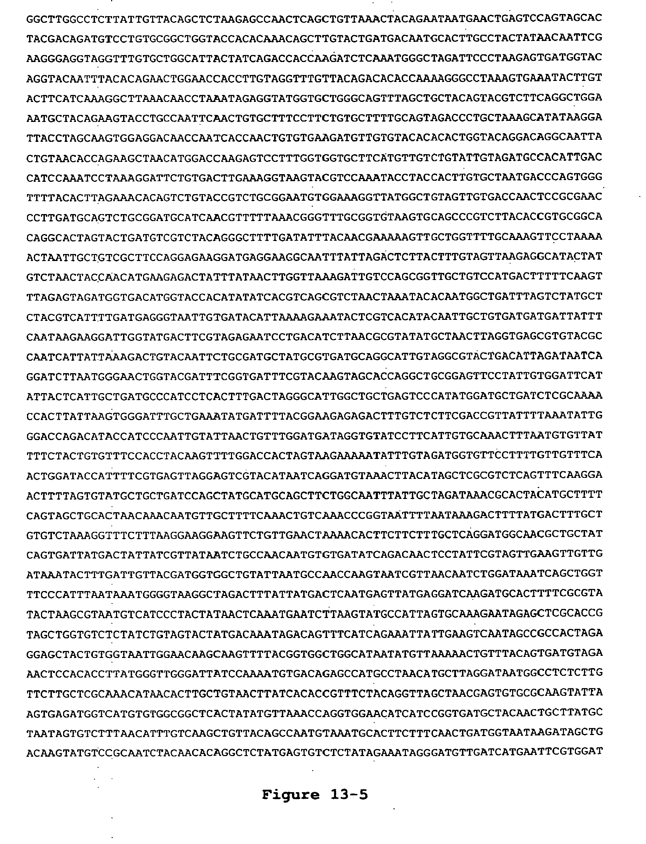

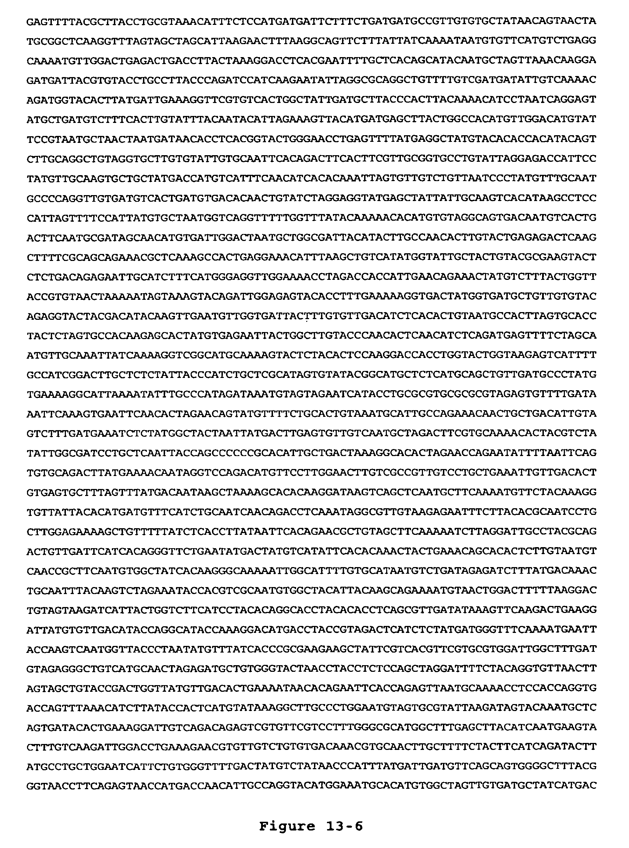

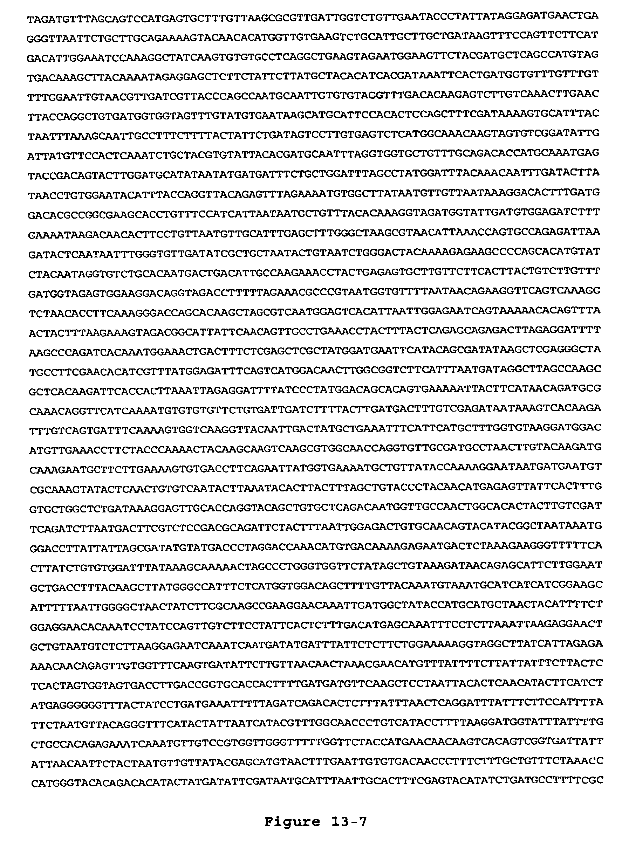

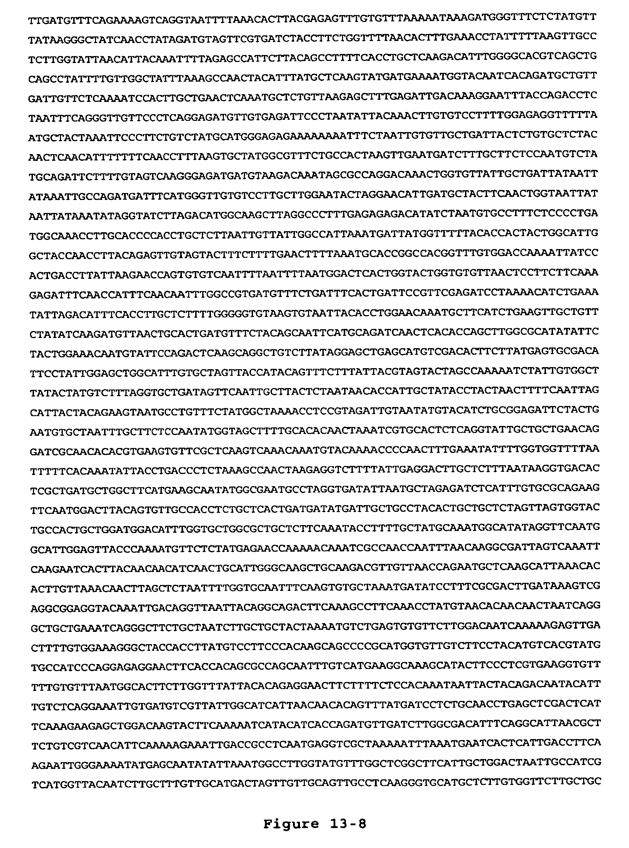

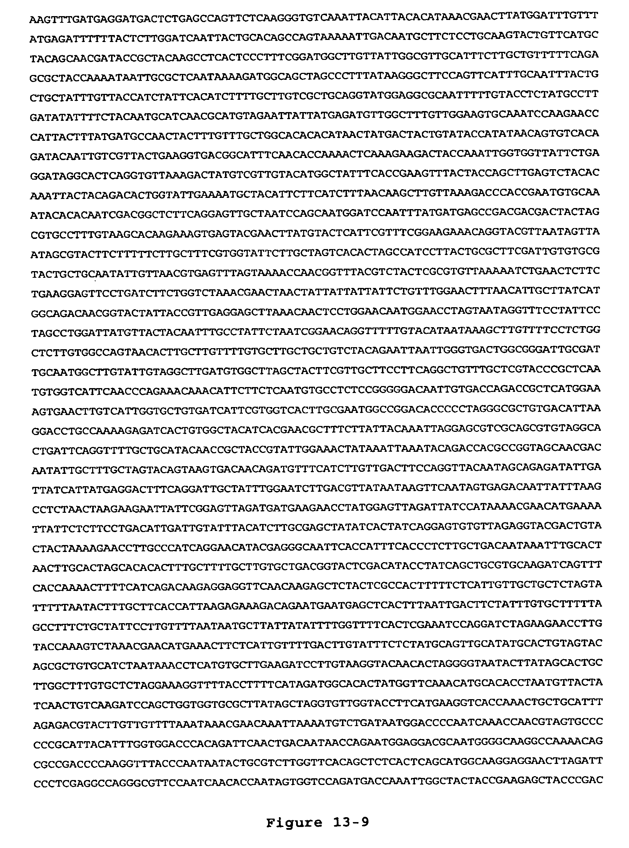

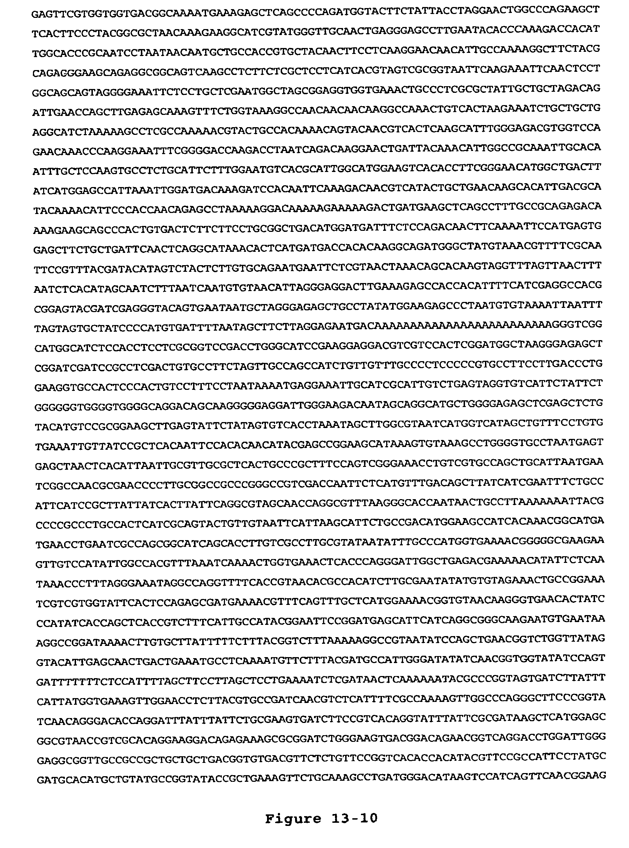

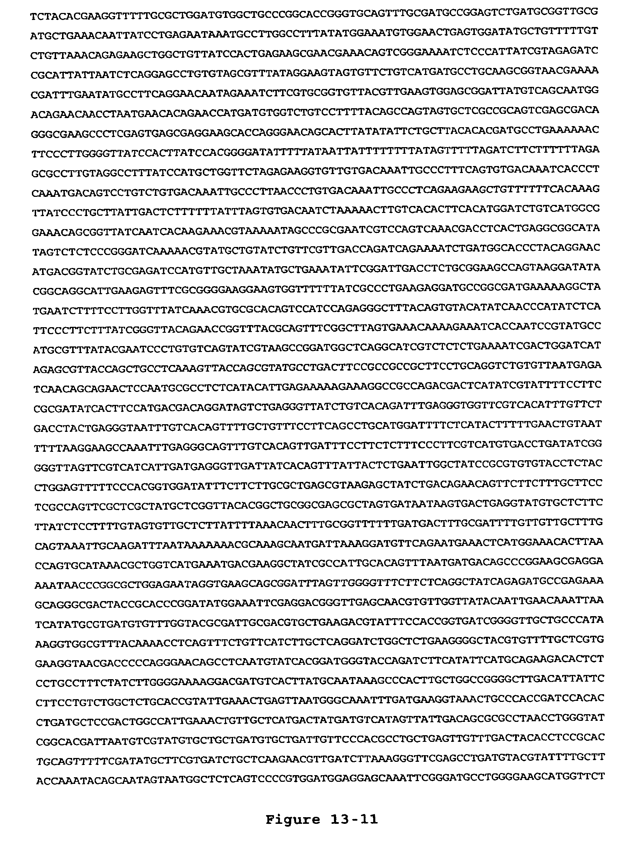

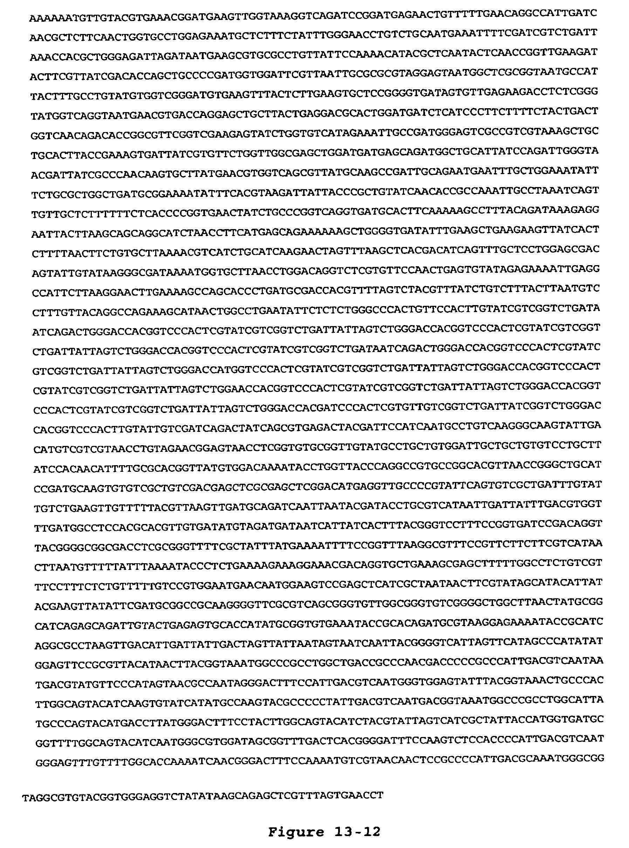

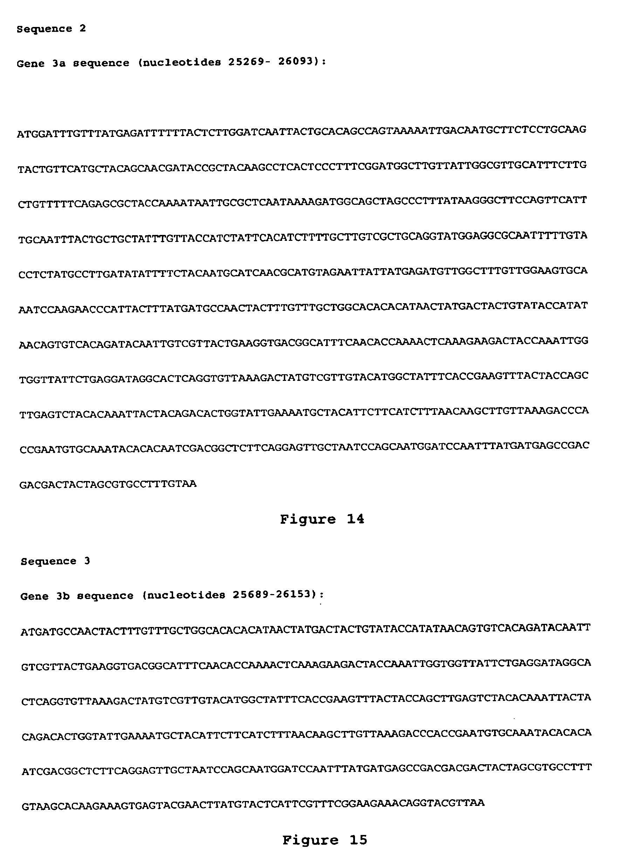

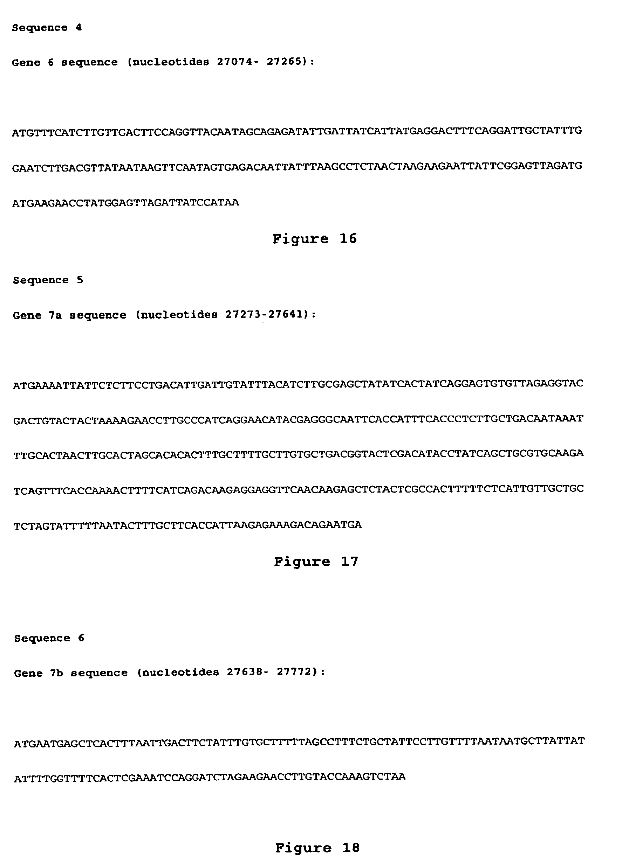

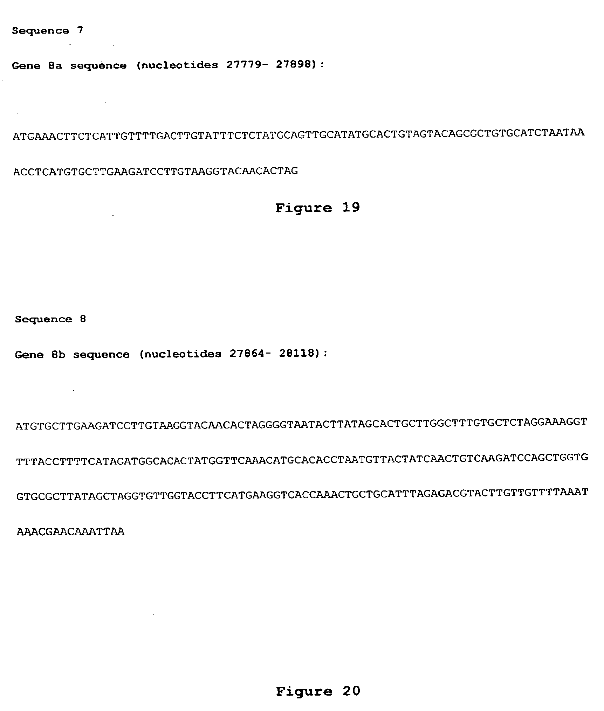

In the enclosed sequence listing and Figures, sequences of the following SARS-CoV are provided:
SARS-CoV Repla: SEQ ID NO:1
SARS-CoV Replb: SEQ ID NO:2
SARS-CoV N: SEQ ID NO:3
SARS-CoV S: SEQ ID NO:4
SARS-CoV M: SEQ ID NO:5
SARS-CoV E: SEQ ID NO:6
SARS-CoV Full lengh clone:
   SEQ ID NO: 11 Fig. 13
   SARS-CoV ORF 3a: SEQ ID NO: 12 Fig. 14
   ORF of gene 3b: SEQ ID NO: 13 Fig. 15
   ORF of gene 6: SEQ ID NO: 14 Fig. 16
   ORF of gene 7a: SEQ ID NO: 15 Fig. 17
   ORF of gene 7b: SEQ ID NO: 16 Fig. 18
   ORF of gene 8a: SEQ ID NO: 17 Fig. 19
   ORF of gene 8b: SEQ ID NO: 18 Fig. 20
   ORF of gene 9b SEQ ID NO: 19 Fig. 21

The sequence of the N protein is also designated sequence 9a. The 9b sequence is included in the 9a sequence by a frame shift. Any amendment of the 9b sequence should preferably be carried out such that the sequence of 9a is not substantially amended, i.e. in a manner that still allows expression of a functional 9a or N protein.

The SARS-CoV sequences may be based on the Urbani strain genome (Genebank, accession number AY278741). In the full-length cDNA clone two silent genetic markers at positions 10338 (C>T) and 11163 (T>A) were introduced. The nomenclature for the genes outlined above is different to that used in the sequence deposited in the GeneBank. The equivalences are:
Gene 3a is equivalent to X1
Gene 3b is equivalent to X2
Gene 6 is equivalent to X3
Gene 7a is equivalent to X4
Gene 8b is equivalent to X5

The sequences of the SARS genes and features of the SARS-CoV:
5' UTR 1-264
ORF 1a (Rep 1a) 264-13413
ORF 1b (Rep 1b) 13398-21485
S (Spike protein) 21492-25259
3a 25268-26092
3b 25689-26153
E (Envelope protein) 26117-26347
M (membrane protein) 26398-27063
6 27074-27265
7a 27273-27641
7b 27638-27772
8a 27779-27898
8b 27864-28118
9b 28130-28426
N (Nucleoprotein) 28120-29388
3' UTR 29389-29727;
   wherein UTR means "untranslated sequence".

For the purposes of the present application sequence homology is determined using the Clustal computer program available from the European Bioinformatics Institute (EBI), unless otherwise stated.

In a further embodiment of the present invention, the following proteins encoded by the above sequences of the SARS-CoV may be characterized by functional features. The Rep 1a and 1b sequences encode the viral replicase which is characterized in that it allows replication of the viral RNA. The M protein is a membrane protein and is characterized in that it associates with other proteins to an envelope of a virus particle. The N protein is a nucleocapsid protein and is characterized in that it associates with the viral RNA to a nucleocapsid of a virus particle. The S or spike protein is characterized in that it binds to the cellular receptor. The protein of 3a is characterized in that it is also present in the viral membrane.

In a further alternative embodiment of the present invention, the nucleic acids encoding an attenuated SARS-CoV may further include a nucleic acid sequence not derived from SARS-CoV, for example a foreign gene. The foreign gene may be of any origin. For example, the nucleic acids of the present invention may be used as a vector for the expression of foreign genes of other pathological microorganisms, such as viruses, bacteria, mycoplasma, etc. A vaccination with a respective nucleic acid or a SARS-CoV encoded thereby may thus provide protection against both SARS-CoV and the further pathological microorganism.

The virus particles in the vaccines of the present invention are preferably prepared starting from a SARS-CoV replicon. Methods to prepare the nucleic acids and the virus particles of the present invention may comprise the preparation of a SARS-CoV derived replicon in a bacterial artificial chromosome (BAC). Based on this BAC containing a SARS viral replicon, a full length copy of the genomic sequences of the SARS-CoV nucleic acid can be prepared as described in the Examples. The full length clone can then be used to introduce point mutations, deletions or substitutions which will inactivate certain structural or non-structural genes. Alternatively, one might also start with the replicon as deposited and amend the same by introducing only those sequences which one wants to have in the nucleic acid and deleting sequences which should not be included. In other words it is not a necessity to prepare a full length clone as an intermediate product for the preparation of a nucleic acid encoding an attenuated virus.

In accordance with the present invention vaccines are provided, which are preferably capable of inducing both a systemic immune response and a mucosal immune response against SARS-CoV.

Similar to other coronaviruses, the spike protein of the virus interacts with a cellular receptor and mediates membrane fusion to allow viral entry into susceptible target cells. Accordingly, the spike protein plays an important role in virus infection cycle and is the primary target of neutralizing antibodies.

In another embodiment of the invention the nucleic acid further encodes co-stimulating molecules, for example CD80 or CD86 or immunostimulating cytokines, for example TNF-alpha, IFN-gamma, granulocyte/macrophage colony-stimulating factor, interleukin-2 (IL-2), IL-12 or IL-18.

The vaccine of the present invention may be administered to a mammal to obtain an immune response that reduces or eliminates the disease symptoms caused by infection with the SARS-CoV virus. The vaccine is preferably used for vaccinating a mammal, especially a human, a civet cat, a racoon or a ferret. All of these animals are known to serve as a host for SARS-CoV.

The vaccine may be administered in accordance with methods routinely used in the art. Specifically vaccine may be administered by oral, intramuscular, intravenous, subcutaneous or intranasal administration. Oral administration is especially preferred.

The vaccines may also comprise pharmaceutically acceptable carriers, excipients and/or adjuvants.

Adjuvants and carriers suitable for administering genetic vaccines and immunogens via the mucosal route are known in the art. Conventional carriers and adjuvants are for example reviewed in Kiyono et al. 1996. The addition of chemokines that are used to modulate immune responses are also encompassed by the present invention. Respective compounds and their medical use has been reviewed in Toka et al. 2004. It is specifically advantagous to use one of granulocyte/macrophage colony-stimulating factor, interleukin-2 (IL-2), IL-12, IL-18. Combinatorial approaches utilizing several cytokines and chemokines might also be applied. In addition, as more is discovered regarding the requirements for memory development of T cells, boosters involving key cytokines such as IL-15 and IL-23 may prove beneficial to long-term maintenance of the memory pool.

A replicon can be generated using a set of overlapping cDNAs spanning the untranslated 5' and 3' ends of the genome, the entire replicase gene, and the nucleoprotein (N) gene.

The strategy for the construction of the SARS-CoV replicon as a bacterial artificial chromosome (BAC) can be summarized as follows:
(i) Identification of appropriate restriction sites in the viral genome to be used in the engineering of the replicon.
(ii) Generation of an intermediate plasmid as the basis for construction of the SARS-CoV replicon.
(iii) Generation of overlapping SARS-CoV cDNA subclones by RT-PCR and replicon assembly using the restriction sites selected.

The construction of a SARS-CoV replicon cDNA is illustrated with further details in Example 1.

Methods for preparing the virus particles used in the vaccines of the present invention may use a replicon as described above. According to a preferred aspect of the present invention these methods are based on the use of the plasmid pBAC-SARS-CoV-REP. Bacteria containing this plasmid were deposited on September 1, 2004 with the Colección Española de Cultivos (Tipo, CECT in Valencia, Spain). The deposit was given the provisional accession number 7020.

The plasmid pBAC-SARS-CoV-REP comprises sequences of a replication competent, non-infectious SARS-CoV genome encoding the SARS-CoV replicase and the SARS-CoV N protein. This vector provides a stable, safe and easy to handle basis for producing the virus particles and the vaccines of the present invention. The virus particles and the vaccines may be produced by cloning the at least one further SARS-CoV gene into the replicon.

Genomic viral RNA may be used as a starting material to obtain the sequences encoding the further SARS-CoV proteins. Genomic RNA of the SARS-CoV Urbani strain can be obtained from the Center for Disease Control in Atlanta, USA. A cDNA library is prepared from the virus RNAs according to methods known in the art. The full length genes can be reconstructed from the cDNA library. These genes may then be combined with transcriptional regulatory sequences (TRS) and/or translation regulatory sequences (such as internal ribosome entry sites, IRES) and can be inserted into a cloning site of the replicon. As is shown in the examples, in this manner a full length clone, encoding a full length copy of the genomic nucleic acid of SARS-CoV can be prepared and may subsequently be amended to produce a nucleic acid encoding an attenuated virus.

The nucleic acid may then be used to transfect a helper cell line. If the replicon is used, a cell line expressing the essential SARS-CoV proteins not encoded by the nucleic acid. Alternatively, a host cell may be transfected with the replicon nucleic acid as described above and a helper virus or other nucleic acid expressing the essential SARS-CoV proteins. In this manner the nucleic acid encoding SARS-CoV replicase, SARS-CoV N protein and at least one further SARS-CoV protein (i.e. the replicon) will associate with the further essential SARS-CoV proteins to form the virus particle of the vaccines of the present invention.

The vaccines can be obtained by producing a virus particle as outlined above and formulating the same into a vaccine, for example by mixing the same with a carrier, adjuvants and/or excipient.

The testing of the vaccines preferably comprises a sequence of several steps. For example the immunogenicity of attenuated SARS-CoV may be tested by producing them in tissue culture of Vero E6 cells. The antigens obtained can be partially purified by conventional procedures including centrifugation and it can be tested whether these induce SARS-CoV neutralizing antibodies in mice and rabbits.

Selected vaccines may further be tested for protection in animal systems, such as BalB/c mice, ferrets and macaques (using conventional methods as described for example in Enserink, 2003; Yang et al., 2004; ter Meulen et al., 2004; Martina et al., 2003; and Kuiken et al., 2004).

The animal model systems can be sequentially used for vaccine efficacy and preliminary safety testing. First, vaccine candidates can be tested in the mice model, then in the ferret SARS-CoV model (Martina et al., 2003). Further decisions can be based on protection against SARS-CoV challenge, the selected vaccine candidates may then be tested in macaques. To achieve this goal, a virus stock can be titrated in young adult cynomolgus macaques. In first experiments increasing logarithmic dilutions of this virus stock (10°, 10¹, 10², 10³, 10⁴, 10⁵ and 10⁶ TCID₅₀) should be used to infect macaques intra-tracheally (n= 2 per dilution). The clinical, virological, gross pathological and immunological (neutralizing antibodies, cytokine profiles in plasma and cells by mRNA analyses) data may be collected systematically from these SARS-CoV-infected animals, according to protocols that have been established previously (Kuiken et al., 2004).

### Brief description of the Figures:

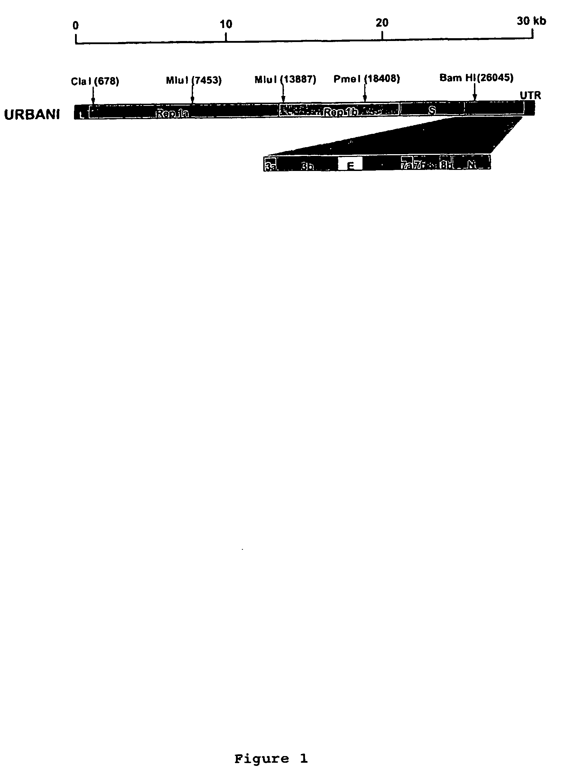

Figure 1 shows the genetic structure of SARS-CoV Urbani strain. Letters and number inside the boxes indicate the viral genes. L, leader sequence; UTR, untranslated region. Relevant restriction sites are indicated.

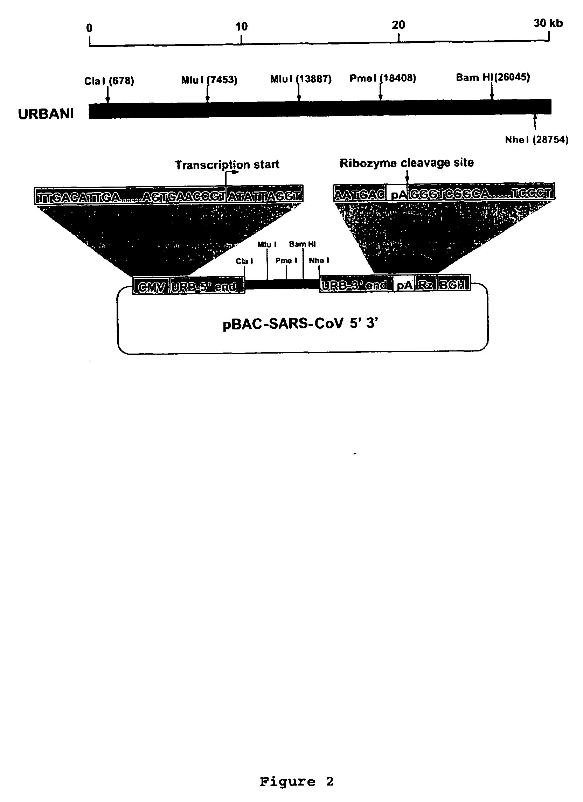

Figure 2 shows the construction of the intermediate plasmid pBAC-SARS-CoV 5' 3'. A PCR-fragment containing the 5' end 678 nt and the 3' end 973 nt of the genome of SARS-CoV Urbani strain, flanked at its left end by the cytomegalovirus (CMV) immediate-early promoter and at its right end by a poly (A) tail followed by the hepatitis delta virus ribozyme (Rz) and the bovine growth hormone (BGH) termination and polyadenylation sequences, is cloned in BAC. To facilitate the assembly of SARS-CoV replicon, a multicloning site containing the restriction sites *Cla* I, *Mlu* I, Pme I, and *Bam* HI, is cloned downstream of the 5' end viral sequence.

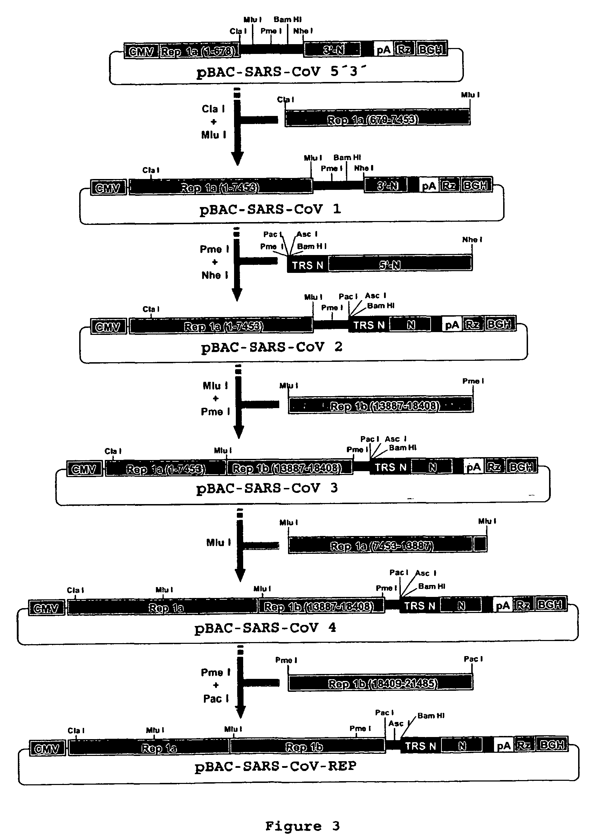

Figure 3 shows the generation of the SARS-CoV derived replicon pBAC-SARS-CoV-REP. The strategy for the construction of a SARS-CoV replicon is illustrated. SARS-CoV replicon is assembled by sequential cloning of subgenomic overlapping cDNA fragments generated by RT-PCR, into plasmid pBAC-SARS-CoV 5' 3'. The genetic map of SARS-CoV replicon is shown at the bottom. Relevant restriction sites used in the cloning step are indicated. Rep 1a, Rep 1b, and N indicate the viral genes. CMV, CMV immediate-early promoter; TRS N, natural TRS of N gene; pA, tail of 25 A residues; Rz, hepatitis delta virus ribozyme; BGH, bovine growth hormone termination and polyadenylation sequences.

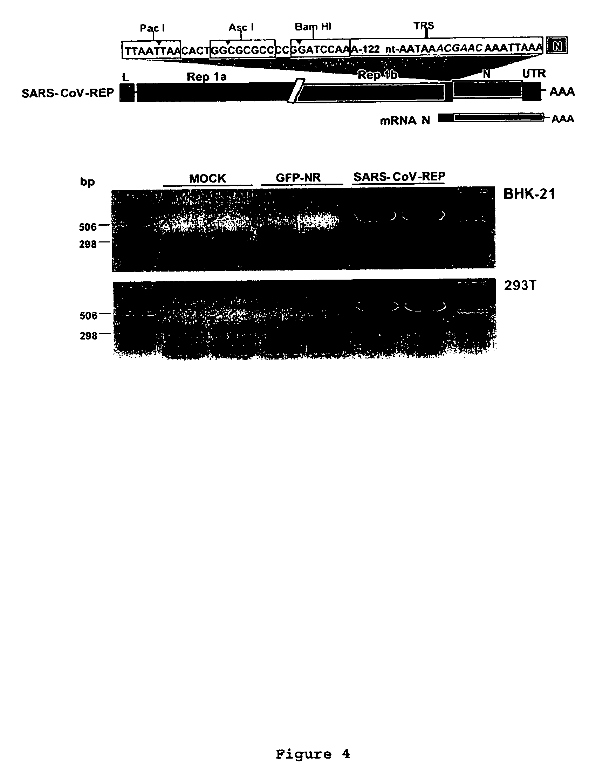

Figure 4 shows the functional analysis of SARS-CoV derived replicon. The genetic structure of the SARS-CoV replicon is illustrated at the top. The N gene TRS , the core sequence (italic letters), and the relevant restriction sites are indicated. L, leader sequence; UTR, untranslated region. BHK-21 and human 293T cells are mock transfected (MOCK) or transfected with SARS-CoV replicon (SARS-CoV-REP) or a non-replicative cDNA clone (GFP-NR) using Lipofectamine 2000. Total RNA is isolated at 24 hpt and analyzed by RT-PCR with specific oligonucleotides to detect gene N mRNA. Duplicate RT-PCR products amplified in parallel were resolved by electrophoresis in 1% agarose gels.

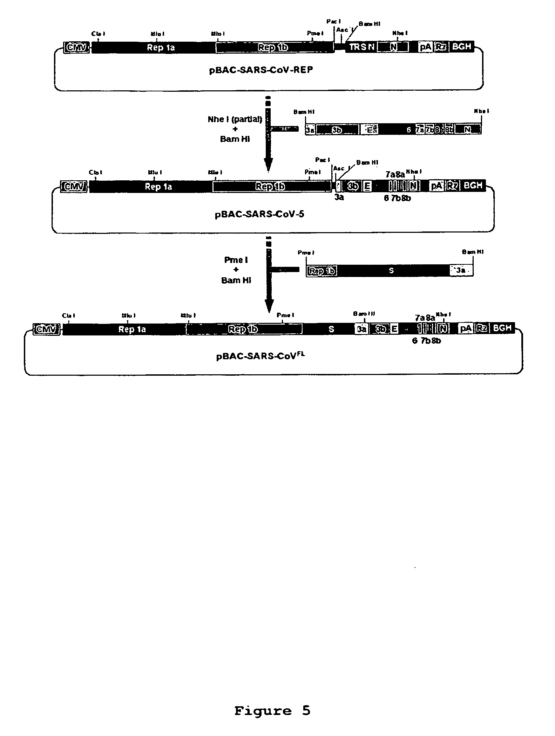

Fig. 5 shows the construction of a SARS-CoV full-length cDNA clone from the SARS-CoV replicon. Starting from the SARS-CoV replicon cloned in a BAC (see Figure 3, above), the full-length clone was been assembled by introducing the remaining SARS-CoV sequences in two steps. In a first step, a Bam HI-Nhe I fragment including the following genes: part of 3a, 3b, E, M, 7a, 7b, 8a, 8b, and part of N was inserted into the replicon. In a second step, the full-length cONA clone was completed by cloning the Pme I-Bam HI fragment including genes 3' end of Rep 1b, S, and 5' fragment of 3a into the former plasmid. Genes and relevant restriction sites used in the cloning steps are indicated. The following abbreviations are used: CMV, cytomegalovirus immediate-early promoter; pA, tail of 25 A residues; Rz, hepatitis delta virus ribozyme; BGH, bovine growth hormone termination and polyadenylation sequences.

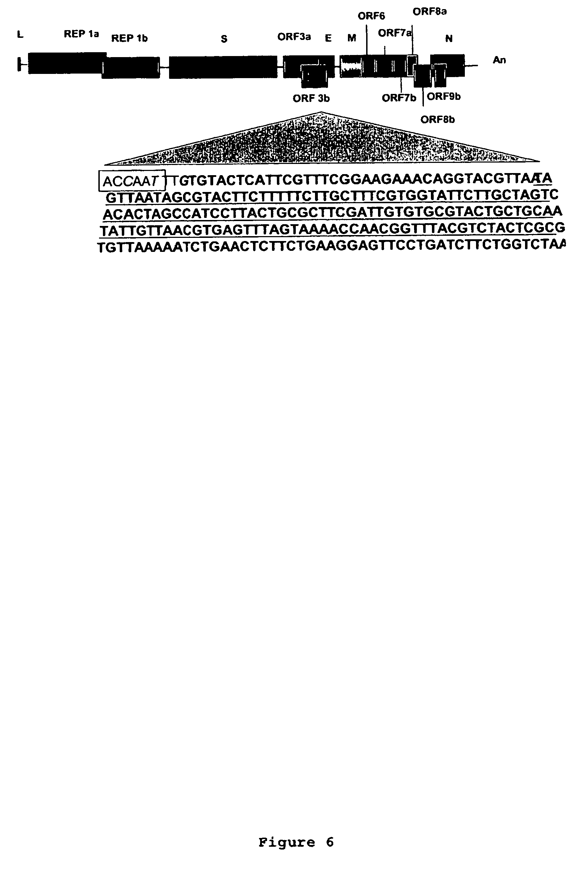

Fig. 6 shows the strategy used to generate a cDNA encoding SARS-CoV-E⁻. Core sequence and gene E open reading frame are shown. The point mutation to change initial ATG to GTG is shown in bold and italics. Gene E core sequence (CS, a highly conserved sequence that is in the middle of the TRS) is shown in a box. The two point mutations introduced in gene E CS (ACGAAC to ACCAAT) are shown in italics. The deleted sequence within gene E ORF is underlined.

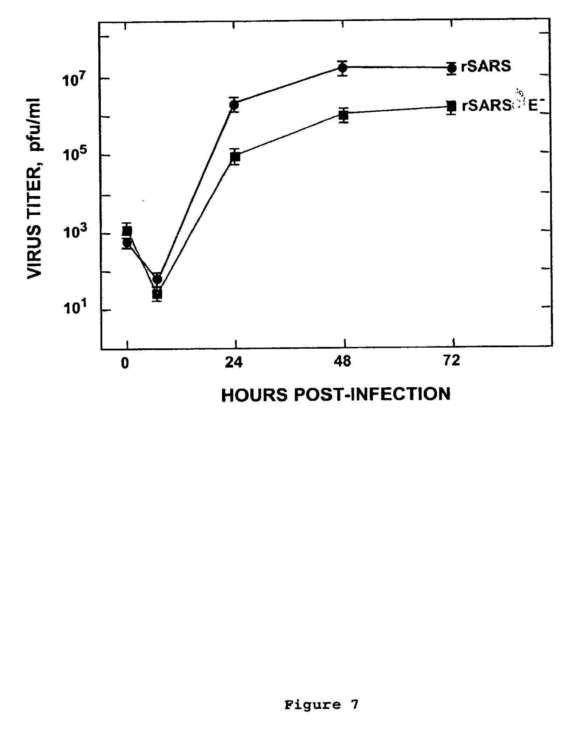

Fig. 7 shows the kinetic of SARS-CoV and SARS-CoV-E⁻ virus production in Vero E6 cells. In other words, this Figure shows that the E⁻ derivative is reduced in its growth and thus attenuated. Briefly, to rescue the engineered virus, Vero E6 cells were transfected with the cDNA encoding the SARS-CoV sequences. The E⁻ virus was rescued, indicating that gene E is not essential for the viral cycle. The titer of this defective virus was 20 to 50-fold lower than that of the parental virus (SARS-CoV). Viral titers were around 5·10⁷ pfu/ml for the parental virus and 3·10⁶ pfu/ml for the defective virus, as determined in a plaque assay on Vero E6 cells (Fig. 7).

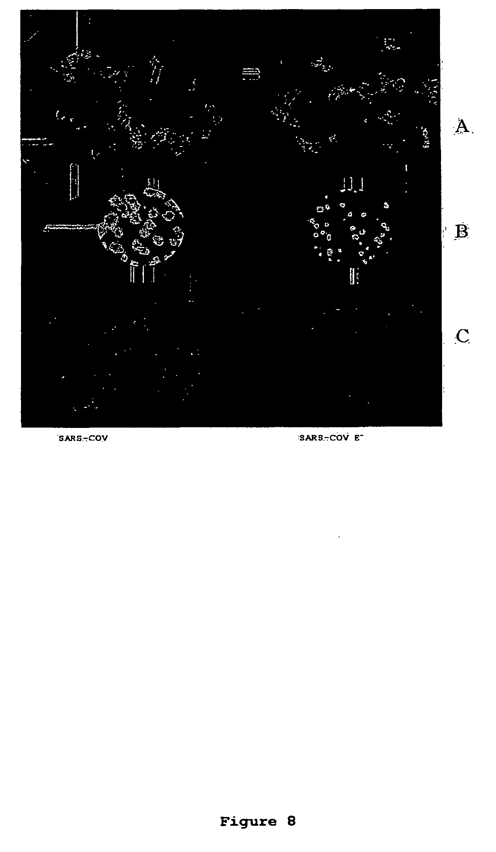

Fig. 8 shows (A) Cytopathic effects produced by the defective and parental viruses in Vero E6 cells at 24 hours post infection (upper squares); and (B) Plaque morphology of the indicated viruses on Vero E6 cells at 72 hours post infection (circles in the middle); (C) Immunofluorescence microscopy of the indicated viruses with a SARS-CoV specific polivalent antibody (bottom squares). The left half of the Figure shows infection with SARS-CoV wild-type and the right part shows SARS-CoV E⁻. Both viruses, the parental and the E⁻ produced a significant cytopatic effect in Vero E6 cells (Fig. 8A). The effect was observed earlier with the full length virus and the SARS-CoV-E⁻ viral plaques were also smaller than plaques produced by the wild type virus (Fig. 8B). An additional demonstration of the rescue of the recombinant E⁻ virus was the staining of the infected cells in immunofluorescence using SARS-CoV specific antibodies (Fig. 8C).

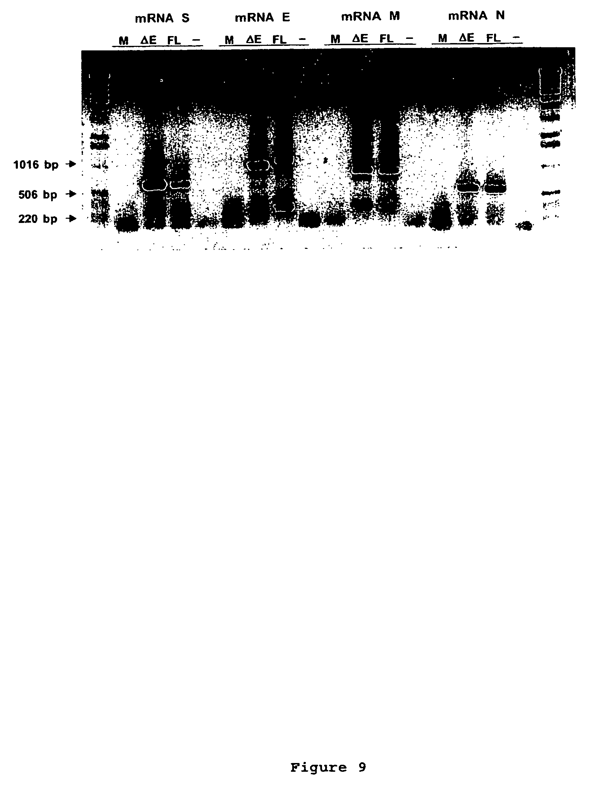

Fig. 9 shows the RT-PCR analysis of infected cells. RT-PCR was carried out using a viral sense primer which hybridizes in the viral leader sequence and reverse sense primers which hybridize in open reading frames of genes S, E, M and N respectively. M, mock infected cells; E⁻, SARS-CoV-E⁻.

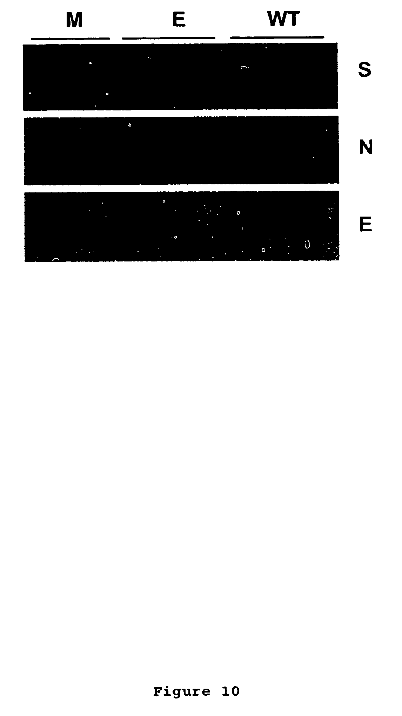

Fig. 10 shows the analysis of SARS-CoV protein expression in Vero E6 cells after being infected with SARS-CoV (WT) or SARS-CoV-E⁻ (E⁻) S, N, and E viral proteins. M, mock infected cells.

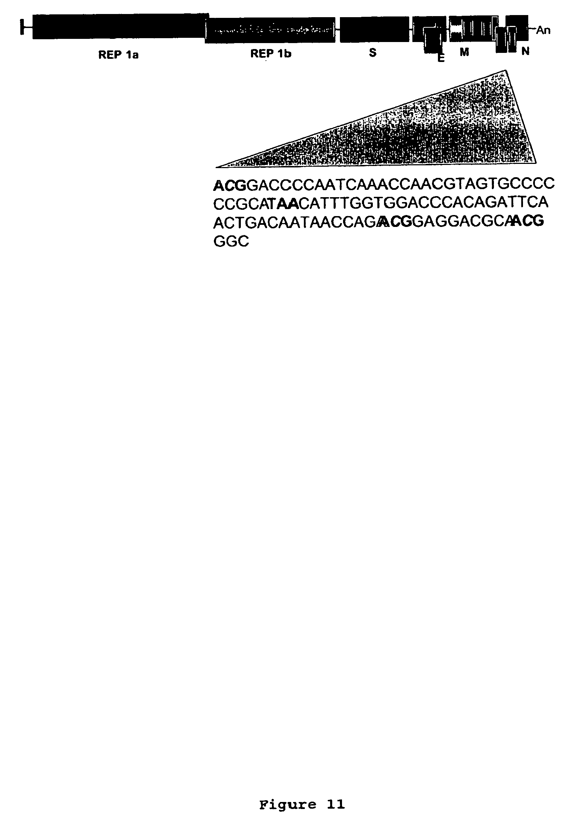

Fig. 11 shows the mutations introduced to abolish expresssion of 9b gene. The first 97 nucleotides of gene 9b ORF are shown. Three ATG codons were mutated to ACG and are shown in bold characters. The C mutated is shown in bold and in italics. The stop codon introduced, TAA, is shown in bold.

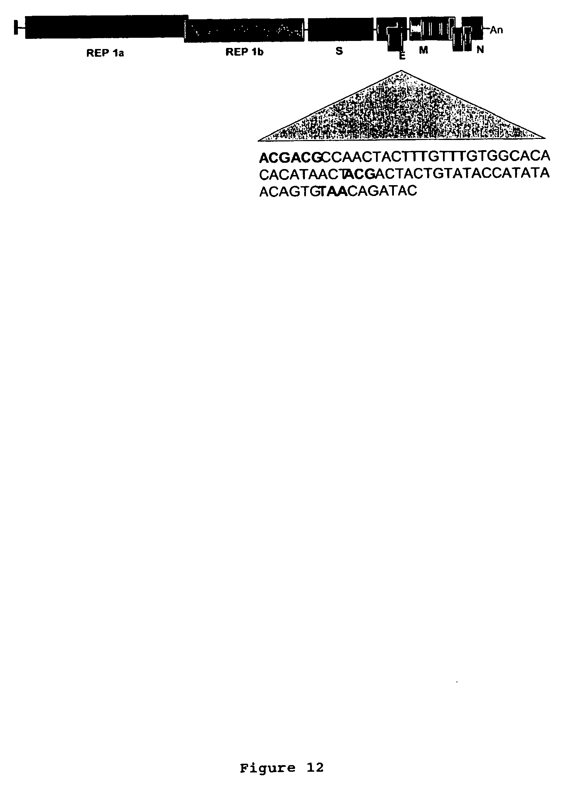

Fig. 12 shows the mutations introduced to abolish expresssion of gene 3b. The first 76 nucleotides of gene 3b ORF are shown. Three ATG codons mutated to ACG in phase are shown in bold and the C mutated is shown in bold and in italics. The stop codon introduced by changing the codon TCA to TAA, is shown in bold.
Fig. 13 to 20 show the sequences of the full lengths SARS-CoV Urbanis strain and of selected genes thereof.

The following examples illustrate but do not limit the embodiments of the invention.

### EXAMPLES

The following cells, viruses, plasmids and bacteria strains were used:

**Cells and viruses.** Baby hamster kidney cells (BHK-21) and human 293T cells were purchased from the American Collection of Cell Cultures (ATCC). All cells were maintained in DMEM supplemented with 10% fetal bovine serum and antibiotics at 37°C in a CO₂ incubator. The genomic RNA of Urbani strain was obtained from the Center for Disease Control and Prevention (CDC) after signature of a material transfer agreement.

**Plasmids and bacteria strains.** Plasmid pBeloBAC11 (Wang et al., 1997) was kindly provided by H. Shizuya and M. Simon (California Institute of Technology, Pasadena, Ca). *Escherichia coli* DH10B strain was obtained from GIBCO/BRL. DH10B cells were transformed by electroporation at 25 µF, 2.5 kV, and 200 Ω with a Gene Pulser unit (Bio-Rad) according to the manufacturer's instructions. Plasmid DNA was isolated with the Qiagen (Chatsworth, CA) Large Construct Kit according to the manufacturer's specification.

### EXAMPLE 1

### Construction of a SARS-CoV replicon cDNA

A cDNA that contained the untranslated 5' and 3' end of the Urbani genome and the replicase and N genes, was cloned as a BAC under the control of a CMV promoter.

Additionally, a multicloning site containing unique restriction sites *Pac* I, *Asc* I, and *Bam* HI, was cloned downstream of the replicase gene to allow cloning of heterologous genes (Fig. 3). This approach use a two-step amplification system that couples replicon RNA transcription in the nucleus from the CMV promoter with a second amplification step in the cytoplasm driven by the viral polymerase. The plasmid pBAC-SARS-CoV-REP, encoding the SARS-CoV replicon, was stable for at least 180 generations during its propagation in DH10B cells, as determined by restriction endonuclease analysis.

In a first step the appropriate restriction sites in the viral genome that can be used in the engineering of the replicon and the full length cDNA clone of SARS-CoV were identified (Fig. 1).

In a second step the intermediate plasmid pBAC-SARS-CoV 5' 3' was generated as the basis to construct the SARS-CoV replicon and also the full lenght cDNA construction (Fig. 2). This plasmid contained the first 678 nt of the 5'end of the Urbani strain genome under the control of the cytomegalovirus (CMV) immediate-early promoter and the last 973 nt of the genome followed by a 25-bp synthetic poly (A), the hepatitis delta virus ribozyme, and the bovine growth hormone termination and polyadenylation sequences to make an accurate 3' end. Additionally, a multicloning site containing the restriction sites *Cla* I, *Mlu* I, *Pme* I, and *Bam* HI, selected in the first step, was cloned in between the SARS-CoV sequences to allow the assembly of the SARS-CoV replicon.

In a third step the overlapping SARS-CoV cDNA subclones were generated by RT-PCR and the replicon was assembled using the restriction sites selected. Using the pBAC-SARS-CoV 5' 3' as starting point, a SARS-CoV replicon was engineered following the strategy described in Figure 3. The plasmid pBAC-SARS-CoV-REP contained the untranslated 5' and 3' ends of the Urbani genome, the replicase gene followed by a multicloning site containing unique restriction sites *Pac* I, *Asc* I, and *Bam* HI, to allow cloning and expression of heterologous genes, and the nucleoprotein (N) gene under the control of its natural TRS.

### EXAMPLE 2

### Analysis of cloned cDNA stability

The stability of the viral sequences cloned into pBeloBAC11 was analyzed by studying the restriction endonuclease pattern at different passages. Bacteria transformed with recombinant plasmid were grown in 10 ml of LB containing 12.5 µg/ml chloramphenicol at 37°C. Cells from these primary cultures (considered passage 0) were propagated serially by diluting 10⁶-fold daily. Each passage was considered to represent about 20 generations.

### EXAMPLE 3

### Sequence analysis

DNA was sequenced using an automatic 373 DNA sequencer (Applied Biosystem) using fluorochrome labeled dideoxynucleotides and temperature resistant DNA polymerase (Perkin Elmer).

### EXAMPLE 4

### Transfection of SARS-CoV replicon cDNA

BHK-21 and 293T cells were grown to 95% confluence on 35-mm-diameter plates and transfected with 5 µg of the SARS-CoV replicon, using 6 µg of Lipofectamine 2000 (Invitrogen) according to the manufacturer's specifications.

### EXAMPLE 5

### RNA isolation and RT-PCR analysis

Total intracellular RNA was extracted at 24 h post-transfection (hpt) with the RNeasy Mini Kit (Qiagen) and used as template for RT-PCR analysis of gene N mRNA transcription. RT reactions were performed with Moloney murine leukaemia virus reverse transcriptase (Ambion) and the antisense primer URB-28630RS (5'-TGCTTCCCTCTGCGTAGAAGCC-3') (SEQ ID NO:7), complementary to nucleotides 510 to 531 of gene N. The cDNAs generated were amplified by PCR using the reverse primer URB-28630RS and the forward primer URB-29VS (5'-GCCAACCAACCTCGATCTCTTG-3') (SEQ ID NO:8), spanning nucleotides 29 to 50 of the Urbani leader sequence. For the quantitative analysis by real-time RT-PCR of gene N mRNA, the primers used for RT (URB-28163RS, 5'-TGGGTCCACCAAATGTAATGC-3' (SEQ ID N0:9), complementary to nucleotides 43 to 63 of gene N) and PCR (reverse primer URB-28163RS and the forward primer URB-27VS, 5'-AAGCCAACCAACCTCGATCTC-3' (SEQ ID NO:10), spanning nucleotides 27 to 47 of the Urbani leader sequence) were designed using the Primer Express software (Applied Biosystems). The SYBR Green PCR master mix was used in the PCR step following the manufacturer's specifications (Applied Biosystems).

### EXAMPLE 6

### Analysis of the SARS-CoV derived replicon

The SARS-CoV derived replicon was functional in several cell lines. Expression of gene N mRNA was used to study replicon activity by RT-PCR analysis. BHK-21 and human 293T cells were transfected with either SARS-CoV replicon or a non-replicative cDNA clone using Lipofectamine 2000. Total intracellular RNA was extracted at 24 hpt and used as template for RT-PCR analysis of gene N mRNA transcription using specific oligonucleotides. High levels of gene N mRNA were detected in both BHK-21 and 293T cells transfected with the SARS-CoV replicon (Fig. 4), showing that the replicon isactive in these cell lines. A quantitative analysis of gene N mRNA in transfected 293T and BHK-21 cells was performed by real-time RT-PCR using the primers described in Example 5. The SARS-CoV replicon activity was 8-fold higher in 293T cells than in BHK-21 cells. This activity increase could be explained by a higher replication level of SARS-CoV replicon in human 293T cells or simply because the transfection efficiency of 293T cells is twice higher than that of BHK-21 cells.

The role of the N protein in SARS-CoV replicon activity was analyzed. To achieve this objective, a SARS-CoV replicon lacking the N gene was constructed and the replicon activity compared with that of the replicon expressing the N gene. A basal activity of the replicon lacking N gene was detected, but replicon activity increased more than 100-fold when N protein was present. These data indicated that N protein plays an important role as an enhancer of coronavirus replicon activity.

Bacteria containing the plasmid pBAC-SARS-CoV-REP comprising sequences of a replication competent, non-infectious SARS-CoV genome encoding the SARS-CoV replicase and the SARS-CoV N protein were deposited on September 1, 2004 with the Colección Española de Cultivos (Tipo, CECT in Valencia, Spain). The deposit was given the provisional accession number 7020.

### EXAMPLE 7

### Preparing a Full Length Clone

A SARS-CoV full-length cDNA clone was prepared according to the cloning strategy outlined in Fig. 5.

Starting from the SARS-CoV replicon cloned into a BAC (Figure 3; and Examples 1 to 6, above), the full-length clone was assembled by adding the rest of the genes in two steps. In a first step, a Bam HI-Nhe I fragment including the following genes: part of 3a, 3b, E, M, 7a, 7b, 8a, 8b, and part of N was inserted into the BAC containing the replicon. In a second step, the full-length cDNA clone was completed by cloning the Pme I-Bam HI fragment including genes 3' end of Rep 1b, S, and 5' fragment of 3a in the former plasmid.

The cDNA for these two fragments was obtained by RT-PCR cloning of RNA fragments which were obtained from the Center for Disease Control in Atlanta. The RNA corresponds to the genomic sequences of the Urbanis strain of SARS-CoV.

Genes and relevant restriction sites used in the cloning steps are shown in Fig. 5. Functional analysis of the resulting full-length cDNA clone showed that the clone was fully stable when propagated in E. coli.

After transfection of Vero cells, infectious virus was recovered from the cDNA clone, and was found to be identical to the parental virus in terms of plaque morphology, growth kinetics, as well as mRNA and protein patterns.

### EXAMPLE 8

### Preparing Attenuated Viruses

A collection of recombinant vaccines based on a SARS-CoV defective virus able to replicate and propagate both in cultured cells and in vivo have been generated. The nucleic acid encodes SARS-CoV replicase and some but not all of the structural and non-structural proteins. One of the main characteristics of the SARS-CoV engineered vaccine viruses is that they are infectious but highly attenuated in vivo.

### (1) A virus containing all genes of SARS-CoV except gene encoding protein E, an structural protein.

Protein E was considered to be essential for coronaviruses. To abolish gene E expression three strategies to make a cDNA encoding SARS-CoV with gene E deleted (SARS-CoV-E⁻) were used (Fig. 6):
(a) Two point mutations in gene E core sequence (CS) have been introduced. This part is the center of the sequences regulating gene transcription (TRS) and the introduction of the mutations will prevent expression of gene E. The original TRS sequence ACGAAC has been replaced by ACCAAT.
(b) A point mutation in the initiation codon of E gene open reading frame has been introduced. Initial codon ATG has been change to GTG.
(c) A deletion of 142 nucleotides within ORF gene E has been introduced.

To rescue the engineered virus, Vero E6 cells were transfected with the constructed cDNA. The E⁻ virus was rescued, indicating that gene E is not essential for the viral cycle. The titer of this defective virus was 20 to 50-fold lower than that of the parental virus (SARS-CoV). Viral titers were around 5·10⁷ pfu/ml for the parental virus and 3·10⁶ pfu/ml for the defective virus, as determined in a plaque assay on Vero E6 cells (Fig.7).

Both viruses, the parental and the E⁻ produced a significant cytopatic effect in Vero E6 cells (Fig. 8A), although the parental virus this effect was observed earlier than with the E⁻ virus. SARS-CoV-E⁻ viral plaques were smaller than plaques produced by the wild type virus (Fig. 8B). An additional demonstration of the rescue of the recombinant E⁻ virus was the staining of the infected cells in immunofluorescence using SARS-CoV specific antibodies (Fig.8C).

The identity of the E⁻ virus was confirmed by analyzing the viral mRNAs using an RT-PCR assay (Fig.9). The results showed that while gene E mRNA was produced by the parental virus this mRNA was not observed in the E⁻ virus. In addition, the absence of E protein production was shown in cells infected with the E⁻ virus, in contrast to the presence of this protein in cells infected with the parental virus, as determined by Western blot assays with E specific antibodies (Fig. 10).

The engineered recombinant virus was stable after six passages in tissue culture. Virus titers were identical at 32 an at 37°C, in contrast to what has been described for another group 2 coronavirus in which E gene was also deleted (Fischer et al., 1998).

The defective virus grew in different cell lines (CaCo-2, human, intestinal; Huh-7, human, hepatic; and Mv1Lu, mink, lung), indicating that gene E was also non-essential within these cells.

Using previously established methods (see Martina et al., 2003), the degree of attenuation of the SARS-CoV-E⁻ virus is evaluated in animal models, specifically in ferrets using the sequential dilution tests described above. The attenuated SARS-CoV E⁻ are fully attenuated in this experimental animal model. Similar experiments are performed with all vaccine strains.

### (2) Viruses lacking one or more structural and non-structural genes

The following further mutants were prepared:
A virus lacking all non structural genes of SARS-CoV (genes 3b, 6, 7a, 7b, 8a and 8b).
A virus lacking all non structural genes (genes 3b, 6, 7a, 7b, 8a, 8b) and a structural gene (gene E).
A virus lacking all non structural genes (genes 3b, 6, 7a, 7b, 8a, 8b) and structural gene 3a.
A virus lacking all non structural genes (genes 3b, 6, 7a, 7b, 8a, 8b) and the two structural genes 3a and E.
Viruses lacking structural genes (E, 3a, or both and any combination of non-structural genes 6, 7a, 7b, 8a, 8b and 9b.

### Gene 9b deletion.

To abolish expression of gene 9b:
(a) A point mutation in the initiation codon of this gene was introduced.
(b) The ATG present in the parental virus was mutated to ACG to prevent expression of the corresponding protein.
(c) A stop codon was introduced after this mutated ACG and we have change another two ATG in phase within the ORF (see Fig. 11).
   Gene 9b completely overlaps with gene N; all the N gene introduced mutations were therefore silent with respect to the sequence of the N gene (i.e. did not lead to an amino acid change).

### Gene 3b deletion

To abolish expression of gene 3b the following strategy was used (Fig. 12):
A point mutation was introduced in the initiation codon of this gene. The initiation codon ATG present in the parental SARS-CoV has been mutated to ACG. The second and third ATGs in phase present in the gene were replaced by ACG.
All these mutations have been designed to introduce silent changes in the genes 3a and E, which overlap with gene 3b.

### Simultaneous deletion of genes 3a and 3b.

To abolish expression of genes 3a and 3b we have employed three strategies to engineer a cDNA encoding SARS-CoV with non-functional genes 3a and 3b (Fig. 12):
(a) The core sequence present in the TRS that controls the expression of gene 3 mRNA was inactivated by changing the sequence ACGAAC to ACCAAT.
(b) Parts of these genes were partially deleted by introducing a deletion between nucleotides 25274 and 26015 of SARS-CoV sequence.
(c) The initiation codon ATG of gene 3a were replaced by ACG. In addition, a stop codon has been introduced close to the first.

### Bibliography

Almazán, F., J. M. González, Z. Pénzes, A. Izeta, E. Calvo, J. Plana-Durán, and L. Enjuanes (2000). Engineering the largest RNA virus genome as an infectious bacterial artificial chromosome. Proc. Natl. Acad. Sci. USA 97:5516-5521.

Alonso, S., Izeta A., Sola I., and Enjuanes L. (2002a). Transcription regulatory sequences and mRNA expression levels in the coronavirus transmissible gastroenteritis virus. J. Virol. 76:1293-1308

Alonso, S., Sola, I., Teifke, J., Reimann, I., Izeta, A., Balach, M., Plana-Durán, J., Moormann, R. J. M., and Enjuanes, L. (2002b). In vitro and in vivo expression of foreign genes by transmissible gastroenteritis coronavirus-derived minigenomes. J. Gen. Virol. 83, 567-579.

Casais, R., V. Thiel, S. G. Siddell, D. Cavanagh, and P. Britton (2001). Reverse genetics system for the avian coronavirus infectious bronchitis virus. J. Virol. 75:12359-12369.

Chang, K.-Y., and Tinoco, I. (1994). Characterization of a "kissing" hairpin complex derived from the human immunodeficiency virus genome. Proc. Natl. Acad. Sci. USA 91, 8705-8709.

Drosten, C., S. Gunther, W. Preiser, S. van der Werf, H. R. Brodt, S. Becker, H. Rabenau, M. Panning, L. Kolesnikova, R. A. Fouchier, A. Berger, A. M. Burguiere, J. Cinatl, M. Eickmann, N. Escriou, K. Grywna, S. Kramme, J. C. Manuguerra, S. Muller, V. Rickerts, M. Sturmer, S. Vieth, H. D. Klenk, A. D. Osterhaus, H. Schmitz, and H. W. Doerr (2003). Identification of a novel coronavirus in patients with severe acute respiratory syndrome. N. Engl. J. Med. 348:1967-1976.

Enserink M. (2003). Infectious diseases. SARS-CoV researchers report new animal models. Science 302(5643):213.

Fischer, F., Stegen, C. F., Masters, P. S., and Samsonoff, W. A. (1998). Analysis of constructed E gene mutants of mouse hepatitis virus confirms a pivotal role for E protein in coronavirus assembly. J. Virol. 72, 7885-7894.

González, J. M., Z. Pénzes, F. Almazán, E. Calvo, and L. Enjuanes (2002). Stabilization of a full-length infectious cDNA clone of transmissible gastroenteritis coronavirus by insertion of an intron. J. Virol. **76**:4655-4661.

Holmes, K. V., and L. Enjuanes (2003). Virology. The SARS-CoV coronavirus: a postgenomic era. Science **300**:1377-1378.

Izeta, A., Smerdou, C., Alonso, S., Penzes, Z., Méndez, A., Plana-Durán, J., and Enjuanes, L. (1999). Replication and packaging of transmissible gastroenteritis coronavirus-derived synthetic minigenomes. *J*. *Virol.* **73**, 1535-1545.

Kiyono et al. (1996). Mucosal Vaccines. Academic Press, New York.

Kuiken T, van den Hoogen BG, van Riel DA, Laman JD, van Amerongen G, Sprong L, Fouchier RA, Osterhaus AD. (2004). Experimental human metapneumovirus infection of cynomolgus macaques (Macaca fascicularis) results in virus replication in ciliated epithelial cells and pneumocytes with associated lesions throughout the respiratory tract. Am J Pathol. **164**:1893.

Kuo, L., Hurst, R., and Masters, P. S. (2002). 21st Annual Meeting, Lexington, Kentucky.

Marra, M. A., S. J. Jones, C. R. Astell, R. A. Holt, A. Brooks-Wilson, Y. S. Butterfield, J. Khattra, J. K. Asano, S. A. Barber, S. Y. Chan, A. Cloutier, S. M. Coughlin, D. Freeman, N. Girn, O. L. Griffith, S. R. Leach, M. Mayo, H. McDonald, S. B. Montgomery, P. K. Pandoh, A. S. Petrescu, A. G. Robertson, J. E. Schein, A. Siddiqui, D. E. Smailus, J. M. Stott, G. S. Yang, F. Plummer, A. Andonov, H. Artsob, N. Bastien, K. Bernard, T. F. Booth, D. Bowness, M. Czub, M. Drebot, L. Fernando, R. Flick, M. Garbutt, M. Gray, A. Grolla, S. Jones, H. Feldmann, A. Meyers, A. Kabani, Y. Li, S. Normand, U. Stroher, G. A. Tipples, S. Tyler, R. Vogrig, D. Ward, B. Watson, R. C. Brunham, M. Krajden, M. Petric, D. M. Skowronski, C. Upton, and R. L. Roper (2003). The genome sequence of the SARS-CoV-associated coronavirus. Science 300:1399-1404.

Martina BE, Haagmans BL, Kuiken T, Fouchier RA, Rimmelzwaan GF, Van Amerongen G, Peiris JS, Lim W, Osterhaus AD. (2003). Virology: SARS-CoV virus infection of cats and ferrets. Nature 425:915.

Mortola E, and Roy P. Efficient assembly and release of SARS coronavirus-like particles by a heterologous expression system. FEBS Letters 2004; 576:174-178.

Ortego, J., Escors, D., Laude, H., and Enjuanes, L. (2002). Generation of a replication-competent, propagation-deficient virus vector based on the transmissible gastroenteritis coronavirus genome. J. Virol. 76, 11518-11529.

Ortego, J., Sola, I., Almazan, F., Ceriani, J. E., Riquelme, C., Balasch, M., Plana-Durán, J., and Enjuanes, L. (2003). Transmissible gastroenteritis coronavirus gene 7 is not essential but influences in vivo virus replication and virulence. Virology 308, 13-22.

Rota, P. A., M. S. Oberste, S. S. Monroe, W. A. Nix, R. Campagnoli, J. P. Icenogle, S. Penaranda, B. Bankamp, K. Maher, M. H. Chen, S. Tong, A. Tamin, L. Lowe, M. Frace, J. L. De-Risi, Q. Chen, D. Wang, D. D. Erdman, T. C. Peret, C. Burns, T. G. Ksiazek, P. E. Rollin, A. Sanchez, S. Liffick, B. Holloway, J. Limor, K. McCaustland, M. Olsen-Rasmussen, R. Fouchier, S. Gunther, A. D. Osterhaus, C. Drosten, M. A. Pallansch, L. J. Anderson, and W. J. Bellini (2003). Characterization of a novel coronavirus associated with severe acute respiratory syndrome. Science 300:1394-1399.

Sawicki, S. G., and D. L. Sawicki (1998). A new model for coronavirus transcription. Adv. Exp. Med. Biol. 440:215-219.

Sawicki & Sawicki (1990). Coronavirus transcription: subgenomic mouse hepatitis virus replicative intermediates function in RNA synthesis. J Virol. 64(3):1050-6.

Sethna, P. B., Hung, S.-L., and Brian, D. A. (1989). Coronavirus subgenomic minus-strand RNAs and the potential for mRNA replicons. Proc. Natl. Acad. Sci. USA 86, 5626-5630.

Siddell, S. G. (1995). "The Coronaviridae." The Viruses (H. Fraenkel-Conrat, and R. R. Wagner, Eds.) Plenum Press, New York.

Sánchez, C. M., Izeta, A., Sánchez-Morgado, J. M., Alonso, S., Sola, I., Balasch, M., Plana-Durán, J. and Enjuanes, L. (1999). Targeted recombination demonstrates that the spike gene of transmissible gastroenteritis coronavirus is a determinant of its enteric tropism and virulence. J. Virol. 73:7607-7618.

ter Meulen J, Bakker AB, van den Brink EN, Weverling GJ, Martina BE, Haagmans BL, Kuiken T, de Kruif J, Preiser W, Spaan W, Gelderblom HR, Goudsmit J, Osterhaus AD. (2004). Human monoclonal antibody as prophylaxis for SARS-CoV coronavirus infection in ferrets. Lancet. 26;363(9427):2139-41.

Thiel, V., J. Herold, B. Schelle, and S. G. Siddell (2001). Infectious RNA transcribed in vitro from a cDNA copy of the human coronavirus genome cloned in vaccinia virus. J. Gen. Virol. 82:1273-1281.

Thiel, V., K. A. Ivanov, A. Putics, T. Hertzig, B. Schelle, S. Bayer, B. Weibrich, E. J. Snijder, H. Rabenau, H. W. Doerr, A. E. Gorbalenya and J. Ziebuhr (2003). Mechanism and enzymes involved in SARS-CoV coronavirus genome expression. J. Gen. Virol. 84: 2305-2315.

Toka, F.N. et al. (2004). Molecular adjuvants for mucosal immunity. Immunol Rev. 199:100-112.

van der Most, R. G., and Spaan, W. J. M. (1995). Coronavirus replication, transcription, and RNA recombination. In "The Coronaviridae" (S. G. Siddell, Ed.), pp. 11-31. Plenum Press, New York.

Wang, K., C. Boysen, H. Shizuya, M. I. Simon and L. Hoods (1997). Complete nucleotide sequence of two generations of a bacterial artificial chromosome cloning vector. BioTechniques 23: 992-994.

Yang ZY, Kong WP, Huang Y, Roberts A, Murphy BR, Subbarao K, Nabel GJ. (2004). A DNA vaccine induces SARS-CoV coronavirus neutralization and protective immunity in mice. Am J Pathol. 164:1893-900.

Yount, B., K. M. Curtis, and R. S. Baric (2000). Strategy for systematic assembly of large RNA and DNA genomes: transmissible gastroenteritis virus model. J. Virol. 74:10600-10611.

Yount, B., M. R. Denison, S. R. Weiss, and R. S. Baric (2002). Systematic assembly of a full-length infectious cDNA of mouse hepatitis virus strain A59. J. Virol. 76:11065-11078.

Yount, B., K. M. Curtis, E. A. Fritz, L. E. Hensley, P. B. Jahrling, E. Prentice, M. R. Denison, T. W. Geisbert, and R. S. Baric (2003). Reverse genetics with a full-length infectious cDNA of severe acute respiratory syndrome coronavirus. Proc. Natl. Acad. Sci. USA 100:12995-13000.

Ziebuhr, J., E. J. Snijder, and A. E. Gorbalenya (2000). Virus-encoded proteinases and proteolytic processing in the Nidovirales. J. Gen. Virol. 81:853-879.

Zúñiga, S., I. Sola, S. Alonso, and L. Enjuanes (2004). Sequence motifs involved in the regulation of discontinuous coronavirus subgenomic RNA synthesis. J. Virol. 78:980-994.

## Claims

1. Nucleic acid encoding an attenuated SARS-CoV virus capable of producing a maximum viral titer in cell culture that is reduced at least by a factor of 2 when compared to the maximum viral titer of wild-type SARS-CoV virus in the same cell culture.

2. Nucleic acid encoding an attenuated SARS-CoV virus, wherein the nucleic acid is obtainable by a method comprising steps, wherein the genome of a SARS-CoV virus is modified by amending the sequence of the gene encoding the SARS-CoV E protein so that the nucleic acid cannot express a functional E protein.

3. Nucleic acid encoding an attenuated SARS-CoV virus, wherein the nucleic acid does not encode a sequence of a functional protein E of the SARS-CoV virus.

4. Nucleic acid encoding an attenuated SARS-CoV virus, wherein the nucleic acid comprises the sequences encoding the viral proteins replicase, S, M and N and further comprising or or not comprising the sequences encoding protein 3a .

5. Nucleic acid encoding an attenuated SARS-CoV virus, wherein the nucleic acid comprises the sequences of the viral proteins replicase, S, M, N and E and further comprising or or not comprising the sequences encoding protein 3a.

6. Nucleic acid according to one of claims 1 to 5, wherein the nucleic acid does not contain any SARS-CoV derived protein coding sequences other than replicase S, M, N, and/or E and further comprising or or not comprising the sequences encoding protein 3a.

7. Nucleic acid according to one of claims 1 to 6, wherein the nucleic acid sequence encoding
(a) the replicase is SEQ ID Nos: 1 and 2 or a sequence having a homology of at least 70%, preferably at least 85% or at least 95% to the sequence of SEQ ID NOs: 1 and 2; and/or
(b) the N or 9a protein is SEQ ID No: 3 or a sequence having a homology of at least 70%, preferably at least 85% or at least 95% to the sequence of SEQ ID NO: 3; and/or
(c) the S protein is SEQ ID No: 4 or a sequence having a homology of at least 70%, preferably at least 85% or at least 95% to the sequence of SEQ ID NO: 4; and/or
(d) the M protein is SEQ ID No: 5 or a sequence having a homology of at least 70%, preferably at least 85% or at least 95% to the sequence of SEQ ID NO: 5; and/or
(e) the E protein is SEQ ID No: 6 or a sequence having a homology of at least 70%, preferably at least 85% or at least 95% to the sequence of SEQ ID NO: 6; and/or
(f) the protein of gene 3a is SEQ ID No: 12 or a sequence having a homology of at least 70%, preferably at least 85% or at least 95% to the sequence of SEQ ID NO: 4; and/or
(g) the protein of gene 6 is SEQ ID No: 14 or a sequence having a homology of at least 70%, preferably at least 85% or at least 95% to the sequence of SEQ ID NO: 14; and/or
(h) the protein of gene 7a is SEQ ID No: 15 or a sequence having a homology of at least 70%, preferably at least 85% or at least 95% to the sequence of SEQ ID NO: 15; and/or
(i) the protein of gene 7b is SEQ ID No: 16 or a sequence having a homology of at least 70%, preferably at least 85% or at least 95% to the sequence of SEQ ID NO: 16; and/or
(j) the protein of gene 8a is SEQ ID No: 17 or a sequence having a homology of at least 70%, preferably at least 85% or at least 95% to the sequence of SEQ ID NO: 17; and/or
(k) the protein of gene 8b is SEQ ID No: 18 or a sequence having a homology of at least 70%, preferably at least 85% or at least 95% to the sequence of SEQ ID NO: 18; and/or
(1) the protein of gene 9b is SEQ ID No: 19 or a sequence having a homology of at least 70%, preferably at least 85% or at least 95% to the sequence of SEQ ID NO: 19.

8. Nucleic acid according to one of claims 1 to 7, wherein the attenuated SARS-CoV virus is capable of producing a maximum viral titer in cell culture that is at least by a factor of 5, preferably at least by a factor of 10 or 20 reduced when compared to maximum viral titer of wild-type SARS-CoV virus in the same cell culture.

9. Nucleic acid according to claim 1, wherein the cell culture is a culture of Vero E6 or Huh7 cells transfected with the nucleic acid.

10. Nucleic acid according to one of claims 1 to 9, wherein the nucleic acid further comprises a sequence not derived from SARS-CoV, preferably a foreign gene.

11. Host cell comprising a nucleic acid according to any of claims 1 to 10.

12. SARS-CoV virus particle comprising the nucleic acid of any of claims 1 to 10.

13. SARS-CoV virus particle obtainable by transfecting a host cell with a nucleic acid of any of claims 1 to 10.

14. Vaccine comprising a SARS-CoV virus particle according to claim 12 or 13 or an inactivated SARS-CoV virus particle or a nucleic acid according to any of claims 1 to 10 or a host cell according to claim 10 and a pharmaceutically acceptable adjuvans, carrier or excipient.

15. Vaccine according to claim 14, wherein administration of the vaccine to a mammal gives rise to an immune response that reduces or eliminates the disease symptoms caused by infection with the SARS-CoV virus.

16. Vaccine according to claim 14 or 15, wherein the vaccine is to be administered to a human or an animal, wherein the animal is preferably a civet cat, a racoon or a ferret.

17. Vaccine according to one of claims 14 to 16, wherein the vaccine is to be administered orally, intramuscularly, intranvenously, subcutaneously or intranasally.

18. Vaccine according to one of claims 13 to 16, wherein the adjuvant is selected from the list consisting of cytokines, chemokines granulocyte/macrophage colony-stimulating factor, interleukin-2 (IL-2), IL-12, IL-18, IL-15 and IL-23.

19. Use of a virus particle according to claim 11 or 12 or a nucleic acid according to claims 1 to 9 for the preparation of a vaccine for vaccinating a mammal, specifically a human, a civet cat, a racoon or a ferret against SARS-CoV infection.

20. Method for preparing a SARS-CoV vaccine, comprising modifying a nucleic acid comprising the genome of a SARS-CoV virus by amending the sequence of the gene encoding the SARS-CoV E protein so that the gene cannot express a functional E protein.

21. Method according to claim 20, wherein the method further comprises amending the sequence of the genome of a SARS-CoV such that the nucleic acid is not capable of expressing a protein having a homology of at least 80%, preferably a homology of at least 90% or a homology of at least 95% to the protein encoded by SARS-CoV genes 3a, 3b, 6, 7a, 7b, 8a and 8b (corresponding to SEQ ID NO: ...).

22. Method according to claim 20 or claim 21, wherein the method further comprises introducing the modified nucleic acid into a host cell and isolating a nucleic acid encoding the amended genome or SARS-CoV virus particles comprising the modified nucleic acid.

23. Method according to one of claims 20 to 22, wherein the method further comprises the mixing of the nucleic acid or of the SARS-CoV virus particle with a pharmaceutically acceptable adjuvans, carrier or excipient.


### Annex to the application documents - subsequently filed sequences listing

SEQUENCE LISTING

<110> Consejo Superior de Investigaciones Cientificas

<120> SARS-VACCINE

<130> P69513

<160> 19

<170> PatentIn version 3.1

<210> 1
<211> 13149
<212> DNA
<213> SARS-CoV

<400> 1
atggagagcc ttgttcttgg tgtcaacgag aaaacacacg tccaactcag tttgcctgtc 60

cttcaggtta gagacgtgct agtgcgtggc ttcggggact ctgtggaaga ggccctatcg 120

gaggcacgtg aacacctcaa aaatggcact tgtggtctag tagagctgga aaaaggcgta 180

ctgccccagc ttgaacagcc ctatgtgttc attaaacgtt ctgatgcctt aagcaccaat 240

cacggccaca aggtcgttga gctggttgca gaaatggacg gcattcagta cggtcgtagc 300

ggtataacac tgggagtact cgtgccacat gtgggcgaaa ccccaattgc ataccgcaat 360

gttcttcttc gtaagaacgg taataaggga gccggtggtc atagctatgg catcgatcta 420

aagtcttatg acttaggtga cgagcttggc actgatccca ttgaagatta tgaacaaaac 480

tggaacacta agcatggcag tggtgcactc cgtgaactca ctcgtgagct caatggaggt 540

gcagtcactc gctatgtcga caacaatttc tgtggcccag atgggtaccc tcttgattgc 600

atcaaagatt ttctcgcacg cgcgggcaag tcaatgtgca ctctttccga acaacttgat 660

tacatcgagt cgaagagagg tgtctactgc tgccgtgacc atgagcatga aattgcctgg 720

ttcactgagc gctctgataa gagctacgag caccagacac ccttcgaaat taagagtgcc 780

aagaaatttg acactttcaa aggggaatgc ccaaagtttg tgtttcctct taactcaaaa 840

gtcaaagtca ttcaaccacg tgttgaaaag aaaaagactg agggtttcat ggggcgtata 900

cgctctgtgt accctgttgc atctccacag gagtgtaaca atatgcactt gtctaccttg 960

atgaaatgta atcattgcga tgaagtttca tggcagacgt gcgactttct gaaagccact 1020

tgtgaacatt gtggcactga aaatttagtt attgaaggac ctactacatg tgggtaccta 1080

cctactaatg ctgtagtgaa aatgccatgt cctgcctgtc aagacccaga gattggacct 1140

gagcatagtg ttgcagatta tcacaaccac tcaaacattg aaactcgact ccgcaaggga 1200

ggtaggacta gatgttttgg aggctgtgtg tttgcctatg ttggctgcta taataagcgt 1260

gcctactggg ttcctcgtgc tagtgctgat attggctcag gccatactgg cattactggt 1320

gacaatgtgg agaccttgaa tgaggatctc cttgagatac tgagtcgtga acgtgttaac 1380

attaacattg ttggcgattt tcatttgaat gaagaggttg ccatcatttt ggcatctttc 1440

tctgcttcta caagtgcctt tattgacact ataaagagtc ttgattacaa gtctttcaaa 1500

accattgttg agtcctgcgg taactataaa gttaccaagg gaaagcccgt aaaaggtgct 1560

tggaacattg gacaacagag atcagtttta acaccactgt gtggtttccc ctcacaggct 1620

gctggtgtta tcagatcaat ttttgcgcgc acacttgatg cagcaaacca ctcaattcct 1680

gatttgcaaa gagcagctgt caccatactt gatggtattt ctgaacagtc attacgtctt 1740

gtcgacgcca tggtttatac ttcagacctg ctcaccaaca gtgtcattat tatggcatat 1800

gtaactggtg gtcttgtaca acagacttct cagtggttgt ctaatctttt gggcactact 1860

gttgaaaaac tcaggcctat ctttgaatgg attgaggcga aacttagtgc aggagttgaa 1920

tttctcaagg atgcttggga gattctcaaa tttctcatta caggtgtttt tgacatcgtc 1980

aagggtcaaa tacaggttgc ttcagataac atcaaggatt gtgtaaaatg cttcattgat 2040

gttgttaaca aggcactcga aatgtgcatt gatcaagtca ctatcgctgg cgcaaagttg 2100

cgatcactca acttaggtga agtcttcatc gctcaaagca agggacttta ccgtcagtgt 2160

atacgtggca aggagcagct gcaactactc atgcctctta aggcaccaaa agaagtaacc 2220

tttcttgaag gtgattcaca tgacacagta cttacctctg aggaggttgt tctcaagaac 2280

ggtgaactcg aagcactcga gacgcccgtt gatagcttca caaatggagc tatcgttggc 2340

acaccagtct gtgtaaatgg cctcatgctc ttagagatta aggacaaaga acaatactgc 2400

gcattgtctc ctggtttact ggctacaaac aatgtctttc gcttaaaagg gggtgcacca 2460

attaaaggtg taacctttgg agaagatact gtttgggaag ttcaaggtta caagaatgtg 2520

agaatcacat ttgagcttga tgaacgtgtt gacaaagtgc ttaatgaaaa gtgctctgtc 2580

tacactgttg aatccggtac cgaagttact gagtttgcat gtgttgtagc agaggctgtt 2640

gtgaagactt tacaaccagt ttctgatctc cttaccaaca tgggtattga tcttgatgag 2700

tggagtgtag ctacattcta cttatttgat gatgctggtg aagaaaactt ttcatcacgt 2760

atgtattgtt ccttttaccc tccagatgag gaagaagagg acgatgcaga gtgtgaggaa 2820

gaagaaattg atgaaacctg tgaacatgag tacggtacag aggatgatta tcaaggtctc 2880

cctctggaat ttggtgcctc agctgaaaca gttcgagttg aggaagaaga agaggaagac 2940

tggctggatg atactactga gcaatcagag attgagccag aaccagaacc tacacctgaa 3000

gaaccagtta atcagtttac tggttattta aaacttactg acaatgttgc cattaaatgt 3060

gttgacatcg ttaaggaggc acaaagtgct aatcctatgg tgattgtaaa tgctgctaac 3120

atacacctga aacatggtgg tggtgtagca ggtgcactca acaaggcaac caatggtgcc 3180

atgcaaaagg agagtgatga ttacattaag ctaaatggcc ctcttacagt aggagggtct 3240

tgtttgcttt ctggacataa tcttgctaag aagtgtctgc atgttgttgg acctaaccta 3300

aatgcaggtg aggacatcca gcttcttaag gcagcatatg aaaatttcaa ttcacaggac 3360

atcttacttg caccattgtt gtcagcaggc atatttggtg ctaaaccact tcagtcttta 3420

caagtgtgcg tgcagacggt tcgtacacag gtttatattg cagtcaatga caaagctctt 3480

tatgagcagg ttgtcatgga ttatcttgat aacctgaagc ctagagtgga agcacctaaa 3540

caagaggagc caccaaacac agaagattcc aaaactgagg agaaatctgt cgtacagaag 3600

cctgtcgatg tgaagccaaa aattaaggcc tgcattgatg aggttaccac aacactggaa 3660

gaaactaagt ttcttaccaa taagttactc ttgtttgctg atatcaatgg taagctttac 3720

catgattctc agaacatgct tagaggtgaa gatatgtctt tccttgagaa ggatgcacct 3780

tacatggtag gtgatgttat cactagtggt gatatcactt gtgttgtaat accctccaaa 3840

aaggctggtg gcactactga gatgctctca agagctttga agaaagtgcc agttgatgag 3900

tatataacca cgtaccctgg acaaggatgt gctggttata cacttgagga agctaagact 3960

gctcttaaga aatgcaaatc tgcattttat gtactacctt cagaagcacc taatgctaag 4020

gaagagattc taggaactgt atcctggaat ttgagagaaa tgcttgctca tgctgaagag 4080

acaagaaaat taatgcctat atgcatggat gttagagcca taatggcaac catccaacgt 4140

aagtataaag gaattaaaat tcaagagggc atcgttgact atggtgtccg attcttcttt 4200

tatactagta aagagcctgt agcttctatt attacgaagc tgaactctct aaatgagccg 4260

cttgtcacaa tgccaattgg ttatgtgaca catggtttta atcttgaaga ggctgcgcgc 4320

tgtatgcgtt ctcttaaagc tcctgccgta gtgtcagtat catcaccaga tgctgttact 4380

acatataatg gatacctcac ttcgtcatca aagacatctg aggagcactt tgtagaaaca 4440

gtttctttgg ctggctctta cagagattgg tcctattcag gacagcgtac agagttaggt 4500

gttgaatttc ttaagcgtgg tgacaaaatt gtgtaccaca ctctggagag ccccgtcgag 4560

tttcatcttg acggtgaggt tctttcactt gacaaactaa agagtctctt atccctgcgg 4620

gaggttaaga ctataaaagt gttcacaact gtggacaaca ctaatctcca cacacagctt 4680

gtggatatgt ctatgacata tggacagcag tttggtccaa catacttgga tggtgctgat 4740

gttacaaaaa ttaaacctca tgtaaatcat gagggtaaga ctttctttgt actacctagt 4800

gatgacacac tacgtagtga agctttcgag tactaccata ctcttgatga gagttttctt 4860

ggtaggtaca tgtctgcttt aaaccacaca aagaaatgga aatttcctca agttggtggt 4920

ttaacttcaa ttaaatgggc tgataacaat tgttatttgt ctagtgtttt attagcactt 4980

caacagcttg aagtcaaatt caatgcacca gcacttcaag aggcttatta tagagcccgt 5040

gctggtgatg ctgctaactt ttgtgcactc atactcgctt acagtaataa aactgttggc 5100

gagcttggtg atgtcagaga aactatgacc catcttctac agcatgctaa tttggaatct 5160

gcaaagcgag ttcttaatgt ggtgtgtaaa cattgtggtc agaaaactac taccttaacg 5220

ggtgtagaag ctgtgatgta tatgggtact ctatcttatg ataatcttaa gacaggtgtt 5280

tccattccat gtgtgtgtgg tcgtgatgct acacaatatc tagtacaaca agagtcttct 5340

tttgttatga tgtctgcacc acctgctgag tataaattac agcaaggtac attcttatgt 5400

gcgaatgagt acactggtaa ctatcagtgt ggtcattaca ctcatataac tgctaaggag 5460

accctctatc gtattgacgg agctcacctt acaaagatgt cagagtacaa aggaccagtg 5520

actgatgttt tctacaagga aacatcttac actacaacca tcaagcctgt gtcgtataaa 5580

ctcgatggag ttacttacac agagattgaa ccaaaattgg atgggtatta taaaaaggat 5640

aatgcttact atacagagca gcctatagac cttgtaccaa ctcaaccatt accaaatgcg 5700

agttttgata atttcaaact cacatgttct aacacaaaat ttgctgatga tttaaatcaa 5760

atgacaggct tcacaaagcc agcttcacga gagctatctg tcacattctt cccagacttg 5820

aatggcgatg tagtggctat tgactataga cactattcag cgagtttcaa gaaaggtgct 5880

aaattactgc ataagccaat tgtttggcac attaaccagg ctacaaccaa gacaacgttc 5940

aaaccaaaca cttggtgttt acgttgtctt tggagtacaa agccagtaga tacttcaaat 6000

tcatttgaag ttctggcagt agaagacaca caaggaatgg acaatcttgc ttgtgaaagt 6060

caacaaccca cctctgaaga agtagtggaa aatcctacca tacagaagga agtcatagag 6120

tgtgacgtga aaactaccga agttgtaggc aatgtcatac ttaaaccatc agatgaaggt 6180

gttaaagtaa cacaagagtt aggtcatgag gatcttatgg ctgcttatgt ggaaaacaca 6240

agcattacca ttaagaaacc taatgagctt tcactagcct taggtttaaa aacaattgcc 6300

actcatggta ttgctgcaat taatagtgtt ccttggagta aaattttggc ttatgtcaaa 6360

ccattcttag gacaagcagc aattacaaca tcaaattgcg ctaagagatt agcacaacgt 6420

gtgtttaaca attatatgcc ttatgtgttt acattattgt tccaattgtg tacttttact 6480

aaaagtacca attctagaat tagagcttca ctacctacaa ctattgctaa aaatagtgtt 6540

aagagtgttg ctaaattatg tttggatgcc ggcattaatt atgtgaagtc acccaaattt 6600

tctaaattgt tcacaatcgc tatgtggcta ttgttgttaa gtatttgctt aggttctcta 6660

atctgtgtaa ctgctgcttt tggtgtactc ttatctaatt ttggtgctcc ttcttattgt 6720

aatggcgtta gagaattgta tcttaattcg tctaacgtta ctactatgga tttctgtgaa 6780

ggttcttttc cttgcagcat ttgtttaagt ggattagact cccttgattc ttatccagct 6840

cttgaaacca ttcaggtgac gatttcatcg tacaagctag acttgacaat tttaggtctg 6900

gccgctgagt gggttttggc atatatgttg ttcacaaaat tcttttattt attaggtctt 6960

tcagctataa tgcaggtgtt ctttggctat tttgctagtc atttcatcag caattcttgg 7020

ctcatgtggt ttatcattag tattgtacaa atggcacccg tttctgcaat ggttaggatg 7080

tacatcttct ttgcttcttt ctactacata tggaagagct atgttcatat catggatggt 7140

tgcacctctt cgacttgcat gatgtgctat aagcgcaatc gtgccacacg cgttgagtgt 7200

acaactattg ttaatggcat gaagagatct ttctatgtct atgcaaatgg aggccgtggc 7260

ttctgcaaga ctcacaattg gaattgtctc aattgtgaca cattttgcac tggtagtaca 7320

ttcattagtg atgaagttgc tcgtgatttg tcactccagt ttaaaagacc aatcaaccct 7380

actgaccagt catcgtatat tgttgatagt gttgctgtga aaaatggcgc gcttcacctc 7440

tactttgaca aggctggtca aaagacctat gagagacatc cgctctccca ttttgtcaat 7500

ttagacaatt tgagagctaa caacactaaa ggttcactgc ctattaatgt catagttttt 7560

gatggcaagt ccaaatgcga cgagtctgct tctaagtctg cttctgtgta ctacagtcag 7620

ctgatgtgcc aacctattct gttgcttgac caagttcttg tatcagacgt tggagatagt 7680

actgaagttt ccgttaagat gtttgatgct tatgtcgaca ccttttcagc aacttttagt 7740

gttcctatgg aaaaacttaa ggcacttgtt gctacagctc acagcgagtt agcaaagggt 7800

gtagctttag atggtgtcct ttctacattc gtgtcagctg cccgacaagg tgttgttgat 7860

accgatgttg acacaaagga tgttattgaa tgtctcaaac tttcacatca ctctgactta 7920

gaagtgacag gtgacagttg taacaatttc atgctcacct ataataaggt tgaaaacatg 7980

acgcccagag atcttggcgc atgtattgac tgtaatgcaa ggcatatcaa tgcccaagta 8040

gcaaaaagtc acaatgtttc actcatctgg aatgtaaaag actacatgtc tttatctgaa 8100

cagctgcgta aacaaattcg tagtgctgcc aagaagaaca acataccttt tagactaact 8160

tgtgctacaa ctagacaggt tgtcaatgtc ataactacta aaatctcact caagggtggt 8220

aagattgtta gtacttgttt taaacttatg cttaaggcca cattattgtg cgttcttgct 8280

gcattggttt gttatatcgt tatgccagta catacattgt caatccatga tggttacaca 8340

aatgaaatca ttggttacaa agccattcag gatggtgtca ctcgtgacat catttctact 8400

gatgattgtt ttgcaaataa acatgctggt tttgacgcat ggtttagcca gcgtggtggt 8460

tcatacaaaa atgacaaaag ctgccctgta gtagctgcta tcattacaag agagattggt 8520
ttcatagtgc ctggcttacc gggtactgtg ctgagagcaa tcaatggtga cttcttgcat 8580
tttctacctc gtgtttttag tgctgttggc aacatttgct acacaccttc caaactcatt 8640
gagtatagtg attttgctac ctctgcttgc gttcttgctg ctgagtgtac aatttttaag 8700
gatgctatgg gcaaacctgt gccatattgt tatgacacta atttgctaga gggttctatt 8760
tcttatagtg agcttcgtcc agacactcgt tatgtgctta tggatggttc catcatacag 8820
tttcctaaca cttacctgga gggttctgtt agagtagtaa caacttttga tgctgagtac 8880
tgtagacatg gtacatgcga aaggtcagaa gtaggtattt gcctatctac cagtggtaga 8940
tgggttctta ataatgagca ttacagagct ctatcaggag ttttctgtgg tgttgatgcg 9000
atgaatctca tagctaacat ctttactcct cttgtgcaac ctgtgggtgc tttagatgtg 9060
tctgcttcag tagtggctgg tggtattatt gccatattgg tgacttgtgc tgcctactac 9120
tttatgaaat tcagacgtgt ttttggtgag tacaaccatg ttgttgctgc taatgcactt 9180
ttgtttttga tgtctttcac tatactctgt ctggtaccag cttacagctt tctgccggga 9240
gtctactcag tcttttactt gtacttgaca ttctatttca ccaatgatgt ttcattcttg 9300
gctcaccttc aatggtttgc catgttttct cctattgtgc ctttttggat aacagcaatc 9360
tatgtattct gtatttctct gaagcactgc cattggttct ttaacaacta tcttaggaaa 9420
agagtcatgt ttaatggagt tacatttagt accttcgagg aggctgcttt gtgtaccttt 9480
ttgctcaaca aggaaatgta cctaaaattg cgtagcgaga cactgttgcc acttacacag 9540
tataacaggt atcttgctct atataacaag tacaagtatt tcagtggagc cttagatact 9600
accagctatc gtgaagcagc ttgctgccac ttagcaaagg ctctaaatga ctttagcaac 9660
tcaggtgctg atgttctcta ccaaccacca cagacatcaa tcacttctgc tgttctgcag 9720
agtggtttta ggaaaatggc attcccgtca ggcaaagttg aagggtgcat ggtacaagta 9780
acctgtggaa ctacaactct taatggattg tggttggatg acacagtata ctgtccaaga 9840
catgtcattt gcacagcaga agacatgctt aatcctaact atgaagatct gctcattcgc 9900
aaatccaacc atagctttct tgttcaggct ggcaatgttc aacttcgtgt tattggccat 9960
tctatgcaaa attgtctgct taggcttaaa gttgatactt ctaaccctaa gacacccaag 10020
tataaatttg tccgtatcca acctggtcaa acattttcag ttctagcatg ctacaatggt 10080
tcaccatctg gtgtttatca gtgtgccatg agacctaatc ataccattaa aggttctttc 10140
cttaatggat catgtggtag tgttggtttt aacattgatt atgattgcgt gtctttctgc 10200
tatatgcatc atatggagct tccaacagga gtacacgctg gtactgactt agaaggtaaa 10260

ttctatggtc catttgttga cagacaaact gcacaggctg caggtacaga cacaaccata 10320
acattaaatg ttttggcatg gctgtatgct gctgttatca atggtgatag gtggtttctt 10380
aatagattca ccactacttt gaatgacttt aaccttgtgg caatgaagta caactatgaa 10440
cctttgacac aagatcatgt tgacatattg ggacctcttt ctgctcaaac aggaattgcc 10500
gtcttagata tgtgtgctgc tttgaaagag ctgctgcaga atggtatgaa tggtcgtact 10560
atccttggta gcactatttt agaagatgag tttacaccat ttgatgttgt tagacaatgc 10620
tctggtgtta ccttccaagg taagttcaag aaaattgtta agggcactca tcattggatg 10680
cttttaactt tcttgacatc actattgatt cttgttcaaa gtacacagtg gtcactgttt 10740
ttctttgttt acgagaatgc tttcttgcca tttactcttg gtattatggc aattgctgca 10800
tgtgctatgc tgcttgttaa gcataagcac gcattcttgt gcttgtttct gttaccttct 10860
cttgcaacag ttgcttactt taatatggtc tacatgcctg ctagctgggt gatgcgtatc 10920
atgacatggc ttgaattggc tgacactagc ttgtctggtt ataggcttaa ggattgtgtt 10980
atgtatgctt cagctttagt tttgcttatt ctcatgacag ctcgcactgt ttatgatgat 11040
gctgctagac gtgtttggac actgatgaat gtcattacac ttgtttacaa agtctactat 11100
ggtaatgctt tagatcaagc tatttccatg tgggccttag ttatttctgt aacctctaac 11160
tattctggtg tcgttacgac tatcatgttt ttagctagag ctatagtgtt tgtgtgtgtt 11220
gagtattacc cattgttatt tattactggc aacaccttac agtgtatcat gcttgtttat 11280
tgtttcttag gctattgttg ctgctgctac tttggccttt tctgtttact caaccgttac 11340
ttcaggctta ctcttggtgt ttatgactac ttggtctcta cacaagaatt taggtatatg 11400
aactcccagg ggcttttgcc tcctaagagt agtattgatg ctttcaagct taacattaag 11460
ttgttgggta ttggaggtaa accatgtatc aaggttgcta ctgtacagtc taaaatgtct 11520
gacgtaaagt gcacatctgt ggtactgctc tcggttcttc aacaacttag agtagagtca 11580
tcttctaaat tgtgggcaca atgtgtacaa ctccacaatg atattcttct tgcaaaagac 11640
acaactgaag ctttcgagaa gatggtttct cttttgtctg ttttgctatc catgcagggt 11700
gctgtagaca ttaataggtt gtgcgaggaa atgctcgata accgtgctac tcttcaggct 11760
attgcttcag aatttagttc tttaccatca tatgccgctt atgccactgc ccaggaggcc 11820
tatgagcagg ctgtagctaa tggtgattct gaagtcgttc tcaaaaagtt aaagaaatct 11880
ttgaatgtgg ctaaatctga gtttgaccgt gatgctgcca tgcaacgcaa gttggaaaag 11940
atggcagatc aggctatgac ccaaatgtac aaacaggcaa gatctgagga caagagggca 12000
aaagtaacta gtgctatgca aacaatgctc ttcactatgc ttaggaagct tgataatgat 12060

gcacttaaca acattatcaa caatgcgcgt gatggttgtg ttccactcaa catcatacca 12120
ttgactacag cagccaaact catggttgtt gtccctgatt atggtaccta caagaacact 12180
tgtgatggta acacctttac atatgcatct gcactctggg aaatccagca agttgttgat 12240
gcggatagca agattgttca acttagtgaa attaacatgg acaattcacc aaatttggct 12300
tggcctctta ttgttacagc tctaagagcc aactcagctg ttaaactaca gaataatgaa 12360
ctgagtccag tagcactacg acagatgtcc tgtgcggctg gtaccacaca aacagcttgt 12420
actgatgaca atgcacttgc ctactataac aattcgaagg gaggtaggtt tgtgctggca 12480
ttactatcag accaccaaga tctcaaatgg gctagattcc ctaagagtga tggtacaggt 12540
acaatttaca cagaactgga accaccttgt aggtttgtta cagacacacc aaaagggcct 12600
aaagtgaaat acttgtactt catcaaaggc ttaaacaacc taaatagagg tatggtgctg 12660
ggcagtttag ctgctacagt acgtcttcag gctggaaatg ctacagaagt acctgccaat 12720
tcaactgtgc tttccttctg tgcttttgca gtagaccctg ctaaagcata taaggattac 12780
ctagcaagtg gaggacaacc aatcaccaac tgtgtgaaga tgttgtgtac acacactggt 12840
acaggacagg caattactgt aacaccagaa gctaacatgg accaagagtc ctttggtggt 12900
gcttcatgtt gtctgtattg tagatgccac attgaccatc caaatcctaa aggattctgt 12960
gacttgaaag gtaagtacgt ccaaatacct accacttgtg ctaatgaccc agtgggtttt 13020
acacttagaa acacagtctg taccgtctgc ggaatgtgga aaggttatgg ctgtagttgt 13080
gaccaactcc gcgaaccctt gatgcagtct gcggatgcat caacgttttt aaacgggttt 13140
gcggtgtaa 13149

<210> 2
<211> 8088
<212> DNA
<213> SARS-CoV

<400> 2
cgggtttgcg gtgtaagtgc agcccgtctt acaccgtgcg gcacaggcac tagtactgat 60
gtcgtctaca gggcttttga tatttacaac gaaaaagttg ctggttttgc aaagttccta 120
aaaactaatt gctgtcgctt ccaggagaag gatgaggaag gcaatttatt agactcttac 180
tttgtagtta agaggcatac tatgtctaac taccaacatg aagagactat ttataacttg 240
gttaaagatt gtccagcggt tgctgtccat gactttttca agtttagagt agatggtgac 300
atggtaccac atatatcacg tcagcgtcta actaaataca caatggctga tttagtctat 360
gctctacgtc attttgatga gggtaattgt gatacattaa aagaaatact cgtcacatac 420
aattgctgtg atgatgatta tttcaataag aaggattggt atgacttcgt agagaatcct 480

gacatcttac gcgtatatgc taacttaggt gagcgtgtac gccaatcatt attaaagact 540
gtacaattct gcgatgctat gcgtgatgca ggcattgtag gcgtactgac attagataat 600
caggatctta atgggaactg gtacgatttc ggtgatttcg tacaagtagc accaggctgc 660
ggagttccta ttgtggattc atattactca ttgctgatgc ccatcctcac tttgactagg 720
gcattggctg ctgagtccca tatggatgct gatctcgcaa aaccacttat taagtgggat 780
ttgctgaaat atgattttac ggaagagaga ctttgtctct tcgaccgtta ttttaaatat 840
tgggaccaga cataccatcc caattgtatt aactgtttgg atgataggtg tatccttcat 900
tgtgcaaact ttaatgtgtt attttctact gtgtttccac ctacaagttt tggaccacta 960
gtaagaaaaa tatttgtaga tggtgttcct tttgttgttt caactggata ccattttcgt 1020
gagttaggag tcgtacataa tcaggatgta aacttacata gctcgcgtct cagtttcaag 1080
gaacttttag tgtatgctgc tgatccagct atgcatgcag cttctggcaa tttattgcta 1140
gataaacgca ctacatgctt ttcagtagct gcactaacaa acaatgttgc ttttcaaact 1200
gtcaaacccg gtaattttaa taaagacttt tatgactttg ctgtgtctaa aggtttcttt 1260
aaggaaggaa gttctgttga actaaaacac ttcttctttg ctcaggatgg caacgctgct 1320
atcagtgatt atgactatta tcgttataat ctgccaacaa tgtgtgatat cagacaactc 1380
ctattcgtag ttgaagttgt tgataaatac tttgattgtt acgatggtgg ctgtattaat 1440
gccaaccaag taatcgttaa caatctggat aaatcagctg gtttcccatt taataaatgg 1500
ggtaaggcta gactttatta tgactcaatg agttatgagg atcaagatgc acttttcgcg 1560
tatactaagc gtaatgtcat ccctactata actcaaatga atcttaagta tgccattagt 1620
gcaaagaata gagctcgcac cgtagctggt gtctctatct gtagtactat gacaaataga 1680
cagtttcatc agaaattatt gaagtcaata gccgccacta gaggagctac tgtggtaatt 1740
ggaacaagca agttttacgg tggctggcat aatatgttaa aaactgttta cagtgatgta 1800
gaaactccac accttatggg ttgggattat ccaaaatgtg acagagccat gcctaacatg 1860
cttaggataa tggcctctct tgttcttgct cgcaaacata acacttgctg taacttatca 1920
caccgtttct acaggttagc taacgagtgt gcgcaagtat taagtgagat ggtcatgtgt 1980
ggcggctcac tatatgttaa accaggtgga acatcatccg gtgatgctac aactgcttat 2040
gctaatagtg tctttaacat ttgtcaagct gttacagcca atgtaaatgc acttctttca 2100
actgatggta ataagatagc tgacaagtat gtccgcaatc tacaacacag gctctatgag 2160
tgtctctata gaaataggga tgttgatcat gaattcgtgg atgagtttta cgcttacctg 2220
cgtaaacatt tctccatgat gattctttct gatgatgccg ttgtgtgcta taacagtaac 2280

tatgcggctc aaggtttagt agctagcatt aagaacttta aggcagttct ttattatcaa 2340

aataatgtgt tcatgtctga ggcaaaatgt tggactgaga ctgaccttac taaaggacct 2400

cacgaatttt gctcacagca tacaatgcta gttaaacaag gagatgatta cgtgtacctg 2460

ccttacccag atccatcaag aatattaggc gcaggctgtt ttgtcgatga tattgtcaaa 2520

acagatggta cacttatgat tgaaaggttc gtgtcactgg ctattgatgc ttacccactt 2580

acaaaacatc ctaatcagga gtatgctgat gtctttcact tgtatttaca atacattaga 2640

aagttacatg atgagcttac tggccacatg ttggacatgt attccgtaat gctaactaat 2700

gataacacct cacggtactg ggaacctgag ttttatgagg ctatgtacac accacataca 2760

gtcttgcagg ctgtaggtgc ttgtgtattg tgcaattcac agacttcact tcgttgcggt 2820

gcctgtatta ggagaccatt cctatgttgc aagtgctgct atgaccatgt catttcaaca 2880

tcacacaaat tagtgttgtc tgttaatccc tatgtttgca atgccccagg ttgtgatgtc 2940

actgatgtga cacaactgta tctaggaggt atgagctatt attgcaagtc acataagcct 3000

cccattagtt ttccattatg tgctaatggt caggtttttg gtttatacaa aaacacatgt 3060

gtaggcagtg acaatgtcac tgacttcaat gcgatagcaa catgtgattg gactaatgct 3120

ggcgattaca tacttgccaa cacttgtact gagagactca agcttttcgc agcagaaacg 3180

ctcaaagcca ctgaggaaac atttaagctg tcatatggta ttgctactgt acgcgaagta 3240

ctctctgaca gagaattgca tctttcatgg gaggttggaa aacctagacc accattgaac 3300

agaaactatg tctttactgg ttaccgtgta actaaaaata gtaaagtaca gattggagag 3360

tacacctttg aaaaaggtga ctatggtgat gctgttgtgt acagaggtac tacgacatac 3420

aagttgaatg ttggtgatta ctttgtgttg acatctcaca ctgtaatgcc acttagtgca 3480

cctactctag tgccacaaga gcactatgtg agaattactg gcttgtaccc aacactcaac 3540

atctcagatg agttttctag caatgttgca aattatcaaa aggtcggcat gcaaaagtac 3600

tctacactcc aaggaccacc tggtactggt aagagtcatt ttgccatcgg acttgctctc 3660

tattacccat ctgctcgcat agtgtatacg gcatgctctc atgcagctgt tgatgcccta 3720

tgtgaaaagg cattaaaata tttgcccata gataaatgta gtagaatcat acctgcgcgt 3780

gcgcgcgtag agtgttttga taaattcaaa gtgaattcaa cactagaaca gtatgttttc 3840

tgcactgtaa atgcattgcc agaaacaact gctgacattg tagtctttga tgaaatctct 3900

atggctacta attatgactt gagtgttgtc aatgctagac ttcgtgcaaa acactacgtc 3960

tatattggcg atcctgctca attaccagcc ccccgcacat tgctgactaa aggcacacta 4020

gaaccagaat attttaattc agtgtgcaga cttatgaaaa caataggtcc agacatgttc 4080

cttggaactt gtcgccgttg tcctgctgaa attgttgaca ctgtgagtgc tttagtttat 4140

gacaataagc taaaagcaca caaggataag tcagctcaat gcttcaaaat gttctacaaa 4200

ggtgttatta cacatgatgt ttcatctgca atcaacagac ctcaaatagg cgttgtaaga 4260

gaatttctta cacgcaatcc tgcttggaga aaagctgttt ttatctcacc ttataattca 4320

cagaacgctg tagcttcaaa aatcttagga ttgcctacgc agactgttga ttcatcacag 4380

ggttctgaat atgactatgt catattcaca caaactactg aaacagcaca ctcttgtaat 4440

gtcaaccgct tcaatgtggc tatcacaagg gcaaaaattg gcattttgtg cataatgtct 4500

gatagagatc tttatgacaa actgcaattt acaagtctag aaataccacg tcgcaatgtg 4560

gctacattac aagcagaaaa tgtaactgga ctttttaagg actgtagtaa gatcattact 4620

ggtcttcatc ctacacaggc acctacacac ctcagcgttg atataaagtt caagactgaa 4680

ggattatgtg ttgacatacc aggcatacca aaggacatga cctaccgtag actcatctct 4740

atgatgggtt tcaaaatgaa ttaccaagtc aatggttacc ctaatatgtt tatcacccgc 4800

gaagaagcta ttcgtcacgt tcgtgcgtgg attggctttg atgtagaggg ctgtcatgca 4860

actagagatg ctgtgggtac taacctacct ctccagctag gattttctac aggtgttaac 4920

ttagtagctg taccgactgg ttatgttgac actgaaaata acacagaatt caccagagtt 4980

aatgcaaaac ctccaccagg tgaccagttt aaacatctta taccactcat gtataaaggc 5040

ttgccctgga atgtagtgcg tattaagata gtacaaatgc tcagtgatac actgaaagga 5100

ttgtcagaca gagtcgtgtt cgtcctttgg gcgcatggct ttgagcttac atcaatgaag 5160

tactttgtca agattggacc tgaaagaacg tgttgtctgt gtgacaaacg tgcaacttgc 5220

ttttctactt catcagatac ttatgcctgc tggaatcatt ctgtgggttt tgactatgtc 5280

tataacccat ttatgattga tgttcagcag tggggcttta cgggtaacct tcagagtaac 5340

catgaccaac attgccaggt acatggaaat gcacatgtgg ctagttgtga tgctatcatg 5400

actagatgtt tagcagtcca tgagtgcttt gttaagcgcg ttgattggtc tgttgaatac 5460

cctattatag gagatgaact gagggttaat tctgcttgca gaaaagtaca acacatggtt 5520

gtgaagtctg cattgcttgc tgataagttt ccagttcttc atgacattgg aaatccaaag 5580

gctatcaagt gtgtgcctca ggctgaagta gaatggaagt tctacgatgc tcagccatgt 5640

agtgacaaag cttacaaaat agaggagctc ttctattctt atgctacaca tcacgataaa 5700

ttcactgatg gtgtttgttt gttttggaat tgtaacgttg atcgttaccc agccaatgca 5760

attgtgtgta ggtttgacac aagagtcttg tcaaacttga acttaccagg ctgtgatggt 5820

ggtagtttgt atgtgaataa gcatgcattc cacactccag ctttcgataa aagtgcattt 5880

actaatttaa agcaattgcc tttcttttac tattctgata gtccttgtga gtctcatggc 5940
aaacaagtag tgtcggatat tgattatgtt ccactcaaat ctgctacgtg tattacacga 6000
tgcaatttag gtggtgctgt ttgcagacac catgcaaatg agtaccgaca gtacttggat 6060
gcatataata tgatgatttc tgctggattt agcctatgga tttacaaaca atttgatact 6120
tataacctgt ggaatacatt taccaggtta cagagtttag aaaatgtggc ttataatgtt 6180
gttaataaag gacactttga tggacacgcc ggcgaagcac ctgtttccat cattaataat 6240
gctgtttaca caaaggtaga tggtattgat gtggagatct ttgaaaataa gacaacactt 6300
cctgttaatg ttgcatttga gctttgggct aagcgtaaca ttaaaccagt gccagagatt 6360
aagatactca ataatttggg tgttgatatc gctgctaata ctgtaatctg ggactacaaa 6420
agagaagccc cagcacatgt atctacaata ggtgtctgca caatgactga cattgccaag 6480
aaacctactg agagtgcttg ttcttcactt actgtcttgt ttgatggtag agtggaagga 6540
caggtagacc tttttagaaa cgcccgtaat ggtgttttaa taacagaagg ttcagtcaaa 6600
ggtctaacac cttcaaaggg accagcacaa gctagcgtca atggagtcac attaattgga 6660
gaatcagtaa aaacacagtt taactacttt aagaaagtag acggcattat tcaacagttg 6720
cctgaaacct actttactca gagcagagac ttagaggatt ttaagcccag atcacaaatg 6780
gaaactgact ttctcgagct cgctatggat gaattcatac agcgatataa gctcgagggc 6840
tatgccttcg aacacatcgt ttatggagat ttcagtcatg gacaacttgg cggtcttcat 6900
ttaatgatag gcttagccaa gcgctcacaa gattcaccac ttaaattaga ggattttatc 6960
cctatggaca gcacagtgaa aaattacttc ataacagatg cgcaaacagg ttcatcaaaa 7020
tgtgtgtgtt ctgtgattga tcttttactt gatgactttg tcgagataat aaagtcacaa 7080
gatttgtcag tgatttcaaa agtggtcaag gttacaattg actatgctga aatttcattc 7140
atgctttggt gtaaggatgg acatgttgaa accttctacc caaaactaca agcaagtcaa 7200
gcgtggcaac caggtgttgc gatgcctaac ttgtacaaga tgcaaagaat gcttcttgaa 7260
aagtgtgacc ttcagaatta tggtgaaaat gctgttatac caaaaggaat aatgatgaat 7320
gtcgcaaagt atactcaact gtgtcaatac ttaaatacac ttactttagc tgtaccctac 7380
aacatgagag ttattcactt tggtgctggc tctgataaag gagttgcacc aggtacagct 7440
gtgctcagac aatggttgcc aactggcaca ctacttgtcg attcagatct taatgacttc 7500
gtctccgacg cagattctac tttaattgga gactgtgcaa cagtacatac ggctaataaa 7560
tgggacctta ttattagcga tatgtatgac cctaggacca aacatgtgac aaaagagaat 7620
gactctaaag aagggttttt cacttatctg tgtggattta taaagcaaaa actagccctg 7680

ggtggttcta tagctgtaaa gataacagag cattcttgga atgctgacct ttacaagctt 7740

atgggccatt tctcatggtg gacagctttt gttacaaatg taaatgcatc atcatcggaa 7800

gcatttttaa ttggggctaa ctatcttggc aagccgaagg aacaaattga tggctatacc 7860

atgcatgcta actacatttt ctggaggaac acaaatccta tccagttgtc ttcctattca 7920

ctctttgaca tgagcaaatt tcctcttaaa ttaagaggaa ctgctgtaat gtctcttaag 7980

gagaatcaaa tcaatgatat gatttattct cttctggaaa aaggtaggct tatcattaga 8040

gaaaacaaca gagttgtggt ttcaagtgat attcttgtta acaactaa 8088

<210> 3
<211> 1269
<212> DNA
<213> SARS-CoV

<400> 3
atgtctgata atggacccca atcaaaccaa cgtagtgccc cccgcattac atttggtgga 60

cccacagatt caactgacaa taaccagaat ggaggacgca atggggcaag gccaaaacag 120

cgccgacccc aaggtttacc caataatact gcgtcttggt tcacagctct cactcagcat 180

ggcaaggagg aacttagatt ccctcgaggc cagggcgttc caatcaacac caatagtggt 240

ccagatgacc aaattggcta ctaccgaaga gctacccgac gagttcgtgg tggtgacggc 300

aaaatgaaag agctcagccc cagatggtac ttctattacc taggaactgg cccagaagct 360

tcacttccct acggcgctaa caaagaaggc atcgtatggg ttgcaactga gggagccttg 420

aatacaccca aagaccacat tggcacccgc aatcctaata acaatgctgc caccgtgcta 480

caacttcctc aaggaacaac attgccaaaa ggcttctacg cagagggaag cagaggcggc 540

agtcaagcct cttctcgctc ctcatcacgt agtcgcggta attcaagaaa ttcaactcct 600

ggcagcagta ggggaaattc tcctgctcga atggctagcg gaggtggtga aactgccctc 660

gcgctattgc tgctagacag attgaaccag cttgagagca aagtttctgg taaaggccaa 720

caacaacaag gccaaactgt cactaagaaa tctgctgctg aggcatctaa aaagcctcgc 780

caaaaacgta ctgccacaaa acagtacaac gtcactcaag catttgggag acgtggtcca 840

gaacaaaccc aaggaaattt cggggaccaa gacctaatca gacaaggaac tgattacaaa 900

cattggccgc aaattgcaca atttgctcca agtgcctctg cattctttgg aatgtcacgc 960

attggcatgg aagtcacacc ttcgggaaca tggctgactt atcatggagc cattaaattg 1020

gatgacaaag atccacaatt caaagacaac gtcatactgc tgaacaagca cattgacgca 1080

tacaaaacat tcccaccaac agagcctaaa aaggacaaaa agaaaaagac tgatgaagct 1140

cagcctttgc cgcagagaca aaagaagcag cccactgtga ctcttcttcc tgcggctgac 1200

atggatgatt tctccagaca acttcaaaat tccatgagtg gagcttctgc tgattcaact 1260

caggcataa 1269

<210> 4
<211> 3768
<212> DNA
<213> SARS-CoV

<400> 4
atgtttattt tcttattatt tcttactctc actagtggta gtgaccttga ccggtgcacc 60

acttttgatg atgttcaagc tcctaattac actcaacata cttcatctat gaggggggtt 120

tactatcctg atgaaatttt tagatcagac actctttatt taactcagga tttatttctt 180

ccattttatt ctaatgttac agggtttcat actattaatc atacgtttgg caaccctgtc 240

atacctttta aggatggtat ttattttgct gccacagaga aatcaaatgt tgtccgtggt 300

tgggtttttg gttctaccat gaacaacaag tcacagtcgg tgattattat taacaattct 360

actaatgttg ttatacgagc atgtaacttt gaattgtgtg acaacccttt ctttgctgtt 420

tctaaaccca tgggtacaca gacacatact atgatattcg ataatgcatt taattgcact 480

ttcgagtaca tatctgatgc cttttcgctt gatgtttcag aaaagtcagg taattttaaa 540

cacttacgag agtttgtgtt taaaaataaa gatgggtttc tctatgttta taagggctat 600

caacctatag atgtagttcg tgatctacct tctggtttta acactttgaa acctattttt 660

aagttgcctc ttggtattaa cattacaaat tttagagcca ttcttacagc cttttcacct 720

gctcaagaca tttggggcac gtcagctgca gcctattttg ttggctattt aaagccaact 780

acatttatgc tcaagtatga tgaaaatggt acaatcacag atgctgttga ttgttctcaa 840

aatccacttg ctgaactcaa atgctctgtt aagagctttg agattgacaa aggaatttac 900

cagacctcta atttcagggt tgttccctca ggagatgttg tgagattccc taatattaca 960

aacttgtgtc cttttggaga ggtttttaat gctactaaat tcccttctgt ctatgcatgg 1020

gagagaaaaa aaatttctaa ttgtgttgct gattactctg tgctctacaa ctcaacattt 1080

ttttcaacct ttaagtgcta tggcgtttct gccactaagt tgaatgatct ttgcttctcc 1140

aatgtctatg cagattcttt tgtagtcaag ggagatgatg taagacaaat agcgccagga 1200

caaactggtg ttattgctga ttataattat aaattgccag atgatttcat gggttgtgtc 1260

cttgcttgga atactaggaa cattgatgct acttcaactg gtaattataa ttataaatat 1320

aggtatctta gacatggcaa gcttaggccc tttgagagag acatatctaa tgtgcctttc 1380

tcccctgatg gcaaaccttg caccccacct gctcttaatt gttattggcc attaaatgat 1440

tatggttttt acaccactac tggcattggc taccaacctt acagagttgt agtactttct 1500

tttgaacttt taaatgcacc ggccacggtt tgtggaccaa aattatccac tgaccttatt 1560

aagaaccagt gtgtcaattt taattttaat ggactcactg gtactggtgt gttaactcct 1620

tcttcaaaga gatttcaacc atttcaacaa tttggccgtg atgtttctga tttcactgat 1680

tccgttcgag atcctaaaac atctgaaata ttagacattt caccttgctc ttttgggggt 1740

gtaagtgtaa ttacacctgg aacaaatgct tcatctgaag ttgctgttct atatcaagat 1800

gttaactgca ctgatgtttc tacagcaatt catgcagatc aactcacacc agcttggcgc 1860

atatattcta ctggaaacaa tgtattccag actcaagcag gctgtcttat aggagctgag 1920

catgtcgaca cttcttatga gtgcgacatt cctattggag ctggcatttg tgctagttac 1980

catacagttt ctttattacg tagtactagc caaaaatcta ttgtggctta tactatgtct 2040

ttaggtgctg atagttcaat tgcttactct aataacacca ttgctatacc tactaacttt 2100

tcaattagca ttactacaga agtaatgcct gtttctatgg ctaaaacctc cgtagattgt 2160

aatatgtaca tctgcggaga ttctactgaa tgtgctaatt tgcttctcca atatggtagc 2220

ttttgcacac aactaaatcg tgcactctca ggtattgctg ctgaacagga tcgcaacaca 2280

cgtgaagtgt tcgctcaagt caaacaaatg tacaaaaccc caactttgaa atattttggt 2340

ggttttaatt tttcacaaat attacctgac cctctaaagc caactaagag gtcttttatt 2400

gaggacttgc tctttaataa ggtgacactc gctgatgctg gcttcatgaa gcaatatggc 2460

gaatgcctag gtgatattaa tgctagagat ctcatttgtg cgcagaagtt caatggactt 2520

acagtgttgc cacctctgct cactgatgat atgattgctg cctacactgc tgctctagtt 2580

agtggtactg ccactgctgg atggacattt ggtgctggcg ctgctcttca aatacctttt 2640

gctatgcaaa tggcatatag gttcaatggc attggagtta cccaaaatgt tctctatgag 2700

aaccaaaaac aaatcgccaa ccaatttaac aaggcgatta gtcaaattca agaatcactt 2760

acaacaacat caactgcatt gggcaagctg caagacgttg ttaaccagaa tgctcaagca 2820

ttaaacacac ttgttaaaca acttagctct aattttggtg caatttcaag tgtgctaaat 2880

gatatccttt cgcgacttga taaagtcgag gcggaggtac aaattgacag gttaattaca 2940

ggcagacttc aaagccttca aacctatgta acacaacaac taatcagggc tgctgaaatc 3000

agggcttctg ctaatcttgc tgctactaaa atgtctgagt gtgttcttgg acaatcaaaa 3060

agagttgact tttgtggaaa gggctaccac cttatgtcct tcccacaagc agccccgcat 3120

ggtgttgtct tcctacatgt cacgtatgtg ccatcccagg agaggaactt caccacagcg 3180

ccagcaattt gtcatgaagg caaagcatac ttccctcgtg aaggtgtttt tgtgtttaat 3240

ggcacttctt ggtttattac acagaggaac ttcttttctc cacaaataat tactacagac 3300

aatacatttg tctcaggaaa ttgtgatgtc gttattggca tcattaacaa cacagtttat 3360

gatcctctgc aacctgagct cgactcattc aaagaagagc tggacaagta cttcaaaaat 3420

catacatcac cagatgttga tcttggcgac atttcaggca ttaacgcttc tgtcgtcaac 3480

attcaaaaag aaattgaccg cctcaatgag gtcgctaaaa atttaaatga atcactcatt 3540

gaccttcaag aattgggaaa atatgagcaa tatattaaat ggccttggta tgtttggctc 3600

ggcttcattg ctggactaat tgccatcgtc atggttacaa tcttgctttg ttgcatgact 3660

agttgttgca gttgcctcaa gggtgcatgc tcttgtggtt cttgctgcaa gtttgatgag 3720

gatgactctg agccagttct caagggtgtc aaattacatt acacataa 3768

<210> 5
<211> 666
<212> DNA
<213> SARS-CoV

<400> 5
atggcagaca acggtactat taccgttgag gagcttaaac aactcctgga acaatggaac 60

ctagtaatag gtttcctatt cctagcctgg attatgttac tacaatttgc ctattctaat 120

cggaacaggt ttttgtacat aataaagctt gttttcctct ggctcttgtg gccagtaaca 180

cttgcttgtt ttgtgcttgc tgctgtctac agaattaatt gggtgactgg cgggattgcg 240

attgcaatgg cttgtattgt aggcttgatg tggcttagct acttcgttgc ttccttcagg 300

ctgtttgctc gtacccgctc aatgtggtca ttcaacccag aaacaaacat tcttctcaat 360

gtgcctctcc gggggacaat tgtgaccaga ccgctcatgg aaagtgaact tgtcattggt 420

gctgtgatca ttcgtggtca cttgcgaatg gccggacacc ccctagggcg ctgtgacatt 480

aaggacctgc caaaagagat cactgtggct acatcacgaa cgctttctta ttacaaatta 540

ggagcgtcgc agcgtgtagg cactgattca ggttttgctg catacaaccg ctaccgtatt 600

ggaaactata aattaaatac agaccacgcc ggtagcaacg acaatattgc tttgctagta 660

cagtaa 666

<210> 6
<211> 231
<212> DNA
<213> SARS-CoV

<400> 6
atgtactcat tcgtttcgga agaaacaggt acgttaatag ttaatagcgt acttcttttt 60

cttgctttcg tggtattctt gctagtcaca ctagccatcc ttactgcgct tcgattgtgt 120

gcgtactgct gcaatattgt taacgtgagt ttagtaaaac caacggttta cgtctactcg 180

cgtgttaaaa atctgaactc ttctgaagga gttcctgatc ttctggtcta a 231

<210> 7
<211> 22
<212> DNA
<213> Artificial Sequence
<220>
<223> oligonucleotide primer

<400> 7
tgcttccctc tgcgtagaag cc 22

<210> 8
<211> 22
<212> DNA
<213> Artificial Sequence
<220>
<223> oligonucleotide primer

<400> 8
gccaaccaac ctcgatctct tg 22

<210> 9
<211> 21
<212> DNA
<213> Artificial Sequence
<220>
<223> oligonucleotide primer

<400> 9
tgggtccacc aaatgtaatg c 21

<210> 10
<211> 21
<212> DNA
<213> Artificial Sequence
<220>
<223> oligonucleotide primer

<400> 10
aagccaacca acctcgatct c 21

<210> 11
<211> 37971
<212> DNA
<213> SARS-CoV

<400> 11
   atattaggtt tttacctacc caggaaaagc caaccaacct cgatctcttg tagatctgtt 60
   ctctaaacga actttaaaat ctgtgtagct gtcgctcggc tgcatgccta gtgcacctac 120
   gcagtataaa caataataaa ttttactgtc gttgacaaga aacgagtaac tcgtccctct 180
   tctgcagact gcttacggtt tcgtccgtgt tgcagtcgat catcagcata cctaggtttc 240
   gtccgggtgt gaccgaaagg taagatggag agccttgttc ttggtgtcaa cgagaaaaca 300
   cacgtccaac tcagtttgcc tgtccttcag gttagagacg tgctagtgcg tggcttcggg 360
   gactctgtgg aagaggccct atcggaggca cgtgaacacc tcaaaaatgg cacttgtggt 420
   ctagtagagc tggaaaaagg cgtactgccc cagcttgaac agccctatgt gttcattaaa 480
   cgttctgatg ccttaagcac caatcacggc cacaaggtcg ttgagctggt tgcagaaatg 540
   gacggcattc agtacggtcg tagcggtata acactgggag tactcgtgcc acatgtgggc 600
   gaaaccccaa ttgcataccg caatgttctt cttcgtaaga acggtaataa gggagccggt 660
   ggtcatagct atggcatcga tctaaagtct tatgacttag gtgacgagct tggcactgat 720
   cccattgaag attatgaaca aaactggaac actaagcatg gcagtggtgc actccgtgaa 780
   ctcactcgtg agctcaatgg aggtgcagtc actcgctatg tcgacaacaa tttctgtggc 840
   ccagatgggt accctcttga ttgcatcaaa gattttctcg cacgcgcggg caagtcaatg 900
   tgcactcttt ccgaacaact tgattacatc gagtcgaaga gaggtgtcta ctgctgccgt 960
   gaccatgagc atgaaattgc ctggttcact gagcgctctg ataagagcta cgagcaccag 1020
   acacccttcg aaattaagag tgccaagaaa tttgacactt tcaaagggga atgcccaaag 1080
   tttgtgtttc ctcttaactc aaaagtcaaa gtcattcaac cacgtgttga aaagaaaaag 1140
   actgagggtt tcatggggcg tatacgctct gtgtaccctg ttgcatctcc acaggagtgt 1200
   aacaatatgc acttgtctac cttgatgaaa tgtaatcatt gcgatgaagt ttcatggcag 1260
   acgtgcgact ttctgaaagc cacttgtgaa cattgtggca ctgaaaattt agttattgaa 1320
   ggacctacta catgtgggta cctacctact aatgctgtag tgaaaatgcc atgtcctgcc 1380
   tgtcaagacc cagagattgg acctgagcat agtgttgcag attatcacaa ccactcaaac 1440
   attgaaactc gactccgcaa gggaggtagg actagatgtt ttggaggctg tgtgtttgcc 1500
   tatgttggct gctataataa gcgtgcctac tgggttcctc gtgctagtgc tgatattggc 1560
   tcaggccata ctggcattac tggtgacaat gtggagacct tgaatgagga tctccttgag 1620
   atactgagtc gtgaacgtgt taacattaac attgttggcg attttcattt gaatgaagag 1680
   gttgccatca ttttggcatc tttctctgct tctacaagtg cctttattga cactataaag 1740
   agtcttgatt acaagtcttt caaaaccatt gttgagtcct gcggtaacta taaagttacc 1800
   aagggaaagc ccgtaaaagg tgcttggaac attggacaac agagatcagt tttaacacca 1860
   ctgtgtggtt ttccctcaca ggctgctggt gttatcagat caatttttgc gcgcacactt 1920
   gatgcagcaa accactcaat tcctgatttg caaagagcag ctgtcaccat acttgatggt 1980
   atttctgaac agtcattacg tcttgtcgac gccatggttt atacttcaga cctgctcacc 2040
   aacagtgtca ttattatggc atatgtaact ggtggtcttg tacaacagac ttctcagtgg 2100
   ttgtctaatc ttttgggcac tactgttgaa aaactcaggc ctatctttga atggattgag 2160
   gcgaaactta gtgcaggagt tgaatttctc aaggatgctt gggagattct caaatttctc 2220
   attacaggtg tttttgacat cgtcaagggt caaatacagg ttgcttcaga taacatcaag 2280
   gattgtgtaa aatgcttcat tgatgttgtt aacaaggcac tcgaaatgtg cattgatcaa 2340
   gtcactatcg ctggcgcaaa gttgcgatca ctcaacttag gtgaagtctt catcgctcaa 2400
   agcaagggac tttaccgtca gtgtatacgt ggcaaggagc agctgcaact actcatgcct 2460
   cttaaggcac caaaagaagt aacctttctt gaaggtgatt cacatgacac agtacttacc 2520
   tctgaggagg ttgttctcaa gaacggtgaa ctcgaagcac tcgagacgcc cgttgatagc 2580
   ttcacaaatg gagctatcgt tggcacacca gtctgtgtaa atggcctcat gctcttagag 2640
   attaaggaca aagaacaata ctgcgcattg tctcctggtt tactggctac aaacaatgtc 2700
   tttcgcttaa aggggggtgc accaattaaa ggtgtaacct ttggagaaga tactgtttgg 2760
   gaagttcaag gttacaagaa tgtgagaatc acatttgagc ttgatgaacg tgttgacaaa 2820
   gtgcttaatg aaaagtgctc tgtctacact gttgaatccg gtaccgaagt tactgagttt 2880
   gcatgtgttg tagcagaggc tgttgtgaag actttacaac cagtttctga tctccttacc 2940
   aacatgggta ttgatcttga tgagtggagt gtagctacat tctacttatt tgatgatgct 3000
   ggtgaagaaa acttttcatc acgtatgtat tgttcctttt accctccaga tgaggaagaa 3060
   gaggacgatg cagagtgtga ggaagaagaa attgatgaaa cctgtgaaca tgagtacggt 3120
   acagaggatg attatcaagg tctccctctg gaatttggtg cctcagctga aacagttcga 3180
   gttgaggaag aagaagagga agactggctg gatgatacta ctgagcaatc agagattgag 3240
   ccagaaccag aacctacacc tgaagaacca gttaatcagt ttactggtta tttaaaactt 3300
   actgacaatg ttgccattaa atgtgttgac atcgttaagg aggcacaaag tgctaatcct 3360
   atggtgattg taaatgctgc taacatacac ctgaaacatg gtggtggtgt agcaggtgca 3420
   ctcaacaagg caaccaatgg tgccatgcaa aaggagagtg atgattacat taagctaaat 3480
   ggccctctta cagtaggagg gtcttgtttg ctttctggac ataatcttgc taagaagtgt 3540
   ctgcatgttg ttggacctaa cctaaatgca ggtgaggaca tccagcttct taaggcagca 3600
   tatgaaaatt tcaattcaca ggacatctta cttgcaccat tgttgtcagc aggcatattt 3660
   ggtgctaaac cacttcagtc tttacaagtg tgcgtgcaga cggttcgtac acaggtttat 3720
   attgcagtca atgacaaagc tctttatgag caggttgtca tggattatct tgataacctg 3780
   aagcctagag tggaagcacc taaacaagag gagccaccaa acacagaaga ttccaaaact 3840
   gaggagaaat ctgtcgtaca gaagcctgtc gatgtgaagc caaaaattaa ggcctgcatt 3900
   gatgaggtta ccacaacact ggaagaaact aagtttctta ccaataagtt actcttgttt 3960
   gctgatatca atggtaagct ttaccatgat tctcagaaca tgcttagagg tgaagatatg 4020
   tctttccttg agaaggatgc accttacatg gtaggtgatg ttatcactag tggtgatatc 4080
   acttgtgttg taataccctc caaaaaggct ggtggcacta ctgagatgct ctcaagagct 4140
   ttgaagaaag tgccagttga tgagtatata accacgtacc ctggacaagg atgtgctggt 4200
   tatacacttg aggaagctaa gactgctctt aagaaatgca aatctgcatt ttatgtacta 4260
   ccttcagaag cacctaatgc taaggaagag attctaggaa ctgtatcctg gaatttgaga 4320
   gaaatgcttg ctcatgctga agagacaaga aaattaatgc ctatatgcat ggatgttaga 4380
   gccataatgg caaccatcca acgtaagtat aaaggaatta aaattcaaga gggcatcgtt 4440
   gactatggtg tccgattctt cttttatact agtaaagagc ctgtagcttc tattattacg 4500
   aagctgaact ctctaaatga gccgcttgtc acaatgccaa ttggttatgt gacacatggt 4560
   tttaatcttg aagaggctgc gcgctgtatg cgttctctta aagctcctgc cgtagtgtca 4620
   gtatcatcac cagatgctgt tactacatat aatggatacc tcacttcgtc atcaaagaca 4680
   tctgaggagc actttgtaga aacagtttct ttggctggct cttacagaga ttggtcctat 4740
   tcaggacagc gtacagagtt aggtgttgaa tttcttaagc gtggtgacaa aattgtgtac 4800
   cacactctgg agagccccgt cgagtttcat cttgacggtg aggttctttc acttgacaaa 4860
   ctaaagagtc tcttatccct gcgggaggtt aagactataa aagtgttcac aactgtggac 4920
   aacactaatc tccacacaca gcttgtggat atgtctatga catatggaca gcagtttggt 4980
   ccaacatact tggatggtgc tgatgttaca aaaattaaac ctcatgtaaa tcatgagggt 5040
   aagactttct ttgtactacc tagtgatgac acactacgta gtgaagcttt cgagtactac 5100
   catactcttg atgagagttt tcttggtagg tacatgtctg ctttaaacca cacaaagaaa 5160
   tggaaatttc ctcaagttgg tggtttaact tcaattaaat gggctgataa caattgttat 5220
   ttgtctagtg ttttattagc acttcaacag cttgaagtca aattcaatgc accagcactt 5280
   caagaggctt attatagagc ccgtgctggt gatgctgcta acttttgtgc actcatactc 5340
   gcttacagta ataaaactgt tggcgagctt ggtgatgtca gagaaactat gacccatctt 5400
   ctacagcatg ctaatttgga atctgcaaag cgagttctta atgtggtgtg taaacattgt 5460
   ggtcagaaaa ctactacctt aacgggtgta gaagctgtga tgtatatggg tactctatct 5520
   tatgataatc ttaagacagg tgtttccatt ccatgtgtgt gtggtcgtga tgctacacaa 5580
   tatctagtac aacaagagtc ttcttttgtt atgatgtctg caccacctgc tgagtataaa 5640
   ttacagcaag gtacattctt atgtgcgaat gagtacactg gtaactatca gtgtggtcat 5700
   tacactcata taactgctaa ggagaccctc tatcgtattg acggagctca ccttacaaag 5760
   atgtcagagt acaaaggacc agtgactgat gttttctaca aggaaacatc ttacactaca 5820
   accatcaagc ctgtgtcgta taaactcgat ggagttactt acacagagat tgaaccaaaa 5880
   ttggatgggt attataaaaa ggataatgct tactatacag agcagcctat agaccttgta 5940
   ccaactcaac cattaccaaa tgcgagtttt gataatttca aactcacatg ttctaacaca 6000
   aaatttgctg atgatttaaa tcaaatgaca ggcttcacaa agccagcttc acgagagcta 6060
   tctgtcacat tcttcccaga cttgaatggc gatgtagtgg ctattgacta tagacactat 6120
   tcagcgagtt tcaagaaagg tgctaaatta ctgcataagc caattgtttg gcacattaac 6180
   caggctacaa ccaagacaac gttcaaacca aacacttggt gtttacgttg tctttggagt 6240
   acaaagccag tagatacttc aaattcattt gaagttctgg cagtagaaga cacacaagga 6300
   atggacaatc ttgcttgtga aagtcaacaa cccacctctg aagaagtagt ggaaaatcct 6360
   accatacaga aggaagtcat agagtgtgac gtgaaaacta ccgaagttgt aggcaatgtc 6420
   atacttaaac catcagatga aggtgttaaa gtaacacaag agttaggtca tgaggatctt 6480
   atggctgctt atgtggaaaa cacaagcatt accattaaga aacctaatga gctttcacta 6540
   gccttaggtt taaaaacaat tgccactcat ggtattgctg caattaatag tgttccttgg 6600
   agtaaaattt tggcttatgt caaaccattc ttaggacaag cagcaattac aacatcaaat 6660
   tgcgctaaga gattagcaca acgtgtgttt aacaattata tgccttatgt gtttacatta 6720
   ttgttccaat tgtgtacttt tactaaaagt accaattcta gaattagagc ttcactacct 6780
   acaactattg ctaaaaatag tgttaagagt gttgctaaat tatgtttgga tgccggcatt 6840
   aattatgtga agtcacccaa attttctaaa ttgttcacaa tcgctatgtg gctattgttg 6900
   ttaagtattt gcttaggttc tctaatctgt gtaactgctg cttttggtgt actcttatct 6960
   aattttggtg ctccttctta ttgtaatggc gttagagaat tgtatcttaa ttcgtctaac 7020
   gttactacta tggatttctg tgaaggttct tttccttgca gcatttgttt aagtggatta 7080
   gactcccttg attcttatcc agctcttgaa accattcagg tgacgatttc atcgtacaag 7140
   ctagacttga caattttagg tctggccgct gagtgggttt tggcatatat gttgttcaca 7200
   aaattctttt atttattagg tctttcagct ataatgcagg tgttctttgg ctattttgct 7260
   agtcatttca tcagcaattc ttggctcatg tggtttatca ttagtattgt acaaatggca 7320
   cccgtttctg caatggttag gatgtacatc ttctttgctt ctttctacta catatggaag 7380
   agctatgttc atatcatgga tggttgcacc tcttcgactt gcatgatgtg ctataagcgc 7440
   aatcgtgcca cacgcgttga gtgtacaact attgttaatg gcatgaagag atctttctat 7500
   gtctatgcaa atggaggccg tggcttctgc aagactcaca attggaattg tctcaattgt 7560
   gacacatttt gcactggtag tacattcatt agtgatgaag ttgctcgtga tttgtcactc 7620
   cagtttaaaa gaccaatcaa ccctactgac cagtcatcgt atattgttga tagtgttgct 7680
   gtgaaaaatg gcgcgcttca cctctacttt gacaaggctg gtcaaaagac ctatgagaga 7740
   catccgctct cccattttgt caatttagac aatttgagag ctaacaacac taaaggttca 7800
   ctgcctatta atgtcatagt ttttgatggc aagtccaaat gcgacgagtc tgcttctaag 7860
   tctgcttctg tgtactacag tcagctgatg tgccaaccta ttctgttgct tgaccaagtt 7920
   cttgtatcag acgttggaga tagtactgaa gtttccgtta agatgtttga tgcttatgtc 7980
   gacacctttt cagcaacttt tagtgttcct atggaaaaac ttaaggcact tgttgctaca 8040
   gctcacagcg agttagcaaa gggtgtagct ttagatggtg tcctttctac attcgtgtca 8100
   gctgcccgac aaggtgttgt tgataccgat gttgacacaa aggatgttat tgaatgtctc 8160
   aaactttcac atcactctga cttagaagtg acaggtgaca gttgtaacaa tttcatgctc 8220
   acctataata aggttgaaaa catgacgccc agagatcttg gcgcatgtat tgactgtaat 8280
   gcaaggcata tcaatgccca agtagcaaaa agtcacaatg tttcactcat ctggaatgta 8340
   aaagactaca tgtctttatc tgaacagctg cgtaaacaaa ttcgtagtgc tgccaagaag 8400
   aacaacatac cttttagact aacttgtgct acaactagac aggttgtcaa tgtcataact 8460
   actaaaatct cactcaaggg tggtaagatt gttagtactt gttttaaact tatgcttaag 8520
   gccacattat tgtgcgttct tgctgcattg gtttgttata tcgttatgcc agtacataca 8580
   ttgtcaatcc atgatggtta cacaaatgaa atcattggtt acaaagccat tcaggatggt 8640
   gtcactcgtg acatcatttc tactgatgat tgttttgcaa ataaacatgc tggttttgac 8700
   gcatggttta gccagcgtgg tggttcatac aaaaatgaca aaagctgccc tgtagtagct 8760
   gctatcatta caagagagat tggtttcata gtgcctggct taccgggtac tgtgctgaga 8820
   gcaatcaatg gtgacttctt gcattttcta cctcgtgttt ttagtgctgt tggcaacatt 8880
   tgctacacac cttccaaact cattgagtat agtgattttg ctacctctgc ttgcgttctt 8940
   gctgctgagt gtacaatttt taaggatgct atgggcaaac ctgtgccata ttgttatgac 9000
   actaatttgc tagagggttc tatttcttat agtgagcttc gtccagacac tcgttatgtg 9060
   cttatggatg gttccatcat acagtttcct aacacttacc tggagggttc tgttagagta 9120
   gtaacaactt ttgatgctga gtactgtaga catggtacat gcgaaaggtc agaagtaggt 9180
   atttgcctat ctaccagtgg tagatgggtt cttaataatg agcattacag agctctatca 9240
   ggagttttct gtggtgttga tgcgatgaat ctcatagcta acatctttac tcctcttgtg 9300
   caacctgtgg gtgctttaga tgtgtctgct tcagtagtgg ctggtggtat tattgccata 9360
   ttggtgactt gtgctgccta ctactttatg aaattcagac gtgtttttgg tgagtacaac 9420
   catgttgttg ctgctaatgc acttttgttt ttgatgtctt tcactatact ctgtctggta 9480
   ccagcttaca gctttctgcc gggagtctac tcagtctttt acttgtactt gacattctat 9540
   ttcaccaatg atgtttcatt cttggctcac cttcaatggt ttgccatgtt ttctcctatt 9600
   gtgccttttt ggataacagc aatctatgta ttctgtattt ctctgaagca ctgccattgg 9660
   ttctttaaca actatcttag gaaaagagtc atgtttaatg gagttacatt tagtaccttc 9720
   gaggaggctg ctttgtgtac ctttttgctc aacaaggaaa tgtacctaaa attgcgtagc 9780
   gagacactgt tgccacttac acagtataac aggtatcttg ctctatataa caagtacaag 9840
   tatttcagtg gagccttaga tactaccagc tatcgtgaag cagcttgctg ccacttagca 9900
   aaggctctaa atgactttag caactcaggt gctgatgttc tctaccaacc accacagaca 9960
   tcaatcactt ctgctgttct gcagagtggt tttaggaaaa tggcattccc gtcaggcaaa 10020
   gttgaagggt gcatggtaca agtaacctgt ggaactacaa ctcttaatgg attgtggttg 10080
   gatgacacag tatactgtcc aagacatgtc atttgcacag cagaagacat gcttaatcct 10140
   aactatgaag atctgctcat tcgcaaatcc aaccatagct ttcttgttca ggctggcaat 10200
   gttcaacttc gtgttattgg ccattctatg caaaattgtc tgcttaggct taaagttgat 10260
   acttctaacc ctaagacacc caagtataaa tttgtccgta tccaacctgg tcaaacattt 10320
   tcagttctag catgctataa tggttcacca tctggtgttt atcagtgtgc catgagacct 10380
   aatcatacca ttaaaggttc tttccttaat ggatcatgtg gtagtgttgg ttttaacatt 10440
   gattatgatt gcgtgtcttt ctgctatatg catcatatgg agcttccaac aggagtacac 10500
   gctggtactg acttagaagg taaattctat ggtccatttg ttgacagaca aactgcacag 10560
   gctgcaggta cagacacaac cataacatta aatgttttgg catggctgta tgctgctgtt 10620
   atcaatggtg ataggtggtt tcttaataga ttcaccacta ctttgaatga ctttaacctt 10680
   gtggcaatga agtacaacta tgaacctttg acacaagatc atgttgacat attgggacct 10740
   ctttctgctc aaacaggaat tgccgtctta gatatgtgtg ctgctttgaa agagctgctg 10800
   cagaatggta tgaatggtcg tactatcctt ggtagcacta ttttagaaga tgagtttaca 10860
   ccatttgatg ttgttagaca atgctctggt gttaccttcc aaggtaagtt caagaaaatt 10920
   gttaagggca ctcatcattg gatgctttta actttcttga catcactatt gattcttgtt 10980
   caaagtacac agtggtcact gtttttcttt gtttacgaga atgctttctt gccatttact 11040
   cttggtatta tggcaattgc tgcatgtgct atgctgcttg ttaagcataa gcacgcattc 11100
   ttgtgcttgt ttctgttacc ttctcttgca acagttgctt actttaatat ggtctacatg 11160
   ccagctagct gggtgatgcg tatcatgaca tggcttgaat tggctgacac tagcttgtct 11220
   ggttataggc ttaaggattg tgttatgtat gcttcagctt tagttttgct tattctcatg 11280
   acagctcgca ctgtttatga tgatgctgct agacgtgttt ggacactgat gaatgtcatt 11340
   acacttgttt acaaagtcta ctatggtaat gctttagatc aagctatttc catgtgggcc 11400
   ttagttattt ctgtaacctc taactattct ggtgtcgtta cgactatcat gtttttagct 11460
   agagctatag tgtttgtgtg tgttgagtat tacccattgt tatttattac tggcaacacc 11520
   ttacagtgta tcatgcttgt ttattgtttc ttaggctatt gttgctgctg ctactttggc 11580
   cttttctgtt tactcaaccg ttacttcagg cttactcttg gtgtttatga ctacttggtc 11640
   tctacacaag aatttaggta tatgaactcc caggggcttt tgcctcctaa gagtagtatt 11700
   gatgctttca agcttaacat taagttgttg ggtattggag gtaaaccatg tatcaaggtt 11760
   gctactgtac agtctaaaat gtctgacgta aagtgcacat ctgtggtact gctctcggtt 11820
   cttcaacaac ttagagtaga gtcatcttct aaattgtggg cacaatgtgt acaactccac 11880
   aatgatattc ttcttgcaaa agacacaact gaagctttcg agaagatggt ttctcttttg 11940
   tctgttttgc tatccatgca gggtgctgta gacattaata ggttgtgcga ggaaatgctc 12000
   gataaccgtg ctactcttca ggctattgct tcagaattta gttctttacc atcatatgcc 12060
   gcttatgcca ctgcccagga ggcctatgag caggctgtag ctaatggtga ttctgaagtc 12120
   gttctcaaaa agttaaagaa atctttgaat gtggctaaat ctgagtttga ccgtgatgct 12180
   gccatgcaac gcaagttgga aaagatggca gatcaggcta tgacccaaat gtacaaacag 12240
   gcaagatctg aggacaagag ggcaaaagta actagtgcta tgcaaacaat gctcttcact 12300
   atgcttagga agcttgataa tgatgcactt aacaacatta tcaacaatgc gcgtgatggt 12360
   tgtgttccac tcaacatcat accattgact acagcagcca aactcatggt tgttgtccct 12420
   gattatggta cctacaagaa cacttgtgat ggtaacacct ttacatatgc atctgcactc 12480
   tgggaaatcc agcaagttgt tgatgcggat agcaagattg ttcaacttag tgaaattaac 12540
   atggacaatt caccaaattt ggcttggcct cttattgtta cagctctaag agccaactca 12600
   gctgttaaac tacagaataa tgaactgagt ccagtagcac tacgacagat gtcctgtgcg 12660
   gctggtacca cacaaacagc ttgtactgat gacaatgcac ttgcctacta taacaattcg 12720
   aagggaggta ggtttgtgct ggcattacta tcagaccacc aagatctcaa atgggctaga 12780
   ttccctaaga gtgatggtac aggtacaatt tacacagaac tggaaccacc ttgtaggttt 12840
   gttacagaca caccaaaagg gcctaaagtg aaatacttgt acttcatcaa aggcttaaac 12900
   aacctaaata gaggtatggt gctgggcagt ttagctgcta cagtacgtct tcaggctgga 12960
   aatgctacag aagtacctgc caattcaact gtgctttcct tctgtgcttt tgcagtagac 13020
   cctgctaaag catataagga ttacctagca agtggaggac aaccaatcac caactgtgtg 13080
   aagatgttgt gtacacacac tggtacagga caggcaatta ctgtaacacc agaagctaac 13140
   atggaccaag agtcctttgg tggtgcttca tgttgtctgt attgtagatg ccacattgac 13200
   catccaaatc ctaaaggatt ctgtgacttg aaaggtaagt acgtccaaat acctaccact 13260
   tgtgctaatg acccagtggg ttttacactt agaaacacag tctgtaccgt ctgcggaatg 13320
   tggaaaggtt atggctgtag ttgtgaccaa ctccgcgaac ccttgatgca gtctgcggat 13380
   gcatcaacgt ttttaaacgg gtttgcggtg taagtgcagc ccgtcttaca ccgtgcggca 13440
   caggcactag tactgatgtc gtctacaggg cttttgatat ttacaacgaa aaagttgctg 13500
   gttttgcaaa gttcctaaaa actaattgct gtcgcttcca ggagaaggat gaggaaggca 13560
   atttattaga ctcttacttt gtagttaaga ggcatactat gtctaactac caacatgaag 13620
   agactattta taacttggtt aaagattgtc cagcggttgc tgtccatgac tttttcaagt 13680
   ttagagtaga tggtgacatg gtaccacata tatcacgtca gcgtctaact aaatacacaa 13740
   tggctgattt agtctatgct ctacgtcatt ttgatgaggg taattgtgat acattaaaag 13800
   aaatactcgt cacatacaat tgctgtgatg atgattattt caataagaag gattggtatg 13860
   acttcgtaga gaatcctgac atcttaacgc gtatatgcta acttaggtga gcgtgtacgc 13920
   caatcattat taaagactgt acaattctgc gatgctatgc gtgatgcagg cattgtaggc 13980
   gtactgacat tagataatca ggatcttaat gggaactggt acgatttcgg tgatttcgta 14040
   caagtagcac caggctgcgg agttcctatt gtggattcat attactcatt gctgatgccc 14100
   atcctcactt tgactagggc attggctgct gagtcccata tggatgctga tctcgcaaaa 14160
   ccacttatta agtgggattt gctgaaatat gattttacgg aagagagact ttgtctcttc 14220
   gaccgttatt ttaaatattg ggaccagaca taccatccca attgtattaa ctgtttggat 14280
   gataggtgta tccttcattg tgcaaacttt aatgtgttat tttctactgt gtttccacct 14340
   acaagttttg gaccactagt aagaaaaata tttgtagatg gtgttccttt tgttgtttca 14400
   actggatacc attttcgtga gttaggagtc gtacataatc aggatgtaaa cttacatagc 14460
   tcgcgtctca gtttcaagga acttttagtg tatgctgctg atccagctat gcatgcagct 14520
   tctggcaatt tattgctaga taaacgcact acatgctttt cagtagctgc actaacaaac 14580
   aatgttgctt ttcaaactgt caaacccggt aattttaata aagactttta tgactttgct 14640
   gtgtctaaag gtttctttaa ggaaggaagt tctgttgaac taaaacactt cttctttgct 14700
   caggatggca acgctgctat cagtgattat gactattatc gttataatct gccaacaatg 14760
   tgtgatatca gacaactcct attcgtagtt gaagttgttg ataaatactt tgattgttac 14820
   gatggtggct gtattaatgc caaccaagta atcgttaaca atctggataa atcagctggt 14880
   ttcccattta ataaatgggg taaggctaga ctttattatg actcaatgag ttatgaggat 14940
   caagatgcac ttttcgcgta tactaagcgt aatgtcatcc ctactataac tcaaatgaat 15000
   cttaagtatg ccattagtgc aaagaataga gctcgcaccg tagctggtgt ctctatctgt 15060
   agtactatga caaatagaca gtttcatcag aaattattga agtcaatagc cgccactaga 15120
   ggagctactg tggtaattgg aacaagcaag ttttacggtg gctggcataa tatgttaaaa 15180
   actgtttaca gtgatgtaga aactccacac cttatgggtt gggattatcc aaaatgtgac 15240
   agagccatgc ctaacatgct taggataatg gcctctcttg ttcttgctcg caaacataac 15300
   acttgctgta acttatcaca ccgtttctac aggttagcta acgagtgtgc gcaagtatta 15360
   agtgagatgg tcatgtgtgg cggctcacta tatgttaaac caggtggaac atcatccggt 15420
   gatgctacaa ctgcttatgc taatagtgtc tttaacattt gtcaagctgt tacagccaat 15480
   gtaaatgcac ttctttcaac tgatggtaat aagatagctg acaagtatgt ccgcaatcta 15540
   caacacaggc tctatgagtg tctctataga aatagggatg ttgatcatga attcgtggat 15600
   gagttttacg cttacctgcg taaacatttc tccatgatga ttctttctga tgatgccgtt 15660
   gtgtgctata acagtaacta tgcggctcaa ggtttagtag ctagcattaa gaactttaag 15720
   gcagttcttt attatcaaaa taatgtgttc atgtctgagg caaaatgttg gactgagact 15780
   gaccttacta aaggacctca cgaattttgc tcacagcata caatgctagt taaacaagga 15840
   gatgattacg tgtacctgcc ttacccagat ccatcaagaa tattaggcgc aggctgtttt 15900
   gtcgatgata ttgtcaaaac agatggtaca cttatgattg aaaggttcgt gtcactggct 15960
   attgatgctt acccacttac aaaacatcct aatcaggagt atgctgatgt ctttcacttg 16020
   tatttacaat acattagaaa gttacatgat gagcttactg gccacatgtt ggacatgtat 16080
   tccgtaatgc taactaatga taacacctca cggtactggg aacctgagtt ttatgaggct 16140
   atgtacacac cacatacagt cttgcaggct gtaggtgctt gtgtattgtg caattcacag 16200
   acttcacttc gttgcggtgc ctgtattagg agaccattcc tatgttgcaa gtgctgctat 16260
   gaccatgtca tttcaacatc acacaaatta gtgttgtctg ttaatcccta tgtttgcaat 16320
   gccccaggtt gtgatgtcac tgatgtgaca caactgtatc taggaggtat gagctattat 16380
   tgcaagtcac ataagcctcc cattagtttt ccattatgtg ctaatggtca ggtttttggt 16440
   ttatacaaaa acacatgtgt aggcagtgac aatgtcactg acttcaatgc gatagcaaca 16500
   tgtgattgga ctaatgctgg cgattacata cttgccaaca cttgtactga gagactcaag 16560
   cttttcgcag cagaaacgct caaagccact gaggaaacat ttaagctgtc atatggtatt 16620
   gctactgtac gcgaagtact ctctgacaga gaattgcatc tttcatggga ggttggaaaa 16680
   cctagaccac cattgaacag aaactatgtc tttactggtt accgtgtaac taaaaatagt 16740
   aaagtacaga ttggagagta cacctttgaa aaaggtgact atggtgatgc tgttgtgtac 16800
   agaggtacta cgacatacaa gttgaatgtt ggtgattact ttgtgttgac atctcacact 16860
   gtaatgccac ttagtgcacc tactctagtg ccacaagagc actatgtgag aattactggc 16920
   ttgtacccaa cactcaacat ctcagatgag ttttctagca atgttgcaaa ttatcaaaag 16980
   gtcggcatgc aaaagtactc tacactccaa ggaccacctg gtactggtaa gagtcatttt 17040
   gccatcggac ttgctctcta ttacccatct gctcgcatag tgtatacggc atgctctcat 17100
   gcagctgttg atgccctatg tgaaaaggca ttaaaatatt tgcccataga taaatgtagt 17160
   agaatcatac ctgcgcgtgc gcgcgtagag tgttttgata aattcaaagt gaattcaaca 17220
   ctagaacagt atgttttctg cactgtaaat gcattgccag aaacaactgc tgacattgta 17280
   gtctttgatg aaatctctat ggctactaat tatgacttga gtgttgtcaa tgctagactt 17340
   cgtgcaaaac actacgtcta tattggcgat cctgctcaat taccagcccc ccgcacattg 17400
   ctgactaaag gcacactaga accagaatat tttaattcag tgtgcagact tatgaaaaca 17460
   ataggtccag acatgttcct tggaacttgt cgccgttgtc ctgctgaaat tgttgacact 17520
   gtgagtgctt tagtttatga caataagcta aaagcacaca aggataagtc agctcaatgc 17580
   ttcaaaatgt tctacaaagg tgttattaca catgatgttt catctgcaat caacagacct 17640
   caaataggcg ttgtaagaga atttcttaca cgcaatcctg cttggagaaa agctgttttt 17700
   atctcacctt ataattcaca gaacgctgta gcttcaaaaa tcttaggatt gcctacgcag 17760
   actgttgatt catcacaggg ttctgaatat gactatgtca tattcacaca aactactgaa 17820
   acagcacact cttgtaatgt caaccgcttc aatgtggcta tcacaagggc aaaaattggc 17880
   attttgtgca taatgtctga tagagatctt tatgacaaac tgcaatttac aagtctagaa 17940
   ataccacgtc gcaatgtggc tacattacaa gcagaaaatg taactggact ttttaaggac 18000
   tgtagtaaga tcattactgg tcttcatcct acacaggcac ctacacacct cagcgttgat 18060
   ataaagttca agactgaagg attatgtgtt gacataccag gcataccaaa ggacatgacc 18120
   taccgtagac tcatctctat gatgggtttc aaaatgaatt accaagtcaa tggttaccct 18180
   aatatgttta tcacccgcga agaagctatt cgtcacgttc gtgcgtggat tggctttgat 18240
   gtagagggct gtcatgcaac tagagatgct gtgggtacta acctacctct ccagctagga 18300
   ttttctacag gtgttaactt agtagctgta ccgactggtt atgttgacac tgaaaataac 18360
   acagaattca ccagagttaa tgcaaaacct ccaccaggtg accagtttaa acatcttata 18420
   ccactcatgt ataaaggctt gccctggaat gtagtgcgta ttaagatagt acaaatgctc 18480
   agtgatacac tgaaaggatt gtcagacaga gtcgtgttcg tcctttgggc gcatggcttt 18540
   gagcttacat caatgaagta ctttgtcaag attggacctg aaagaacgtg ttgtctgtgt 18600
   gacaaacgtg caacttgctt ttctacttca tcagatactt atgcctgctg gaatcattct 18660
   gtgggttttg actatgtcta taacccattt atgattgatg ttcagcagtg gggctttacg 18720
   ggtaaccttc agagtaacca tgaccaacat tgccaggtac atggaaatgc acatgtggct 18780
   agttgtgatg ctatcatgac tagatgttta gcagtccatg agtgctttgt taagcgcgtt 18840
   gattggtctg ttgaataccc tattatagga gatgaactga gggttaattc tgcttgcaga 18900
   aaagtacaac acatggttgt gaagtctgca ttgcttgctg ataagtttcc agttcttcat 18960
   gacattggaa atccaaaggc tatcaagtgt gtgcctcagg ctgaagtaga atggaagttc 19020
   tacgatgctc agccatgtag tgacaaagct tacaaaatag aggagctctt ctattcttat 19080
   gctacacatc acgataaatt cactgatggt gtttgtttgt tttggaattg taacgttgat 19140
   cgttacccag ccaatgcaat tgtgtgtagg tttgacacaa gagtcttgtc aaacttgaac 19200
   ttaccaggct gtgatggtgg tagtttgtat gtgaataagc atgcattcca cactccagct 19260
   ttcgataaaa gtgcatttac taatttaaag caattgcctt tcttttacta ttctgatagt 19320
   ccttgtgagt ctcatggcaa acaagtagtg tcggatattg attatgttcc actcaaatct 19380
   gctacgtgta ttacacgatg caatttaggt ggtgctgttt gcagacacca tgcaaatgag 19440
   taccgacagt acttggatgc atataatatg atgatttctg ctggatttag cctatggatt 19500
   tacaaacaat ttgatactta taacctgtgg aatacattta ccaggttaca gagtttagaa 19560
   aatgtggctt ataatgttgt taataaagga cactttgatg gacacgccgg cgaagcacct 19620
   gtttccatca ttaataatgc tgtttacaca aaggtagatg gtattgatgt ggagatcttt 19680
   gaaaataaga caacacttcc tgttaatgtt gcatttgagc tttgggctaa gcgtaacatt 19740
   aaaccagtgc cagagattaa gatactcaat aatttgggtg ttgatatcgc tgctaatact 19800
   gtaatctggg actacaaaag agaagcccca gcacatgtat ctacaatagg tgtctgcaca 19860
   atgactgaca ttgccaagaa acctactgag agtgcttgtt cttcacttac tgtcttgttt 19920
   gatggtagag tggaaggaca ggtagacctt tttagaaacg cccgtaatgg tgttttaata 19980
   acagaaggtt cagtcaaagg tctaacacct tcaaagggac cagcacaagc tagcgtcaat 20040
   ggagtcacat taattggaga atcagtaaaa acacagttta actactttaa gaaagtagac 20100
   ggcattattc aacagttgcc tgaaacctac tttactcaga gcagagactt agaggatttt 20160
   aagcccagat cacaaatgga aactgacttt ctcgagctcg ctatggatga attcatacag 20220
   cgatataagc tcgagggcta tgccttcgaa cacatcgttt atggagattt cagtcatgga 20280
   caacttggcg gtcttcattt aatgataggc ttagccaagc gctcacaaga ttcaccactt 20340
   aaattagagg attttatccc tatggacagc acagtgaaaa attacttcat aacagatgcg 20400
   caaacaggtt catcaaaatg tgtgtgttct gtgattgatc ttttacttga tgactttgtc 20460
   gagataataa agtcacaaga tttgtcagtg atttcaaaag tggtcaaggt tacaattgac 20520
   tatgctgaaa tttcattcat gctttggtgt aaggatggac atgttgaaac cttctaccca 20580
   aaactacaag caagtcaagc gtggcaacca ggtgttgcga tgcctaactt gtacaagatg 20640
   caaagaatgc ttcttgaaaa gtgtgacctt cagaattatg gtgaaaatgc tgttatacca 20700
   aaaggaataa tgatgaatgt cgcaaagtat actcaactgt gtcaatactt aaatacactt 20760
   actttagctg taccctacaa catgagagtt attcactttg gtgctggctc tgataaagga 20820
   gttgcaccag gtacagctgt gctcagacaa tggttgccaa ctggcacact acttgtcgat 20880
   tcagatctta atgacttcgt ctccgacgca gattctactt taattggaga ctgtgcaaca 20940
   gtacatacgg ctaataaatg ggaccttatt attagcgata tgtatgaccc taggaccaaa 21000
   catgtgacaa aagagaatga ctctaaagaa gggtttttca cttatctgtg tggatttata 21060
   aagcaaaaac tagccctggg tggttctata gctgtaaaga taacagagca ttcttggaat 21120
   gctgaccttt acaagcttat gggccatttc tcatggtgga cagcttttgt tacaaatgta 21180
   aatgcatcat catcggaagc atttttaatt ggggctaact atcttggcaa gccgaaggaa 21240
   caaattgatg gctataccat gcatgctaac tacattttct ggaggaacac aaatcctatc 21300
   cagttgtctt cctattcact ctttgacatg agcaaatttc ctcttaaatt aagaggaact 21360
   gctgtaatgt ctcttaagga gaatcaaatc aatgatatga tttattctct tctggaaaaa 21420
   ggtaggctta tcattagaga aaacaacaga gttgtggttt caagtgatat tcttgttaac 21480
   aactaaacga acatgtttat tttcttatta tttcttactc tcactagtgg tagtgacctt 21540
   gaccggtgca ccacttttga tgatgttcaa gctcctaatt acactcaaca tacttcatct 21600
   atgagggggg tttactatcc tgatgaaatt tttagatcag acactcttta tttaactcag 21660
   gatttatttc ttccatttta ttctaatgtt acagggtttc atactattaa tcatacgttt 21720
   ggcaaccctg tcataccttt taaggatggt atttattttg ctgccacaga gaaatcaaat 21780
   gttgtccgtg gttgggtttt tggttctacc atgaacaaca agtcacagtc ggtgattatt 21840
   attaacaatt ctactaatgt tgttatacga gcatgtaact ttgaattgtg tgacaaccct 21900
   ttctttgctg tttctaaacc catgggtaca cagacacata ctatgatatt cgataatgca 21960
   tttaattgca ctttcgagta catatctgat gccttttcgc ttgatgtttc agaaaagtca 22020
   ggtaatttta aacacttacg agagtttgtg tttaaaaata aagatgggtt tctctatgtt 22080
   tataagggct atcaacctat agatgtagtt cgtgatctac cttctggttt taacactttg 22140
   aaacctattt ttaagttgcc tcttggtatt aacattacaa attttagagc cattcttaca 22200
   gccttttcac ctgctcaaga catttggggc acgtcagctg cagcctattt tgttggctat 22260
   ttaaagccaa ctacatttat gctcaagtat gatgaaaatg gtacaatcac agatgctgtt 22320
   gattgttctc aaaatccact tgctgaactc aaatgctctg ttaagagctt tgagattgac 22380
   aaaggaattt accagacctc taatttcagg gttgttccct caggagatgt tgtgagattc 22440
   cctaatatta caaacttgtg tccttttgga gaggttttta atgctactaa attcccttct 22500
   gtctatgcat gggagagaaa aaaaatttct aattgtgttg ctgattactc tgtgctctac 22560
   aactcaacat ttttttcaac ctttaagtgc tatggcgttt ctgccactaa gttgaatgat 22620
   ctttgcttct ccaatgtcta tgcagattct tttgtagtca agggagatga tgtaagacaa 22680
   atagcgccag gacaaactgg tgttattgct gattataatt ataaattgcc agatgatttc 22740
   atgggttgtg tccttgcttg gaatactagg aacattgatg ctacttcaac tggtaattat 22800
   aattataaat ataggtatct tagacatggc aagcttaggc cctttgagag agacatatct 22860
   aatgtgcctt tctcccctga tggcaaacct tgcaccccac ctgctcttaa ttgttattgg 22920
   ccattaaatg attatggttt ttacaccact actggcattg gctaccaacc ttacagagtt 22980
   gtagtacttt cttttgaact tttaaatgca ccggccacgg tttgtggacc aaaattatcc 23040
   actgacctta ttaagaacca gtgtgtcaat tttaatttta atggactcac tggtactggt 23100
   gtgttaactc cttcttcaaa gagatttcaa ccatttcaac aatttggccg tgatgtttct 23160
   gatttcactg attccgttcg agatcctaaa acatctgaaa tattagacat ttcaccttgc 23220
   tcttttgggg gtgtaagtgt aattacacct ggaacaaatg cttcatctga agttgctgtt 23280
   ctatatcaag atgttaactg cactgatgtt tctacagcaa ttcatgcaga tcaactcaca 23340
   ccagcttggc gcatatattc tactggaaac aatgtattcc agactcaagc aggctgtctt 23400
   ataggagctg agcatgtcga cacttcttat gagtgcgaca ttcctattgg agctggcatt 23460
   tgtgctagtt accatacagt ttctttatta cgtagtacta gccaaaaatc tattgtggct 23520
   tatactatgt ctttaggtgc tgatagttca attgcttact ctaataacac cattgctata 23580
   cctactaact tttcaattag cattactaca gaagtaatgc ctgtttctat ggctaaaacc 23640
   tccgtagatt gtaatatgta catctgcgga gattctactg aatgtgctaa tttgcttctc 23700
   caatatggta gcttttgcac acaactaaat cgtgcactct caggtattgc tgctgaacag 23760
   gatcgcaaca cacgtgaagt gttcgctcaa gtcaaacaaa tgtacaaaac cccaactttg 23820
   aaatattttg gtggttttaa tttttcacaa atattacctg accctctaaa gccaactaag 23880
   aggtctttta ttgaggactt gctctttaat aaggtgacac tcgctgatgc tggcttcatg 23940
   aagcaatatg gcgaatgcct aggtgatatt aatgctagag atctcatttg tgcgcagaag 24000
   ttcaatggac ttacagtgtt gccacctctg ctcactgatg atatgattgc tgcctacact 24060
   gctgctctag ttagtggtac tgccactgct ggatggacat ttggtgctgg cgctgctctt 24120
   caaatacctt ttgctatgca aatggcatat aggttcaatg gcattggagt tacccaaaat 24180
   gttctctatg agaaccaaaa acaaatcgcc aaccaattta acaaggcgat tagtcaaatt 24240
   caagaatcac ttacaacaac atcaactgca ttgggcaagc tgcaagacgt tgttaaccag 24300
   aatgctcaag cattaaacac acttgttaaa caacttagct ctaattttgg tgcaatttca 24360
   agtgtgctaa atgatatcct ttcgcgactt gataaagtcg aggcggaggt acaaattgac 24420
   aggttaatta caggcagact tcaaagcctt caaacctatg taacacaaca actaatcagg 24480
   gctgctgaaa tcagggcttc tgctaatctt gctgctacta aaatgtctga gtgtgttctt 24540
   ggacaatcaa aaagagttga cttttgtgga aagggctacc accttatgtc cttcccacaa 24600
   gcagccccgc atggtgttgt cttcctacat gtcacgtatg tgccatccca ggagaggaac 24660
   ttcaccacag cgccagcaat ttgtcatgaa ggcaaagcat acttccctcg tgaaggtgtt 24720
   tttgtgttta atggcacttc ttggtttatt acacagagga acttcttttc tccacaaata 24780
   attactacag acaatacatt tgtctcagga aattgtgatg tcgttattgg catcattaac 24840
   aacacagttt atgatcctct gcaacctgag ctcgactcat tcaaagaaga gctggacaag 24900
   tacttcaaaa atcatacatc accagatgtt gatcttggcg acatttcagg cattaacgct 24960
   tctgtcgtca acattcaaaa agaaattgac cgcctcaatg aggtcgctaa aaatttaaat 25020
   gaatcactca ttgaccttca agaattggga aaatatgagc aatatattaa atggccttgg 25080
   tatgtttggc tcggcttcat tgctggacta attgccatcg tcatggttac aatcttgctt 25140
   tgttgcatga ctagttgttg cagttgcctc aagggtgcat gctcttgtgg ttcttgctgc 25200
   aagtttgatg aggatgactc tgagccagtt ctcaagggtg tcaaattaca ttacacataa 25260
   acgaacttat ggatttgttt atgagatttt ttactcttgg atcaattact gcacagccag 25320
   taaaaattga caatgcttct cctgcaagta ctgttcatgc tacagcaacg ataccgctac 25380
   aagcctcact ccctttcgga tggcttgtta ttggcgttgc atttcttgct gtttttcaga 25440
   gcgctaccaa aataattgcg ctcaataaaa gatggcagct agccctttat aagggcttcc 25500
   agttcatttg caatttactg ctgctatttg ttaccatcta ttcacatctt ttgcttgtcg 25560
   ctgcaggtat ggaggcgcaa tttttgtacc tctatgcctt gatatatttt ctacaatgca 25620
   tcaacgcatg tagaattatt atgagatgtt ggctttgttg gaagtgcaaa tccaagaacc 25680
   cattacttta tgatgccaac tactttgttt gctggcacac acataactat gactactgta 25740
   taccatataa cagtgtcaca gatacaattg tcgttactga aggtgacggc atttcaacac 25800
   caaaactcaa agaagactac caaattggtg gttattctga ggataggcac tcaggtgtta 25860
   aagactatgt cgttgtacat ggctatttca ccgaagttta ctaccagctt gagtctacac 25920
   aaattactac agacactggt attgaaaatg ctacattctt catctttaac aagcttgtta 25980
   aagacccacc gaatgtgcaa atacacacaa tcgacggctc ttcaggagtt gctaatccag 26040
   caatggatcc aatttatgat gagccgacga cgactactag cgtgcctttg taagcacaag 26100
   aaagtgagta cgaacttatg tactcattcg tttcggaaga aacaggtacg ttaatagtta 26160
   atagcgtact tctttttctt gctttcgtgg tattcttgct agtcacacta gccatcctta 26220
   ctgcgcttcg attgtgtgcg tactgctgca atattgttaa cgtgagttta gtaaaaccaa 26280
   cggtttacgt ctactcgcgt gttaaaaatc tgaactcttc tgaaggagtt cctgatcttc 26340
   tggtctaaac gaactaacta ttattattat tctgtttgga actttaacat tgcttatcat 26400
   ggcagacaac ggtactatta ccgttgagga gcttaaacaa ctcctggaac aatggaacct 26460
   agtaataggt ttcctattcc tagcctggat tatgttacta caatttgcct attctaatcg 26520
   gaacaggttt ttgtacataa taaagcttgt tttcctctgg ctcttgtggc cagtaacact 26580
   tgcttgtttt gtgcttgctg ctgtctacag aattaattgg gtgactggcg ggattgcgat 26640
   tgcaatggct tgtattgtag gcttgatgtg gcttagctac ttcgttgctt ccttcaggct 26700
   gtttgctcgt acccgctcaa tgtggtcatt caacccagaa acaaacattc ttctcaatgt 26760
   gcctctccgg gggacaattg tgaccagacc gctcatggaa agtgaacttg tcattggtgc 26820
   tgtgatcatt cgtggtcact tgcgaatggc cggacacccc ctagggcgct gtgacattaa 26880
   ggacctgcca aaagagatca ctgtggctac atcacgaacg ctttcttatt acaaattagg 26940
   agcgtcgcag cgtgtaggca ctgattcagg ttttgctgca tacaaccgct accgtattgg 27000
   aaactataaa ttaaatacag accacgccgg tagcaacgac aatattgctt tgctagtaca 27060
   gtaagtgaca acagatgttt catcttgttg acttccaggt tacaatagca gagatattga 27120
   ttatcattat gaggactttc aggattgcta tttggaatct tgacgttata ataagttcaa 27180
   tagtgagaca attatttaag cctctaacta agaagaatta ttcggagtta gatgatgaag 27240
   aacctatgga gttagattat ccataaaacg aacatgaaaa ttattctctt cctgacattg 27300
   attgtattta catcttgcga gctatatcac tatcaggagt gtgttagagg tacgactgta 27360
   ctactaaaag aaccttgccc atcaggaaca tacgagggca attcaccatt tcaccctctt 27420
   gctgacaata aatttgcact aacttgcact agcacacact ttgcttttgc ttgtgctgac 27480
   ggtactcgac atacctatca gctgcgtgca agatcagttt caccaaaact tttcatcaga 27540
   caagaggagg ttcaacaaga gctctactcg ccactttttc tcattgttgc tgctctagta 27600
   tttttaatac tttgcttcac cattaagaga aagacagaat gaatgagctc actttaattg 27660
   acttctattt gtgcttttta gcctttctgc tattccttgt tttaataatg cttattatat 27720
   tttggttttc actcgaaatc caggatctag aagaaccttg taccaaagtc taaacgaaca 27780
   tgaaacttct cattgttttg acttgtattt ctctatgcag ttgcatatgc actgtagtac 27840
   agcgctgtgc atctaataaa cctcatgtgc ttgaagatcc ttgtaaggta caacactagg 27900
   ggtaatactt atagcactgc ttggctttgt gctctaggaa aggttttacc ttttcataga 27960
   tggcacacta tggttcaaac atgcacacct aatgttacta tcaactgtca agatccagct 28020
   ggtggtgcgc ttatagctag gtgttggtac cttcatgaag gtcaccaaac tgctgcattt 28080
   agagacgtac ttgttgtttt aaataaacga acaaattaaa atgtctgata atggacccca 28140
   atcaaaccaa cgtagtgccc cccgcattac atttggtgga cccacagatt caactgacaa 28200
   taaccagaat ggaggacgca atggggcaag gccaaaacag cgccgacccc aaggtttacc 28260
   caataatact gcgtcttggt tcacagctct cactcagcat ggcaaggagg aacttagatt 28320
   ccctcgaggc cagggcgttc caatcaacac caatagtggt ccagatgacc aaattggcta 28380
   ctaccgaaga gctacccgac gagttcgtgg tggtgacggc aaaatgaaag agctcagccc 28440
   cagatggtac ttctattacc taggaactgg cccagaagct tcacttccct acggcgctaa 28500
   caaagaaggc atcgtatggg ttgcaactga gggagccttg aatacaccca aagaccacat 28560
   tggcacccgc aatcctaata acaatgctgc caccgtgcta caacttcctc aaggaacaac 28620
   attgccaaaa ggcttctacg cagagggaag cagaggcggc agtcaagcct cttctcgctc 28680
   ctcatcacgt agtcgcggta attcaagaaa ttcaactcct ggcagcagta ggggaaattc 28740
   tcctgctcga atggctagcg gaggtggtga aactgccctc gcgctattgc tgctagacag 28800
   attgaaccag cttgagagca aagtttctgg taaaggccaa caacaacaag gccaaactgt 28860
   cactaagaaa tctgctgctg aggcatctaa aaagcctcgc caaaaacgta ctgccacaaa 28920
   acagtacaac gtcactcaag catttgggag acgtggtcca gaacaaaccc aaggaaattt 28980
   cggggaccaa gacctaatca gacaaggaac tgattacaaa cattggccgc aaattgcaca 29040
   atttgctcca agtgcctctg cattctttgg aatgtcacgc attggcatgg aagtcacacc 29100
   ttcgggaaca tggctgactt atcatggagc cattaaattg gatgacaaag atccacaatt 29160
   caaagacaac gtcatactgc tgaacaagca cattgacgca tacaaaacat tcccaccaac 29220
   agagcctaaa aaggacaaaa agaaaaagac tgatgaagct cagcctttgc cgcagagaca 29280
   aaagaagcag cccactgtga ctcttcttcc tgcggctgac atggatgatt tctccagaca 29340
   acttcaaaat tccatgagtg gagcttctgc tgattcaact caggcataaa cactcatgat 29400
   gaccacacaa ggcagatggg ctatgtaaac gttttcgcaa ttccgtttac gatacatagt 29460
   ctactcttgt gcagaatgaa ttctcgtaac taaacagcac aagtaggttt agttaacttt 29520
   aatctcacat agcaatcttt aatcaatgtg taacattagg gaggacttga aagagccacc 29580
   acattttcat cgaggccacg cggagtacga tcgagggtac agtgaataat gctagggaga 29640
   gctgcctata tggaagagcc ctaatgtgta aaattaattt tagtagtgct atccccatgt 29700
   gattttaata gcttcttagg agaatgacaa aaaaaaaaaa aaaaaaaaaa aaagggtcgg 29760
   catggcatct ccacctcctc gcggtccgac ctgggcatcc gaaggaggac gtcgtccact 29820
   cggatggcta agggagagct cggatcgatc cgcctcgact gtgccttcta gttgccagcc 29880
   atctgttgtt tgcccctccc ccgtgccttc cttgaccctg gaaggtgcca ctcccactgt 29940
   cctttcctaa taaaatgagg aaattgcatc gcattgtctg agtaggtgtc attctattct 30000
   ggggggtggg gtggggcagg acagcaaggg ggaggattgg gaagacaata gcaggcatgc 30060
   tggggagagc tcgagctctg tacatgtccg cggaagcttg agtattctat agtgtcacct 30120
   aaatagcttg gcgtaatcat ggtcatagct gtttcctgtg tgaaattgtt atccgctcac 30180
   aattccacac aacatacgag ccggaagcat aaagtgtaaa gcctggggtg cctaatgagt 30240
   gagctaactc acattaattg cgttgcgctc actgcccgct ttccagtcgg gaaacctgtc 30300
   gtgccagctg cattaatgaa tcggccaacg cgaacccctt gcggccgccc gggccgtcga 30360
   ccaattctca tgtttgacag cttatcatcg aatttctgcc attcatccgc ttattatcac 30420
   ttattcaggc gtagcaacca ggcgtttaag ggcaccaata actgccttaa aaaaattacg 30480
   ccccgccctg ccactcatcg cagtactgtt gtaattcatt aagcattctg ccgacatgga 30540
   agccatcaca aacggcatga tgaacctgaa tcgccagcgg catcagcacc ttgtcgcctt 30600
   gcgtataata tttgcccatg gtgaaaacgg gggcgaagaa gttgtccata ttggccacgt 30660
   ttaaatcaaa actggtgaaa ctcacccagg gattggctga gacgaaaaac atattctcaa 30720
   taaacccttt agggaaatag gccaggtttt caccgtaaca cgccacatct tgcgaatata 30780
   tgtgtagaaa ctgccggaaa tcgtcgtggt attcactcca gagcgatgaa aacgtttcag 30840
   tttgctcatg gaaaacggtg taacaagggt gaacactatc ccatatcacc agctcaccgt 30900
   ctttcattgc catacggaat tccggatgag cattcatcag gcgggcaaga atgtgaataa 30960
   aggccggata aaacttgtgc ttatttttct ttacggtctt taaaaaggcc gtaatatcca 31020
   gctgaacggt ctggttatag gtacattgag caactgactg aaatgcctca aaatgttctt 31080
   tacgatgcca ttgggatata tcaacggtgg tatatccagt gatttttttc tccattttag 31140
   cttccttagc tcctgaaaat ctcgataact caaaaaatac gcccggtagt gatcttattt 31200
   cattatggtg aaagttggaa cctcttacgt gccgatcaac gtctcatttt cgccaaaagt 31260
   tggcccaggg cttcccggta tcaacaggga caccaggatt tatttattct gcgaagtgat 31320
   cttccgtcac aggtatttat tcgcgataag ctcatggagc ggcgtaaccg tcgcacagga 31380
   aggacagaga aagcgcggat ctgggaagtg acggacagaa cggtcaggac ctggattggg 31440
   gaggcggttg ccgccgctgc tgctgacggt gtgacgttct ctgttccggt cacaccacat 31500
   acgttccgcc attcctatgc gatgcacatg ctgtatgccg gtataccgct gaaagttctg 31560
   caaagcctga tgggacataa gtccatcagt tcaacggaag tctacacgaa ggtttttgcg 31620
   ctggatgtgg ctgcccggca ccgggtgcag tttgcgatgc cggagtctga tgcggttgcg 31680
   atgctgaaac aattatcctg agaataaatg ccttggcctt tatatggaaa tgtggaactg 31740
   agtggatatg ctgtttttgt ctgttaaaca gagaagctgg ctgttatcca ctgagaagcg 31800
   aacgaaacag tcgggaaaat ctcccattat cgtagagatc cgcattatta atctcaggag 31860
   cctgtgtagc gtttatagga agtagtgttc tgtcatgatg cctgcaagcg gtaacgaaaa 31920
   cgatttgaat atgccttcag gaacaataga aatcttcgtg cggtgttacg ttgaagtgga 31980
   gcggattatg tcagcaatgg acagaacaac ctaatgaaca cagaaccatg atgtggtctg 32040
   tccttttaca gccagtagtg ctcgccgcag tcgagcgaca gggcgaagcc ctcgagtgag 32100
   cgaggaagca ccagggaaca gcacttatat attctgctta cacacgatgc ctgaaaaaac 32160
   ttcccttggg gttatccact tatccacggg gatattttta taattatttt ttttatagtt 32220
   tttagatctt cttttttaga gcgccttgta ggcctttatc catgctggtt ctagagaagg 32280
   tgttgtgaca aattgccctt tcagtgtgac aaatcaccct caaatgacag tcctgtctgt 32340
   gacaaattgc ccttaaccct gtgacaaatt gccctcagaa gaagctgttt tttcacaaag 32400
   ttatccctgc ttattgactc ttttttattt agtgtgacaa tctaaaaact tgtcacactt 32460
   cacatggatc tgtcatggcg gaaacagcgg ttatcaatca caagaaacgt aaaaatagcc 32520
   cgcgaatcgt ccagtcaaac gacctcactg aggcggcata tagtctctcc cgggatcaaa 32580
   aacgtatgct gtatctgttc gttgaccaga tcagaaaatc tgatggcacc ctacaggaac 32640
   atgacggtat ctgcgagatc catgttgcta aatatgctga aatattcgga ttgacctctg 32700
   cggaagccag taaggatata cggcaggcat tgaagagttt cgcggggaag gaagtggttt 32760
   tttatcgccc tgaagaggat gccggcgatg aaaaaggcta tgaatctttt ccttggttta 32820
   tcaaacgtgc gcacagtcca tccagagggc tttacagtgt acatatcaac ccatatctca 32880
   ttcccttctt tatcgggtta cagaaccggt ttacgcagtt tcggcttagt gaaacaaaag 32940
   aaatcaccaa tccgtatgcc atgcgtttat acgaatccct gtgtcagtat cgtaagccgg 33000
   atggctcagg catcgtctct ctgaaaatcg actggatcat agagcgttac cagctgcctc 33060
   aaagttacca gcgtatgcct gacttccgcc gccgcttcct gcaggtctgt gttaatgaga 33120
   tcaacagcag aactccaatg cgcctctcat acattgagaa aaagaaaggc cgccagacga 33180
   ctcatatcgt attttccttc cgcgatatca cttccatgac gacaggatag tctgagggtt 33240
   atctgtcaca gatttgaggg tggttcgtca catttgttct gacctactga gggtaatttg 33300
   tcacagtttt gctgtttcct tcagcctgca tggattttct catacttttt gaactgtaat 33360
   ttttaaggaa gccaaatttg agggcagttt gtcacagttg atttccttct ctttcccttc 33420
   gtcatgtgac ctgatatcgg gggttagttc gtcatcattg atgagggttg attatcacag 33480
   tttattactc tgaattggct atccgcgtgt gtacctctac ctggagtttt tcccacggtg 33540
   gatatttctt cttgcgctga gcgtaagagc tatctgacag aacagttctt ctttgcttcc 33600
   tcgccagttc gctcgctatg ctcggttaca cggctgcggc gagcgctagt gataataagt 33660
   gactgaggta tgtgctcttc ttatctcctt ttgtagtgtt gctcttattt taaacaactt 33720
   tgcggttttt tgatgacttt gcgattttgt tgttgctttg cagtaaattg caagatttaa 33780
   taaaaaaacg caaagcaatg attaaaggat gttcagaatg aaactcatgg aaacacttaa 33840
   ccagtgcata aacgctggtc atgaaatgac gaaggctatc gccattgcac agtttaatga 33900
   tgacagcccg gaagcgagga aaataacccg gcgctggaga ataggtgaag cagcggattt 33960
   agttggggtt tcttctcagg ctatcagaga tgccgagaaa gcagggcgac taccgcaccc 34020
   ggatatggaa attcgaggac gggttgagca acgtgttggt tatacaattg aacaaattaa 34080
   tcatatgcgt gatgtgtttg gtacgcgatt gcgacgtgct gaagacgtat ttccaccggt 34140
   gatcggggtt gctgcccata aaggtggcgt ttacaaaacc tcagtttctg ttcatcttgc 34200
   tcaggatctg gctctgaagg ggctacgtgt tttgctcgtg gaaggtaacg acccccaggg 34260
   aacagcctca atgtatcacg gatgggtacc agatcttcat attcatgcag aagacactct 34320
   cctgcctttc tatcttgggg aaaaggacga tgtcacttat gcaataaagc ccacttgctg 34380
   gccggggctt gacattattc cttcctgtct ggctctgcac cgtattgaaa ctgagttaat 34440
   gggcaaattt gatgaaggta aactgcccac cgatccacac ctgatgctcc gactggccat 34500
   tgaaactgtt gctcatgact atgatgtcat agttattgac agcgcgccta acctgggtat 34560
   cggcacgatt aatgtcgtat gtgctgctga tgtgctgatt gttcccacgc ctgctgagtt 34620
   gtttgactac acctccgcac tgcagttttt cgatatgctt cgtgatctgc tcaagaacgt 34680
   tgatcttaaa gggttcgagc ctgatgtacg tattttgctt accaaataca gcaatagtaa 34740
   tggctctcag tccccgtgga tggaggagca aattcgggat gcctggggaa gcatggttct 34800
   aaaaaatgtt gtacgtgaaa cggatgaagt tggtaaaggt cagatccgga tgagaactgt 34860
   ttttgaacag gccattgatc aacgctcttc aactggtgcc tggagaaatg ctctttctat 34920
   ttgggaacct gtctgcaatg aaattttcga tcgtctgatt aaaccacgct gggagattag 34980
   ataatgaagc gtgcgcctgt tattccaaaa catacgctca atactcaacc ggttgaagat 35040
   acttcgttat cgacaccagc tgccccgatg gtggattcgt taattgcgcg cgtaggagta 35100
   atggctcgcg gtaatgccat tactttgcct gtatgtggtc gggatgtgaa gtttactctt 35160
   gaagtgctcc ggggtgatag tgttgagaag acctctcggg tatggtcagg taatgaacgt 35220
   gaccaggagc tgcttactga ggacgcactg gatgatctca tcccttcttt tctactgact 35280
   ggtcaacaga caccggcgtt cggtcgaaga gtatctggtg tcatagaaat tgccgatggg 35340
   agtcgccgtc gtaaagctgc tgcacttacc gaaagtgatt atcgtgttct ggttggcgag 35400
   ctggatgatg agcagatggc tgcattatcc agattgggta acgattatcg cccaacaagt 35460
   gcttatgaac gtggtcagcg ttatgcaagc cgattgcaga atgaatttgc tggaaatatt 35520
   tctgcgctgg ctgatgcgga aaatatttca cgtaagatta ttacccgctg tatcaacacc 35580
   gccaaattgc ctaaatcagt tgttgctctt ttttctcacc ccggtgaact atctgcccgg 35640
   tcaggtgatg cacttcaaaa agcctttaca gataaagagg aattacttaa gcagcaggca 35700
   tctaaccttc atgagcagaa aaaagctggg gtgatatttg aagctgaaga agttatcact 35760
   cttttaactt ctgtgcttaa aacgtcatct gcatcaagaa ctagtttaag ctcacgacat 35820
   cagtttgctc ctggagcgac agtattgtat aagggcgata aaatggtgct taacctggac 35880
   aggtctcgtg ttccaactga gtgtatagag aaaattgagg ccattcttaa ggaacttgaa 35940
   aagccagcac cctgatgcga ccacgtttta gtctacgttt atctgtcttt acttaatgtc 36000
   ctttgttaca ggccagaaag cataactggc ctgaatattc tctctgggcc cactgttcca 36060
   cttgtatcgt cggtctgata atcagactgg gaccacggtc ccactcgtat cgtcggtctg 36120
   attattagtc tgggaccacg gtcccactcg tatcgtcggt ctgattatta gtctgggacc 36180
   acggtcccac tcgtatcgtc ggtctgataa tcagactggg accacggtcc cactcgtatc 36240
   gtcggtctga ttattagtct gggaccatgg tcccactcgt atcgtcggtc tgattattag 36300
   tctgggacca cggtcccact cgtatcgtcg gtctgattat tagtctggaa ccacggtccc 36360
   actcgtatcg tcggtctgat tattagtctg ggaccacggt cccactcgta tcgtcggtct 36420
   gattattagt ctgggaccac gatcccactc gtgttgtcgg tctgattatc ggtctgggac 36480
   cacggtccca cttgtattgt cgatcagact atcagcgtga gactacgatt ccatcaatgc 36540
   ctgtcaaggg caagtattga catgtcgtcg taacctgtag aacggagtaa cctcggtgtg 36600
   cggttgtatg cctgctgtgg attgctgctg tgtcctgctt atccacaaca ttttgcgcac 36660
   ggttatgtgg acaaaatacc tggttaccca ggccgtgccg gcacgttaac cgggctgcat 36720
   ccgatgcaag tgtgtcgctg tcgacgagct cgcgagctcg gacatgaggt tgccccgtat 36780
   tcagtgtcgc tgatttgtat tgtctgaagt tgtttttacg ttaagttgat gcagatcaat 36840
   taatacgata cctgcgtcat aattgattat ttgacgtggt ttgatggcct ccacgcacgt 36900
   tgtgatatgt agatgataat cattatcact ttacgggtcc tttccggtga tccgacaggt 36960
   tacggggcgg cgacctcgcg ggttttcgct atttatgaaa attttccggt ttaaggcgtt 37020
   tccgttcttc ttcgtcataa cttaatgttt ttatttaaaa taccctctga aaagaaagga 37080
   aacgacaggt gctgaaagcg agctttttgg cctctgtcgt ttcctttctc tgtttttgtc 37140
   cgtggaatga acaatggaag tccgagctca tcgctaataa cttcgtatag catacattat 37200
   acgaagttat attcgatgcg gccgcaaggg gttcgcgtca gcgggtgttg gcgggtgtcg 37260
   gggctggctt aactatgcgg catcagagca gattgtactg agagtgcacc atatgcggtg 37320
   tgaaataccg cacagatgcg taaggagaaa ataccgcatc aggcgcctaa gttgacattg 37380
   attattgact agttattaat agtaatcaat tacggggtca ttagttcata gcccatatat 37440
   ggagttccgc gttacataac ttacggtaaa tggcccgcct ggctgaccgc ccaacgaccc 37500
   ccgcccattg acgtcaataa tgacgtatgt tcccatagta acgccaatag ggactttcca 37560
   ttgacgtcaa tgggtggagt atttacggta aactgcccac ttggcagtac atcaagtgta 37620
   tcatatgcca agtacgcccc ctattgacgt caatgacggt aaatggcccg cctggcatta 37680
   tgcccagtac atgaccttat gggactttcc tacttggcag tacatctacg tattagtcat 37740
   cgctattacc atggtgatgc ggttttggca gtacatcaat gggcgtggat agcggtttga 37800
   ctcacgggga tttccaagtc tccaccccat tgacgtcaat gggagtttgt tttggcacca 37860
   aaatcaacgg gactttccaa aatgtcgtaa caactccgcc ccattgacgc aaatgggcgg 37920
   taggcgtgta cggtgggagg tctatataag cagagctcgt ttagtgaacc t 37971

<210> 12
<211> 825
<212> DNA
<213> SARS-CoV

<400> 12
atggatttgt ttatgagatt ttttactctt ggatcaatta ctgcacagcc agtaaaaatt 60

gacaatgctt ctcctgcaag tactgttcat gctacagcaa cgataccgct acaagcctca 120

ctccctttcg gatggcttgt tattggcgtt gcatttcttg ctgtttttca gagcgctacc 180

aaaataattg cgctcaataa aagatggcag ctagcccttt ataagggctt ccagttcatt 240

tgcaatttac tgctgctatt tgttaccatc tattcacatc ttttgcttgt cgctgcaggt 300

atggaggcgc aatttttgta cctctatgcc ttgatatatt ttctacaatg catcaacgca 360

tgtagaatta ttatgagatg ttggctttgt tggaagtgca aatccaagaa cccattactt 420

tatgatgcca actactttgt ttgctggcac acacataact atgactactg tataccatat 480

aacagtgtca cagatacaat tgtcgttact gaaggtgacg gcatttcaac accaaaactc 540

aaagaagact accaaattgg tggttattct gaggataggc actcaggtgt taaagactat 600

gtcgttgtac atggctattt caccgaagtt tactaccagc ttgagtctac acaaattact 660

acagacactg gtattgaaaa tgctacattc ttcatcttta acaagcttgt taaagaccca 720

ccgaatgtgc aaatacacac aatcgacggc tcttcaggag ttgctaatcc agcaatggat 780

ccaatttatg atgagccgac gacgactact agcgtgcctt tgtaa 825

<210> 13
<211> 465
<212> DNA
<213> SARS-CoV

<400> 13
atgatgccaa ctactttgtt tgctggcaca cacataacta tgactactgt ataccatata 60

acagtgtcac agatacaatt gtcgttactg aaggtgacgg catttcaaca ccaaaactca 120

aagaagacta ccaaattggt ggttattctg aggataggca ctcaggtgtt aaagactatg 180

tcgttgtaca tggctatttc accgaagttt actaccagct tgagtctaca caaattacta 240

cagacactgg tattgaaaat gctacattct tcatctttaa caagcttgtt aaagacccac 300

cgaatgtgca aatacacaca atcgacggct cttcaggagt tgctaatcca gcaatggatc 360

caatttatga tgagccgacg acgactacta gcgtgccttt gtaagcacaa gaaagtgagt 420

acgaacttat gtactcattc gtttcggaag aaacaggtac gttaa 465

<210> 14
<211> 192
<212> DNA
<213> SARS-CoV

<400> 14
atgtttcatc ttgttgactt ccaggttaca atagcagaga tattgattat cattatgagg 60

actttcagga ttgctatttg gaatcttgac gttataataa gttcaatagt gagacaatta 120

tttaagcctc taactaagaa gaattattcg gagttagatg atgaagaacc tatggagtta 180

gattatccat aa 192

<210> 15
<211> 369
<212> DNA
<213> SARS-CoV

<400> 15
atgaaaatta ttctcttcct gacattgatt gtatttacat cttgcgagct atatcactat 60

caggagtgtg ttagaggtac gactgtacta ctaaaagaac cttgcccatc aggaacatac 120

gagggcaatt caccatttca ccctcttgct gacaataaat ttgcactaac ttgcactagc 180

acacactttg cttttgcttg tgctgacggt actcgacata cctatcagct gcgtgcaaga 240

tcagtttcac caaaactttt catcagacaa gaggaggttc aacaagagct ctactcgcca 300

ctttttctca ttgttgctgc tctagtattt ttaatacttt gcttcaccat taagagaaag 360

acagaatga 369

<210> 16
<211> 135
<212> DNA
<213> SARS-CoV

<400> 16
atgaatgagc tcactttaat tgacttctat ttgtgctttt tagcctttct gctattcctt 60

gttttaataa tgcttattat attttggttt tcactcgaaa tccaggatct agaagaacct 120

tgtaccaaag tctaa 135

<210> 17
<211> 120
<212> DNA
<213> SARS-CoV

<400> 17
atgaaacttc tcattgtttt gacttgtatt tctctatgca gttgcatatg cactgtagta 60

cagcgctgtg catctaataa acctcatgtg cttgaagatc cttgtaaggt acaacactag 120

<210> 18
<211> 255
<212> DNA
<213> SARS-CoV

<400> 18
atgtgcttga agatccttgt aaggtacaac actaggggta atacttatag cactgcttgg 60

ctttgtgctc taggaaaggt tttacctttt catagatggc acactatggt tcaaacatgc 120

acacctaatg ttactatcaa ctgtcaagat ccagctggtg gtgcgcttat agctaggtgt 180

tggtaccttc atgaaggtca ccaaactgct gcatttagag acgtacttgt tgttttaaat 240

aaacgaacaa attaa 255


<210> 19
<211> 297
<212> DNA
<213> SARS-CoV

<400> 19
atggacccca atcaaaccaa cgtagtgccc cccgcattac atttggtgga cccacagatt 60

caactgacaa taaccagaat ggaggacgca atggggcaag gccaaaacag cgccgacccc 120

aaggtttacc caataatact gcgtcttggt tcacagctct cactcagcat ggcaaggagg 180

aacttagatt ccctcgaggc cagggcgttc caatcaacac caatagtggt ccagatgacc 240

aaattggcta ctaccgaaga gctacccgac gagttcgtgg tggtgacggc aaaatga 297

SEQUENCE LISTING

<110> Consejo Superior de Investigaciones Cienticicas

<120> SARS-VACCINE

<130> P69513

<160> 18

<170> PatentIn version 3.1

<210> 1
<211> 13149
<212> DNA
<213> SARS-CoV

<400> 1
atggagagcc ttgttcttgg tgtcaacgag aaaacacacg tccaactcag tttgcctgtc 60

cttcaggtta gagacgtgct agtgcgtggc ttcggggact ctgtggaaga ggccctatcg 120

gaggcacgtg aacacctcaa aaatggcact tgtggtctag tagagctgga aaaaggcgta 180

ctgccccagc ttgaacagcc ctatgtgttc attaaacgtt ctgatgcctt aagcaccaat 240

cacggccaca aggtcgttga gctggttgca gaaatggacg gcattcagta cggtcgtagc 300

ggtataacac tgggagtact cgtgccacat gtgggcgaaa ccccaattgc ataccgcaat 360

gttcttcttc gtaagaacgg taataaggga gccggtggtc atagctatgg catcgatcta 420

aagtcttatg acttaggtga cgagcttggc actgatccca ttgaagatta tgaacaaaac 480

tggaacacta agcatggcag tggtgcactc cgtgaactca ctcgtgagct caatggaggt 540

gcagtcactc gctatgtcga caacaatttc tgtggcccag atgggtaccc tcttgattgc 600

atcaaagatt ttctcgcacg cgcgggcaag tcaatgtgca ctctttccga acaacttgat 660

tacatcgagt cgaagagagg tgtctactgc tgccgtgacc atgagcatga aattgcctgg 720

ttcactgagc gctctgataa gagctacgag caccagacac ccttcgaaat taagagtgcc 780

aagaaatttg acactttcaa aggggaatgc ccaaagtttg tgtttcctct taactcaaaa 840

gtcaaagtca ttcaaccacg tgttgaaaag aaaaagactg agggtttcat ggggcgtata 900

cgctctgtgt accctgttgc atctccacag gagtgtaaca atatgcactt gtctaccttg 960

atgaaatgta atcattgcga tgaagtttca tggcagacgt gcgactttct gaaagccact 1020

tgtgaacatt gtggcactga aaatttagtt attgaaggac ctactacatg tgggtaccta 1080

cctactaatg ctgtagtgaa aatgccatgt cctgcctgtc aagacccaga gattggacct 1140

gagcatagtg ttgcagatta tcacaaccac tcaaacattg aaactcgact ccgcaaggga 1200

ggtaggacta gatgttttgg aggctgtgtg tttgcctatg ttggctgcta taataagcgt 1260

gcctactggg ttcctcgtgc tagtgctgat attggctcag gccatactgg cattactggt 1320
gacaatgtgg agaccttgaa tgaggatctc cttgagatac tgagtcgtga acgtgttaac 1380
attaacattg ttggcgattt tcatttgaat gaagaggttg ccatcatttt ggcatctttc 1440
tctgcttcta caagtgcctt tattgacact ataaagagtc ttgattacaa gtctttcaaa 1500
accattgttg agtcctgcgg taactataaa gttaccaagg gaaagcccgt aaaaggtgct 1560
tggaacattg gacaacagag atcagtttta acaccactgt gtggttttcc ctcacaggct 1620
gctggtgtta tcagatcaat ttttgcgcgc acacttgatg cagcaaacca ctcaattcct 1680
gatttgcaaa gagcagctgt caccatactt gatggtattt ctgaacagtc attacgtctt 1740
gtcgacgcca tggtttatac ttcagacctg ctcaccaaca gtgtcattat tatggcatat 1800
gtaactggtg gtcttgtaca acagacttct cagtggttgt ctaatctttt gggcactact 1860
gttgaaaaac tcaggcctat ctttgaatgg attgaggcga aacttagtgc aggagttgaa 1920
tttctcaagg atgcttggga gattctcaaa tttctcatta caggtgtttt tgacatcgtc 1980
aagggtcaaa tacaggttgc ttcagataac atcaaggatt gtgtaaaatg cttcattgat 2040
gttgttaaca aggcactcga aatgtgcatt gatcaagtca ctatcgctgg cgcaaagttg 2100
cgatcactca acttaggtga agtcttcatc gctcaaagca agggacttta ccgtcagtgt 2160
atacgtggca aggagcagct gcaactactc atgcctctta aggcaccaaa agaagtaacc 2220
tttcttgaag gtgattcaca tgacacagta cttacctctg aggaggttgt tctcaagaac 2280
ggtgaactcg aagcactcga gacgcccgtt gatagcttca caaatggagc tatcgttggc 2340
acaccagtct gtgtaaatgg cctcatgctc ttagagatta aggacaaaga acaatactgc 2400
gcattgtctc ctggtttact ggctacaaac aatgtctttc gcttaaaagg gggtgcacca 2460
attaaaggtg taacctttgg agaagatact gtttgggaag ttcaaggtta caagaatgtg 2520
agaatcacat ttgagcttga tgaacgtgtt gacaaagtgc ttaatgaaaa gtgctctgtc 2580
tacactgttg aatccggtac cgaagttact gagtttgcat gtgttgtagc agaggctgtt 2640
gtgaagactt tacaaccagt ttctgatctc cttaccaaca tgggtattga tcttgatgag 2700
tggagtgtag ctacattcta cttatttgat gatgctggtg aagaaaactt ttcatcacgt 2760
atgtattgtt ccttttaccc tccagatgag gaagaagagg acgatgcaga gtgtgaggaa 2820
gaagaaattg atgaaacctg tgaacatgag tacggtacag aggatgatta tcaaggtctc 2880
cctctggaat ttggtgcctc agctgaaaca gttcgagttg aggaagaaga agaggaagac 2940
tggctggatg atactactga gcaatcagag attgagccag aaccagaacc tacacctgaa 3000
gaaccagtta atcagtttac tggttattta aaacttactg acaatgttgc cattaaatgt 3060

gttgacatcg ttaaggaggc acaaagtgct aatcctatgg tgattgtaaa tgctgctaac 3120
atacacctga aacatggtgg tggtgtagca ggtgcactca acaaggcaac caatggtgcc 3180
atgcaaaagg agagtgatga ttacattaag ctaaatggcc ctcttacagt aggagggtct 3240
tgtttgcttt ctggacataa tcttgctaag aagtgtctgc atgttgttgg acctaaccta 3300
aatgcaggtg aggacatcca gcttcttaag gcagcatatg aaaatttcaa ttcacaggac 3360
atcttacttg caccattgtt gtcagcaggc atatttggtg ctaaaccact tcagtcttta 3420
caagtgtgcg tgcagacggt tcgtacacag gtttatattg cagtcaatga caaagctctt 3480
tatgagcagg ttgtcatgga ttatcttgat aacctgaagc ctagagtgga agcacctaaa 3540
caagaggagc caccaaacac agaagattcc aaaactgagg agaaatctgt cgtacagaag 3600
cctgtcgatg tgaagccaaa aattaaggcc tgcattgatg aggttaccac aacactggaa 3660
gaaactaagt ttcttaccaa taagttactc ttgtttgctg atatcaatgg taagctttac 3720
catgattctc agaacatgct tagaggtgaa gatatgtctt tccttgagaa ggatgcacct 3780
tacatggtag gtgatgttat cactagtggt gatatcactt gtgttgtaat accctccaaa 3840
aaggctggtg gcactactga gatgctctca agagctttga agaaagtgcc agttgatgag 3900
tatataacca cgtaccctgg acaaggatgt gctggttata cacttgagga agctaagact 3960
gctcttaaga aatgcaaatc tgcattttat gtactacctt cagaagcacc taatgctaag 4020
gaagagattc taggaactgt atcctggaat ttgagagaaa tgcttgctca tgctgaagag 4080
acaagaaaat taatgcctat atgcatggat gttagagcca taatggcaac catccaacgt 4140
aagtataaag gaattaaaat tcaagagggc atcgttgact atggtgtccg attcttcttt 4200
tatactagta aagagcctgt agcttctatt attacgaagc tgaactctct aaatgagccg 4260
cttgtcacaa tgccaattgg ttatgtgaca catggtttta atcttgaaga ggctgcgcgc 4320
tgtatgcgtt ctcttaaagc tcctgccgta gtgtcagtat catcaccaga tgctgttact 4380
acatataatg gatacctcac ttcgtcatca aagacatctg aggagcactt tgtagaaaca 4440
gtttctttgg ctggctctta cagagattgg tcctattcag gacagcgtac agagttaggt 4500
gttgaatttc ttaagcgtgg tgacaaaatt gtgtaccaca ctctggagag ccccgtcgag 4560
tttcatcttg acggtgaggt tctttcactt gacaaactaa agagtctctt atccctgcgg 4620
gaggttaaga ctataaaagt gttcacaact gtggacaaca ctaatctcca cacacagctt 4680
gtggatatgt ctatgacata tggacagcag tttggtccaa catacttgga tggtgctgat 4740
gttacaaaaa ttaaacctca tgtaaatcat gagggtaaga ctttctttgt actacctagt 4800
gatgacacac tacgtagtga agctttcgag tactaccata ctcttgatga gagttttctt 4860

ggtaggtaca tgtctgcttt aaaccacaca aagaaatgga aatttcctca agttggtggt 4920

ttaacttcaa ttaaatgggc tgataacaat tgttatttgt ctagtgtttt attagcactt 4980

caacagcttg aagtcaaatt caatgcacca gcacttcaag aggcttatta tagagcccgt 5040

gctggtgatg ctgctaactt ttgtgcactc atactcgctt acagtaataa aactgttggc 5100

gagcttggtg atgtcagaga aactatgacc catcttctac agcatgctaa tttggaatct 5160

gcaaagcgag ttcttaatgt ggtgtgtaaa cattgtggtc agaaaactac taccttaacg 5220

ggtgtagaag ctgtgatgta tatgggtact ctatcttatg ataatcttaa gacaggtgtt 5280

tccattccat gtgtgtgtgg tcgtgatgct acacaatatc tagtacaaca agagtcttct 5340

tttgttatga tgtctgcacc acctgctgag tataaattac agcaaggtac attcttatgt 5400

gcgaatgagt acactggtaa ctatcagtgt ggtcattaca ctcatataac tgctaaggag 5460

accctctatc gtattgacgg agctcacctt acaaagatgt cagagtacaa aggaccagtg 5520

actgatgttt tctacaagga aacatcttac actacaacca tcaagcctgt gtcgtataaa 5580

ctcgatggag ttacttacac agagattgaa ccaaaattgg atgggtatta taaaaaggat 5640

aatgcttact atacagagca gcctatagac cttgtaccaa ctcaaccatt accaaatgcg 5700

agttttgata atttcaaact cacatgttct aacacaaaat ttgctgatga tttaaatcaa 5760

atgacaggct tcacaaagcc agcttcacga gagctatctg tcacattctt cccagacttg 5820

aatggcgatg tagtggctat tgactataga cactattcag cgagtttcaa gaaaggtgct 5880

aaattactgc ataagccaat tgtttggcac attaaccagg ctacaaccaa gacaacgttc 5940

aaaccaaaca cttggtgttt acgttgtctt tggagtacaa agccagtaga tacttcaaat 6000

tcatttgaag ttctggcagt agaagacaca caaggaatgg acaatcttgc ttgtgaaagt 6060

caacaaccca cctctgaaga agtagtggaa aatcctacca tacagaagga agtcatagag 6120

tgtgacgtga aaactaccga agttgtaggc aatgtcatac ttaaaccatc agatgaaggt 6180

gttaaagtaa cacaagagtt aggtcatgag gatcttatgg ctgcttatgt ggaaaacaca 6240

agcattacca ttaagaaacc taatgagctt tcactagcct taggtttaaa aacaattgcc 6300

actcatggta ttgctgcaat taatagtgtt ccttggagta aaattttggc ttatgtcaaa 6360

ccattcttag gacaagcagc aattacaaca tcaaattgcg ctaagagatt agcacaacgt 6420

gtgtttaaca attatatgcc ttatgtgttt acattattgt tccaattgtg tacttttact 6480

aaaagtacca attctagaat tagagcttca ctacctacaa ctattgctaa aaatagtgtt 6540

aagagtgttg ctaaattatg tttggatgcc ggcattaatt atgtgaagtc acccaaattt 6600

tctaaattgt tcacaatcgc tatgtggcta ttgttgttaa gtatttgctt aggttctcta 6660

atctgtgtaa ctgctgcttt tggtgtactc ttatctaatt ttggtgctcc ttcttattgt 6720
aatggcgtta gagaattgta tcttaattcg tctaacgtta ctactatgga tttctgtgaa 6780
ggttcttttc cttgcagcat ttgtttaagt ggattagact cccttgattc ttatccagct 6840
cttgaaacca ttcaggtgac gatttcatcg tacaagctag acttgacaat tttaggtctg 6900
gccgctgagt gggttttggc atatatgttg ttcacaaaat tcttttattt attaggtctt 6960
tcagctataa tgcaggtgtt ctttggctat tttgctagtc atttcatcag caattcttgg 7020
ctcatgtggt ttatcattag tattgtacaa atggcacccg tttctgcaat ggttaggatg 7080
tacatcttct ttgcttcttt ctactacata tggaagagct atgttcatat catggatggt 7140
tgcacctctt cgacttgcat gatgtgctat aagcgcaatc gtgccacacg cgttgagtgt 7200
acaactattg ttaatggcat gaagagatct ttctatgtct atgcaaatgg aggccgtggc 7260
ttctgcaaga ctcacaattg gaattgtctc aattgtgaca cattttgcac tggtagtaca 7320
ttcattagtg atgaagttgc tcgtgatttg tcactccagt ttaaaagacc aatcaaccct 7380
actgaccagt catcgtatat tgttgatagt gttgctgtga aaaatggcgc gcttcacctc 7440
tactttgaca aggctggtca aaagacctat gagagacatc cgctctccca ttttgtcaat 7500
ttagacaatt tgagagctaa caacactaaa ggttcactgc ctattaatgt catagttttt 7560
gatggcaagt ccaaatgcga cgagtctgct tctaagtctg cttctgtgta ctacagtcag 7620
ctgatgtgcc aacctattct gttgcttgac caagttcttg tatcagacgt tggagatagt 7680
actgaagttt ccgttaagat gtttgatgct tatgtcgaca ccttttcagc aacttttagt 7740
gttcctatgg aaaaacttaa ggcacttgtt gctacagctc acagcgagtt agcaaagggt 7800
gtagctttag atggtgtcct ttctacattc gtgtcagctg cccgacaagg tgttgttgat 7860
accgatgttg acacaaagga tgttattgaa tgtctcaaac tttcacatca ctctgactta 7920
gaagtgacag gtgacagttg taacaatttc atgctcacct ataataaggt tgaaaacatg 7980
acgcccagag atcttggcgc atgtattgac tgtaatgcaa ggcatatcaa tgcccaagta 8040
gcaaaaagtc acaatgtttc actcatctgg aatgtaaaag actacatgtc tttatctgaa 8100
cagctgcgta aacaaattcg tagtgctgcc aagaagaaca acataccttt tagactaact 8160
tgtgctacaa ctagacaggt tgtcaatgtc ataactacta aaatctcact caagggtggt 8220
aagattgtta gtacttgttt taaacttatg cttaaggcca cattattgtg cgttcttgct 8280
gcattggttt gttatatcgt tatgccagta catacattgt caatccatga tggttacaca 8340
aatgaaatca ttggttacaa agccattcag gatggtgtca ctcgtgacat catttctact 8400
gatgattgtt ttgcaaataa acatgctggt tttgacgcat ggtttagcca gcgtggtggt 8460

tcatacaaaa atgacaaaag ctgccctgta gtagctgcta tcattacaag agagattggt 8520
ttcatagtgc ctggcttacc gggtactgtg ctgagagcaa tcaatggtga cttcttgcat 8580
tttctacctc gtgtttttag tgctgttggc aacatttgct acacaccttc caaactcatt 8640
gagtatagtg attttgctac ctctgcttgc gttcttgctg ctgagtgtac aatttttaag 8700
gatgctatgg gcaaacctgt gccatattgt tatgacacta atttgctaga gggttctatt 8760
tcttatagtg agcttcgtcc agacactcgt tatgtgctta tggatggttc catcatacag 8820
tttcctaaca cttacctgga gggttctgtt agagtagtaa caacttttga tgctgagtac 8880
tgtagacatg gtacatgcga aaggtcagaa gtaggtattt gcctatctac cagtggtaga 8940
tgggttctta ataatgagca ttacagagct ctatcaggag ttttctgtgg tgttgatgcg 9000
atgaatctca tagctaacat ctttactcct cttgtgcaac ctgtgggtgc tttagatgtg 9060
tctgcttcag tagtggctgg tggtattatt gccatattgg tgacttgtgc tgcctactac 9120
tttatgaaat tcagacgtgt ttttggtgag tacaaccatg ttgttgctgc taatgcactt 9180
ttgtttttga tgtctttcac tatactctgt ctggtaccag cttacagctt tctgccggga 9240
gtctactcag tcttttactt gtacttgaca ttctatttca ccaatgatgt ttcattcttg 9300
gctcaccttc aatggtttgc catgttttct cctattgtgc ctttttggat aacagcaatc 9360
tatgtattct gtatttctct gaagcactgc cattggttct ttaacaacta tcttaggaaa 9420
agagtcatgt ttaatggagt tacatttagt accttcgagg aggctgcttt gtgtaccttt 9480
ttgctcaaca aggaaatgta cctaaaattg cgtagcgaga cactgttgcc acttacacag 9540
tataacaggt atcttgctct atataacaag tacaagtatt tcagtggagc cttagatact 9600
accagctatc gtgaagcagc ttgctgccac ttagcaaagg ctctaaatga ctttagcaac 9660
tcaggtgctg atgttctcta ccaaccacca cagacatcaa tcacttctgc tgttctgcag 9720
agtggtttta ggaaaatggc attcccgtca ggcaaagttg aagggtgcat ggtacaagta 9780
acctgtggaa ctacaactct taatggattg tggttggatg acacagtata ctgtccaaga 9840
catgtcattt gcacagcaga agacatgctt aatcctaact atgaagatct gctcattcgc 9900
aaatccaacc atagctttct tgttcaggct ggcaatgttc aacttcgtgt tattggccat 9960
tctatgcaaa attgtctgct taggcttaaa gttgatactt ctaaccctaa gacacccaag 10020
tataaatttg tccgtatcca acctggtcaa acattttcag ttctagcatg ctacaatggt 10080
tcaccatctg gtgtttatca gtgtgccatg agacctaatc ataccattaa aggttctttc 10140
cttaatggat catgtggtag tgttggtttt aacattgatt atgattgcgt gtctttctgc 10200
tatatgcatc atatggagct tccaacagga gtacacgctg gtactgactt agaaggtaaa 10260

ttctatggtc catttgttga cagacaaact gcacaggctg caggtacaga cacaaccata 10320
acattaaatg ttttggcatg gctgtatgct gctgttatca atggtgatag gtggtttctt 10380
aatagattca ccactacttt gaatgacttt aaccttgtgg caatgaagta caactatgaa 10440
cctttgacac aagatcatgt tgacatattg ggacctcttt ctgctcaaac aggaattgcc 10500
gtcttagata tgtgtgctgc tttgaaagag ctgctgcaga atggtatgaa tggtcgtact 10560
atccttggta gcactatttt agaagatgag tttacaccat ttgatgttgt tagacaatgc 10620
tctggtgtta ccttccaagg taagttcaag aaaattgtta agggcactca tcattggatg 10680
cttttaactt tcttgacatc actattgatt cttgttcaaa gtacacagtg gtcactgttt 10740
ttctttgttt acgagaatgc tttcttgcca tttactcttg gtattatggc aattgctgca 10800
tgtgctatgc tgcttgttaa gcataagcac gcattcttgt gcttgtttct gttaccttct 10860
cttgcaacag ttgcttactt taatatggtc tacatgcctg ctagctgggt gatgcgtatc 10920
atgacatggc ttgaattggc tgacactagc ttgtctggtt ataggcttaa ggattgtgtt 10980
atgtatgctt cagctttagt tttgcttatt ctcatgacag ctcgcactgt ttatgatgat 11040
gctgctagac gtgtttggac actgatgaat gtcattacac ttgtttacaa agtctactat 11100
ggtaatgctt tagatcaagc tatttccatg tgggccttag ttatttctgt aacctctaac 11160
tattctggtg tcgttacgac tatcatgttt ttagctagag ctatagtgtt tgtgtgtgtt 11220
gagtattacc cattgttatt tattactggc aacaccttac agtgtatcat gcttgtttat 11280
tgtttcttag gctattgttg ctgctgctac tttggccttt tctgtttact caaccgttac 11340
ttcaggctta ctcttggtgt ttatgactac ttggtctcta cacaagaatt taggtatatg 11400
aactcccagg ggcttttgcc tcctaagagt agtattgatg ctttcaagct taacattaag 11460
ttgttgggta ttggaggtaa accatgtatc aaggttgcta ctgtacagtc taaaatgtct 11520
gacgtaaagt gcacatctgt ggtactgctc tcggttcttc aacaacttag agtagagtca 11580
tcttctaaat tgtgggcaca atgtgtacaa ctccacaatg atattcttct tgcaaaagac 11640
acaactgaag ctttcgagaa gatggtttct cttttgtctg ttttgctatc catgcagggt 11700
gctgtagaca ttaataggtt gtgcgaggaa atgctcgata accgtgctac tcttcaggct 11760
attgcttcag aatttagttc tttaccatca tatgccgctt atgccactgc ccaggaggcc 11820
tatgagcagg ctgtagctaa tggtgattct gaagtcgttc tcaaaaagtt aaagaaatct 11880
ttgaatgtgg ctaaatctga gtttgaccgt gatgctgcca tgcaacgcaa gttggaaaag 11940
atggcagatc aggctatgac ccaaatgtac aaacaggcaa gatctgagga caagagggca 12000
aaagtaacta gtgctatgca aacaatgctc ttcactatgc ttaggaagct tgataatgat 12060

gcacttaaca acattatcaa caatgcgcgt gatggttgtg ttccactcaa catcatacca 12120
ttgactacag cagccaaact catggttgtt gtccctgatt atggtaccta caagaacact 12180
tgtgatggta acacctttac atatgcatct gcactctggg aaatccagca agttgttgat 12240
gcggatagca agattgttca acttagtgaa attaacatgg acaattcacc aaatttggct 12300
tggcctctta ttgttacagc tctaagagcc aactcagctg ttaaactaca gaataatgaa 12360
ctgagtccag tagcactacg acagatgtcc tgtgcggctg gtaccacaca aacagcttgt 12420
actgatgaca atgcacttgc ctactataac aattcgaagg gaggtaggtt tgtgctggca 12480
ttactatcag accaccaaga tctcaaatgg gctagattcc ctaagagtga tggtacaggt 12540
acaatttaca cagaactgga accaccttgt aggtttgtta cagacacacc aaaagggcct 12600
aaagtgaaat acttgtactt catcaaaggc ttaaacaacc taaatagagg tatggtgctg 12660
ggcagtttag ctgctacagt acgtcttcag gctggaaatg ctacagaagt acctgccaat 12720
tcaactgtgc tttccttctg tgcttttgca gtagaccctg ctaaagcata taaggattac 12780
ctagcaagtg gaggacaacc aatcaccaac tgtgtgaaga tgttgtgtac acacactggt 12840
acaggacagg caattactgt aacaccagaa gctaacatgg accaagagtc ctttggtggt 12900
gcttcatgtt gtctgtattg tagatgccac attgaccatc caaatcctaa aggattctgt 12960
gacttgaaag gtaagtacgt ccaaatacct accacttgtg ctaatgaccc agtgggtttt 13020
acacttagaa acacagtctg taccgtctgc ggaatgtgga aaggttatgg ctgtagttgt 13080
gaccaactcc gcgaaccctt gatgcagtct gcggatgcat caacgttttt aaacgggttt 13140
gcggtgtaa 13149

<210> 2
<211> 8088
<212> DNA
<213> SARS-CoV

<400> 2
cgggtttgcg gtgtaagtgc agcccgtctt acaccgtgcg gcacaggcac tagtactgat 60
gtcgtctaca gggcttttga tatttacaac gaaaaagttg ctggttttgc aaagttccta 120
aaaactaatt gctgtcgctt ccaggagaag gatgaggaag gcaatttatt agactcttac 180
tttgtagtta agaggcatac tatgtctaac taccaacatg aagagactat ttataacttg 240
gttaaagatt gtccagcggt tgctgtccat gactttttca agtttagagt agatggtgac 300
atggtaccac atatatcacg tcagcgtcta actaaataca caatggctga tttagtctat 360
gctctacgtc attttgatga gggtaattgt gatacattaa aagaaatact cgtcacatac 420
aattgctgtg atgatgatta tttcaataag aaggattggt atgacttcgt agagaatcct 480

gacatcttac gcgtatatgc taacttaggt gagcgtgtac gccaatcatt attaaagact 540
gtacaattct gcgatgctat gcgtgatgca ggcattgtag gcgtactgac attagataat 600
caggatctta atgggaactg gtacgatttc ggtgatttcg tacaagtagc accaggctgc 660
ggagttccta ttgtggattc atattactca ttgctgatgc ccatcctcac tttgactagg 720
gcattggctg ctgagtccca tatggatgct gatctcgcaa aaccacttat taagtgggat 780
ttgctgaaat atgattttac ggaagagaga ctttgtctct tcgaccgtta ttttaaatat 840
tgggaccaga cataccatcc caattgtatt aactgtttgg atgataggtg tatccttcat 900
tgtgcaaact ttaatgtgtt attttctact gtgtttccac ctacaagttt tggaccacta 960
gtaagaaaaa tatttgtaga tggtgttcct tttgttgttt caactggata ccattttcgt 1020
gagttaggag tcgtacataa tcaggatgta aacttacata gctcgcgtct cagtttcaag 1080
gaacttttag tgtatgctgc tgatccagct atgcatgcag cttctggcaa tttattgcta 1140
gataaacgca ctacatgctt ttcagtagct gcactaacaa acaatgttgc ttttcaaact 1200
gtcaaacccg gtaattttaa taaagacttt tatgactttg ctgtgtctaa aggtttcttt 1260
aaggaaggaa gttctgttga actaaaacac ttcttctttg ctcaggatgg caacgctgct 1320
atcagtgatt atgactatta tcgttataat ctgccaacaa tgtgtgatat cagacaactc 1380
ctattcgtag ttgaagttgt tgataaatac tttgattgtt acgatggtgg ctgtattaat 1440
gccaaccaag taatcgttaa caatctggat aaatcagctg gtttcccatt taataaatgg 1500
ggtaaggcta gactttatta tgactcaatg agttatgagg atcaagatgc acttttcgcg 1560
tatactaagc gtaatgtcat ccctactata actcaaatga atcttaagta tgccattagt 1620
gcaaagaata gagctcgcac cgtagctggt gtctctatct gtagtactat gacaaataga 1680
cagtttcatc agaaattatt gaagtcaata gccgccacta gaggagctac tgtggtaatt 1740
ggaacaagca agttttacgg tggctggcat aatatgttaa aaactgttta cagtgatgta 1800
gaaactccac accttatggg ttgggattat ccaaaatgtg acagagccat gcctaacatg 1860
cttaggataa tggcctctct tgttcttgct cgcaaacata acacttgctg taacttatca 1920
caccgtttct acaggttagc taacgagtgt gcgcaagtat taagtgagat ggtcatgtgt 1980
ggcggctcac tatatgttaa accaggtgga acatcatccg gtgatgctac aactgcttat 2040
gctaatagtg tctttaacat ttgtcaagct gttacagcca atgtaaatgc acttctttca 2100
actgatggta ataagatagc tgacaagtat gtccgcaatc tacaacacag gctctatgag 2160
tgtctctata gaaataggga tgttgatcat gaattcgtgg atgagtttta cgcttacctg 2220
cgtaaacatt tctccatgat gattctttct gatgatgccg ttgtgtgcta taacagtaac 2280

tatgcggctc aaggtttagt agctagcatt aagaacttta aggcagttct ttattatcaa 2340
aataatgtgt tcatgtctga ggcaaaatgt tggactgaga ctgaccttac taaaggacct 2400
cacgaatttt gctcacagca tacaatgcta gttaaacaag gagatgatta cgtgtacctg 2460
ccttacccag atccatcaag aatattaggc gcaggctgtt ttgtcgatga tattgtcaaa 2520
acagatggta cacttatgat tgaaaggttc gtgtcactgg ctattgatgc ttacccactt 2580
acaaaacatc ctaatcagga gtatgctgat gtctttcact tgtatttaca atacattaga 2640
aagttacatg atgagcttac tggccacatg ttggacatgt attccgtaat gctaactaat 2700
gataacacct cacggtactg ggaacctgag ttttatgagg ctatgtacac accacataca 2760
gtcttgcagg ctgtaggtgc ttgtgtattg tgcaattcac agacttcact tcgttgcggt 2820
gcctgtatta ggagaccatt cctatgttgc aagtgctgct atgaccatgt catttcaaca 2880
tcacacaaat tagtgttgtc tgttaatccc tatgtttgca atgccccagg ttgtgatgtc 2940
actgatgtga cacaactgta tctaggaggt atgagctatt attgcaagtc acataagcct 3000
cccattagtt ttccattatg tgctaatggt caggtttttg gtttatacaa aaacacatgt 3060
gtaggcagtg acaatgtcac tgacttcaat gcgatagcaa catgtgattg gactaatgct 3120
ggcgattaca tacttgccaa cacttgtact gagagactca agcttttcgc agcagaaacg 3180
ctcaaagcca ctgaggaaac atttaagctg tcatatggta ttgctactgt acgcgaagta 3240
ctctctgaca gagaattgca tctttcatgg gaggttggaa aacctagacc accattgaac 3300
agaaactatg tctttactgg ttaccgtgta actaaaaata gtaaagtaca gattggagag 3360
tacacctttg aaaaaggtga ctatggtgat gctgttgtgt acagaggtac tacgacatac 3420
aagttgaatg ttggtgatta ctttgtgttg acatctcaca ctgtaatgcc acttagtgca 3480
cctactctag tgccacaaga gcactatgtg agaattactg gcttgtaccc aacactcaac 3540
atctcagatg agttttctag caatgttgca aattatcaaa aggtcggcat gcaaaagtac 3600
tctacactcc aaggaccacc tggtactggt aagagtcatt ttgccatcgg acttgctctc 3660
tattacccat ctgctcgcat agtgtatacg gcatgctctc atgcagctgt tgatgcccta 3720
tgtgaaaagg cattaaaata tttgcccata gataaatgta gtagaatcat acctgcgcgt 3780
gcgcgcgtag agtgttttga taaattcaaa gtgaattcaa cactagaaca gtatgttttc 3840
tgcactgtaa atgcattgcc agaaacaact gctgacattg tagtctttga tgaaatctct 3900
atggctacta attatgactt gagtgttgtc aatgctagac ttcgtgcaaa acactacgtc 3960
tatattggcg atcctgctca attaccagcc ccccgcacat tgctgactaa aggcacacta 4020
gaaccagaat attttaattc agtgtgcaga cttatgaaaa caataggtcc agacatgttc 4080

cttggaactt gtcgccgttg tcctgctgaa attgttgaca ctgtgagtgc tttagtttat 4140
gacaataagc taaaagcaca caaggataag tcagctcaat gcttcaaaat gttctacaaa 4200
ggtgttatta cacatgatgt ttcatctgca atcaacagac ctcaaatagg cgttgtaaga 4260
gaatttctta cacgcaatcc tgcttggaga aaagctgttt ttatctcacc ttataattca 4320
cagaacgctg tagcttcaaa aatcttagga ttgcctacgc agactgttga ttcatcacag 4380
ggttctgaat atgactatgt catattcaca caaactactg aaacagcaca ctcttgtaat 4440
gtcaaccgct tcaatgtggc tatcacaagg gcaaaaattg gcattttgtg cataatgtct 4500
gatagagatc tttatgacaa actgcaattt acaagtctag aaataccacg tcgcaatgtg 4560
gctacattac aagcagaaaa tgtaactgga ctttttaagg actgtagtaa gatcattact 4620
ggtcttcatc ctacacaggc acctacacac ctcagcgttg atataaagtt caagactgaa 4680
ggattatgtg ttgacatacc aggcatacca aaggacatga cctaccgtag actcatctct 4740
atgatgggtt tcaaaatgaa ttaccaagtc aatggttacc ctaatatgtt tatcacccgc 4800
gaagaagcta ttcgtcacgt tcgtgcgtgg attggctttg atgtagaggg ctgtcatgca 4860
actagagatg ctgtgggtac taacctacct ctccagctag gattttctac aggtgttaac 4920
ttagtagctg taccgactgg ttatgttgac actgaaaata acacagaatt caccagagtt 4980
aatgcaaaac ctccaccagg tgaccagttt aaacatctta taccactcat gtataaaggc 5040
ttgccctgga atgtagtgcg tattaagata gtacaaatgc tcagtgatac actgaaagga 5100
ttgtcagaca gagtcgtgtt cgtcctttgg gcgcatggct ttgagcttac atcaatgaag 5160
tactttgtca agattggacc tgaaagaacg tgttgtctgt gtgacaaacg tgcaacttgc 5220
ttttctactt catcagatac ttatgcctgc tggaatcatt ctgtgggttt tgactatgtc 5280
tataacccat ttatgattga tgttcagcag tggggcttta cgggtaacct tcagagtaac 5340
catgaccaac attgccaggt acatggaaat gcacatgtgg ctagttgtga tgctatcatg 5400
actagatgtt tagcagtcca tgagtgcttt gttaagcgcg ttgattggtc tgttgaatac 5460
cctattatag gagatgaact gagggttaat tctgcttgca gaaaagtaca acacatggtt 5520
gtgaagtctg cattgcttgc tgataagttt ccagttcttc atgacattgg aaatccaaag 5580
gctatcaagt gtgtgcctca ggctgaagta gaatggaagt tctacgatgc tcagccatgt 5640
agtgacaaag cttacaaaat agaggagctc ttctattctt atgctacaca tcacgataaa 5700
ttcactgatg gtgtttgttt gttttggaat tgtaacgttg atcgttaccc agccaatgca 5760
attgtgtgta ggtttgacac aagagtcttg tcaaacttga acttaccagg ctgtgatggt 5820
ggtagtttgt atgtgaataa gcatgcattc cacactccag ctttcgataa aagtgcattt 5880

actaatttaa agcaattgcc tttcttttac tattctgata gtccttgtga gtctcatggc 5940
aaacaagtag tgtcggatat tgattatgtt ccactcaaat ctgctacgtg tattacacga 6000
tgcaatttag gtggtgctgt ttgcagacac catgcaaatg agtaccgaca gtacttggat 6060
gcatataata tgatgatttc tgctggattt agcctatgga tttacaaaca atttgatact 6120
tataacctgt ggaatacatt taccaggtta cagagtttag aaaatgtggc ttataatgtt 6180
gttaataaag gacactttga tggacacgcc ggcgaagcac ctgtttccat cattaataat 6240
gctgtttaca caaaggtaga tggtattgat gtggagatct ttgaaaataa gacaacactt 6300
cctgttaatg ttgcatttga gctttgggct aagcgtaaca ttaaaccagt gccagagatt 6360
aagatactca ataatttggg tgttgatatc gctgctaata ctgtaatctg ggactacaaa 6420
agagaagccc cagcacatgt atctacaata ggtgtctgca caatgactga cattgccaag 6480
aaacctactg agagtgcttg ttcttcactt actgtcttgt ttgatggtag agtggaagga 6540
caggtagacc tttttagaaa cgcccgtaat ggtgttttaa taacagaagg ttcagtcaaa 6600
ggtctaacac cttcaaaggg accagcacaa gctagcgtca atggagtcac attaattgga 6660
gaatcagtaa aaacacagtt taactacttt aagaaagtag acggcattat tcaacagttg 6720
cctgaaacct actttactca gagcagagac ttagaggatt ttaagcccag atcacaaatg 6780
gaaactgact ttctcgagct cgctatggat gaattcatac agcgatataa gctcgagggc 6840
tatgccttcg aacacatcgt ttatggagat ttcagtcatg gacaacttgg cggtcttcat 6900
ttaatgatag gcttagccaa gcgctcacaa gattcaccac ttaaattaga ggattttatc 6960
cctatggaca gcacagtgaa aaattacttc ataacagatg cgcaaacagg ttcatcaaaa 7020
tgtgtgtgtt ctgtgattga tcttttactt gatgactttg tcgagataat aaagtcacaa 7080
gatttgtcag tgatttcaaa agtggtcaag gttacaattg actatgctga aatttcattc 7140
atgctttggt gtaaggatgg acatgttgaa accttctacc caaaactaca agcaagtcaa 7200
gcgtggcaac caggtgttgc gatgcctaac ttgtacaaga tgcaaagaat gcttcttgaa 7260
aagtgtgacc ttcagaatta tggtgaaaat gctgttatac caaaaggaat aatgatgaat 7320
gtcgcaaagt atactcaact gtgtcaatac ttaaatacac ttactttagc tgtaccctac 7380
aacatgagag ttattcactt tggtgctggc tctgataaag gagttgcacc aggtacagct 7440
gtgctcagac aatggttgcc aactggcaca ctacttgtcg attcagatct taatgacttc 7500
gtctccgacg cagattctac tttaattgga gactgtgcaa cagtacatac ggctaataaa 7560
tgggacctta ttattagcga tatgtatgac cctaggacca aacatgtgac aaaagagaat 7620
gactctaaag aagggttttt cacttatctg tgtggattta taaagcaaaa actagccctg 7680

ggtggttcta tagctgtaaa gataacagag cattcttgga atgctgacct ttacaagctt 7740

atgggccatt tctcatggtg gacagctttt gttacaaatg taaatgcatc atcatcggaa 7800

gcatttttaa ttggggctaa ctatcttggc aagccgaagg aacaaattga tggctatacc 7860

atgcatgcta actacatttt ctggaggaac acaaatccta tccagttgtc ttcctattca 7920

ctctttgaca tgagcaaatt tcctcttaaa ttaagaggaa ctgctgtaat gtctcttaag 7980

gagaatcaaa tcaatgatat gatttattct cttctggaaa aaggtaggct tatcattaga 8040

gaaaacaaca gagttgtggt ttcaagtgat attcttgtta acaactaa 8088

<210> 3
<211> 1269
<212> DNA
<213> SARS-CoV

<400> 3
atgtctgata atggacccca atcaaaccaa cgtagtgccc cccgcattac atttggtgga 60

cccacagatt caactgacaa taaccagaat ggaggacgca atggggcaag gccaaaacag 120

cgccgacccc aaggtttacc caataatact gcgtcttggt tcacagctct cactcagcat 180

ggcaaggagg aacttagatt ccctcgaggc cagggcgttc caatcaacac caatagtggt 240

ccagatgacc aaattggcta ctaccgaaga gctacccgac gagttcgtgg tggtgacggc 300

aaaatgaaag agctcagccc cagatggtac ttctattacc taggaactgg cccagaagct 360

tcacttccct acggcgctaa caaagaaggc atcgtatggg ttgcaactga gggagccttg 420

aatacaccca aagaccacat tggcacccgc aatcctaata acaatgctgc caccgtgcta 480

caacttcctc aaggaacaac attgccaaaa ggcttctacg cagagggaag cagaggcggc 540

agtcaagcct cttctcgctc ctcatcacgt agtcgcggta attcaagaaa ttcaactcct 600

ggcagcagta ggggaaattc tcctgctcga atggctagcg gaggtggtga aactgccctc 660

gcgctattgc tgctagacag attgaaccag cttgagagca aagtttctgg taaaggccaa 720

caacaacaag gccaaactgt cactaagaaa tctgctgctg aggcatctaa aaagcctcgc 780

caaaaacgta ctgccacaaa acagtacaac gtcactcaag catttgggag acgtggtcca 840

gaacaaaccc aaggaaattt cggggaccaa gacctaatca gacaaggaac tgattacaaa 900

cattggccgc aaattgcaca atttgctcca agtgcctctg cattctttgg aatgtcacgc 960

attggcatgg aagtcacacc ttcgggaaca tggctgactt atcatggagc cattaaattg 1020

gatgacaaag atccacaatt caaagacaac gtcatactgc tgaacaagca cattgacgca 1080

tacaaaacat tcccaccaac agagcctaaa aaggacaaaa agaaaaagac tgatgaagct 1140

cagcctttgc cgcagagaca aaagaagcag cccactgtga ctcttcttcc tgcggctgac 1200

atggatgatt tctccagaca acttcaaaat tccatgagtg gagcttctgc tgattcaact 1260

caggcataa 1269

<210> 4
<211> 3768
<212> DNA
<213> SARS-CoV

<400> 4
atgtttattt tcttattatt tcttactctc actagtggta gtgaccttga ccggtgcacc 60

acttttgatg atgttcaagc tcctaattac actcaacata cttcatctat gaggggggtt 120

tactatcctg atgaaatttt tagatcagac actctttatt taactcagga tttatttctt 180

ccattttatt ctaatgttac agggtttcat actattaatc atacgtttgg caaccctgtc 240

atacctttta aggatggtat ttattttgct gccacagaga aatcaaatgt tgtccgtggt 300

tgggtttttg gttctaccat gaacaacaag tcacagtcgg tgattattat taacaattct 360

actaatgttg ttatacgagc atgtaacttt gaattgtgtg acaacccttt ctttgctgtt 420

tctaaaccca tgggtacaca gacacatact atgatattcg ataatgcatt taattgcact 480

ttcgagtaca tatctgatgc cttttcgctt gatgtttcag aaaagtcagg taattttaaa 540

cacttacgag agtttgtgtt taaaaataaa gatgggtttc tctatgttta taagggctat 600

caacctatag atgtagttcg tgatctacct tctggtttta acactttgaa acctattttt 660

aagttgcctc ttggtattaa cattacaaat tttagagcca ttcttacagc cttttcacct 720

gctcaagaca tttggggcac gtcagctgca gcctattttg ttggctattt aaagccaact 780

acatttatgc tcaagtatga tgaaaatggt acaatcacag atgctgttga ttgttctcaa 840

aatccacttg ctgaactcaa atgctctgtt aagagctttg agattgacaa aggaatttac 900

cagacctcta atttcagggt tgttccctca ggagatgttg tgagattccc taatattaca 960

aacttgtgtc cttttggaga ggtttttaat gctactaaat tcccttctgt ctatgcatgg 1020

gagagaaaaa aaatttctaa ttgtgttgct gattactctg tgctctacaa ctcaacattt 1080

ttttcaacct ttaagtgcta tggcgtttct gccactaagt tgaatgatct ttgcttctcc 1140

aatgtctatg cagattcttt tgtagtcaag ggagatgatg taagacaaat agcgccagga 1200

caaactggtg ttattgctga ttataattat aaattgccag atgatttcat gggttgtgtc 1260

cttgcttgga atactaggaa cattgatgct acttcaactg gtaattataa ttataaatat 1320

aggtatctta gacatggcaa gcttaggccc tttgagagag acatatctaa tgtgcctttc 1380

tcccctgatg gcaaaccttg caccccacct gctcttaatt gttattggcc attaaatgat 1440

tatggttttt acaccactac tggcattggc taccaacctt acagagttgt agtactttct 1500
tttgaacttt taaatgcacc ggccacggtt tgtggaccaa aattatccac tgaccttatt 1560
aagaaccagt gtgtcaattt taattttaat ggactcactg gtactggtgt gttaactcct 1620
tcttcaaaga gatttcaacc atttcaacaa tttggccgtg atgtttctga tttcactgat 1680
tccgttcgag atcctaaaac atctgaaata ttagacattt caccttgctc ttttgggggt 1740
gtaagtgtaa ttacacctgg aacaaatgct tcatctgaag ttgctgttct atatcaagat 1800
gttaactgca ctgatgtttc tacagcaatt catgcagatc aactcacacc agcttggcgc 1860
atatattcta ctggaaacaa tgtattccag actcaagcag gctgtcttat aggagctgag 1920
catgtcgaca cttcttatga gtgcgacatt cctattggag ctggcatttg tgctagttac 1980
catacagttt ctttattacg tagtactagc caaaaatcta ttgtggctta tactatgtct 2040
ttaggtgctg atagttcaat tgcttactct aataacacca ttgctatacc tactaacttt 2100
tcaattagca ttactacaga agtaatgcct gtttctatgg ctaaaacctc cgtagattgt 2160
aatatgtaca tctgcggaga ttctactgaa tgtgctaatt tgcttctcca atatggtagc 2220
ttttgcacac aactaaatcg tgcactctca ggtattgctg ctgaacagga tcgcaacaca 2280
cgtgaagtgt tcgctcaagt caaacaaatg tacaaaaccc caactttgaa atattttggt 2340
ggttttaatt tttcacaaat attacctgac cctctaaagc caactaagag gtcttttatt 2400
gaggacttgc tctttaataa ggtgacactc gctgatgctg gcttcatgaa gcaatatggc 2460
gaatgcctag gtgatattaa tgctagagat ctcatttgtg cgcagaagtt caatggactt 2520
acagtgttgc cacctctgct cactgatgat atgattgctg cctacactgc tgctctagtt 2580
agtggtactg ccactgctgg atggacattt ggtgctggcg ctgctcttca aatacctttt 2640
gctatgcaaa tggcatatag gttcaatggc attggagtta cccaaaatgt tctctatgag 2700
aaccaaaaac aaatcgccaa ccaatttaac aaggcgatta gtcaaattca agaatcactt 2760
acaacaacat caactgcatt gggcaagctg caagacgttg ttaaccagaa tgctcaagca 2820
ttaaacacac ttgttaaaca acttagctct aattttggtg caatttcaag tgtgctaaat 2880
gatatccttt cgcgacttga taaagtcgag gcggaggtac aaattgacag gttaattaca 2940
ggcagacttc aaagccttca aacctatgta acacaacaac taatcagggc tgctgaaatc 3000
agggcttctg ctaatcttgc tgctactaaa atgtctgagt gtgttcttgg acaatcaaaa 3060
agagttgact tttgtggaaa gggctaccac cttatgtcct tcccacaagc agccccgcat 3120
ggtgttgtct tcctacatgt cacgtatgtg ccatcccagg agaggaactt caccacagcg 3180
ccagcaattt gtcatgaagg caaagcatac ttccctcgtg aaggtgtttt tgtgtttaat 3240

ggcacttctt ggtttattac acagaggaac ttcttttctc cacaaataat tactacagac 3300

aatacatttg tctcaggaaa ttgtgatgtc gttattggca tcattaacaa cacagtttat 3360

gatcctctgc aacctgagct cgactcattc aaagaagagc tggacaagta cttcaaaaat 3420

catacatcac cagatgttga tcttggcgac atttcaggca ttaacgcttc tgtcgtcaac 3480

attcaaaaag aaattgaccg cctcaatgag gtcgctaaaa atttaaatga atcactcatt 3540

gaccttcaag aattgggaaa atatgagcaa tatattaaat ggccttggta tgtttggctc 3600

ggcttcattg ctggactaat tgccatcgtc atggttacaa tcttgctttg ttgcatgact 3660

agttgttgca gttgcctcaa gggtgcatgc tcttgtggtt cttgctgcaa gtttgatgag 3720

gatgactctg agccagttct caagggtgtc aaattacatt acacataa 3768

<210> 5
<211> 666
<212> DNA
<213> SARS-CoV

<400> 5
atggcagaca acggtactat taccgttgag gagcttaaac aactcctgga acaatggaac 60

ctagtaatag gtttcctatt cctagcctgg attatgttac tacaatttgc ctattctaat 120

cggaacaggt ttttgtacat aataaagctt gttttcctct ggctcttgtg gccagtaaca 180

cttgcttgtt ttgtgcttgc tgctgtctac agaattaatt gggtgactgg cgggattgcg 240

attgcaatgg cttgtattgt aggcttgatg tggcttagct acttcgttgc ttccttcagg 300

ctgtttgctc gtacccgctc aatgtggtca ttcaacccag aaacaaacat tcttctcaat 360

gtgcctctcc gggggacaat tgtgaccaga ccgctcatgg aaagtgaact tgtcattggt 420

gctgtgatca ttcgtggtca cttgcgaatg gccggacacc ccctagggcg ctgtgacatt 480

aaggacctgc caaaagagat cactgtggct acatcacgaa cgctttctta ttacaaatta 540

ggagcgtcgc agcgtgtagg cactgattca ggttttgctg catacaaccg ctaccgtatt 600

ggaaactata aattaaatac agaccacgcc ggtagcaacg acaatattgc tttgctagta 660

cagtaa 666

<210> 6
<211> 231
<212> DNA
<213> SARS-CoV

<400> 6
atgtactcat tcgtttcgga agaaacaggt acgttaatag ttaatagcgt acttcttttt 60

cttgctttcg tggtattctt gctagtcaca ctagccatcc ttactgcgct tcgattgtgt 120

gcgtactgct gcaatattgt taacgtgagt ttagtaaaac caacggttta cgtctactcg 180

cgtgttaaaa atctgaactc ttctgaagga gttcctgatc ttctggtcta a 231

<210> 7
<211> 22
<212> DNA
<213> Artificial Sequence
<220>
<223> oligonucleotide primer

<400> 7
tgcttccctc tgcgtagaag cc 22

<210> 8
<211> 22
<212> DNA
<213> Artificial Sequence
<220>
<223> oligonucleotide primer

<400> 8
gccaaccaac ctcgatctct tg 22

<210> 9
<211> 21
<212> DNA
<213> Artificial Sequence
<220>
<223> oligonucleotide primer

<400> 9
tgggtccacc aaatgtaatg c 21

<210> 10
<211> 21
<212> DNA
<213> Artificial Sequence
<220>
<223> oligonucleotide primer

<400> 10
aagccaacca acctcgatct c 21

<210> 11
<211> 37971
<212> DNA
<213> SARS-CoV

<400> 11
   atattaggtt tttacctacc caggaaaagc caaccaacct cgatctcttg tagatctgtt 60
   ctctaaacga actttaaaat ctgtgtagct gtcgctcggc tgcatgccta gtgcacctac 120
   gcagtataaa caataataaa ttttactgtc gttgacaaga aacgagtaac tcgtccctct 180
   tctgcagact gcttacggtt tcgtccgtgt tgcagtcgat catcagcata cctaggtttc 240
   gtccgggtgt gaccgaaagg taagatggag agccttgttc ttggtgtcaa cgagaaaaca 300
   cacgtccaac tcagtttgcc tgtccttcag gttagagacg tgctagtgcg tggcttcggg 360
   gactctgtgg aagaggccct atcggaggca cgtgaacacc tcaaaaatgg cacttgtggt 420
   ctagtagagc tggaaaaagg cgtactgccc cagcttgaac agccctatgt gttcattaaa 480
   cgttctgatg ccttaagcac caatcacggc cacaaggtcg ttgagctggt tgcagaaatg 540
   gacggcattc agtacggtcg tagcggtata acactgggag tactcgtgcc acatgtgggc 600
   gaaaccccaa ttgcataccg caatgttctt cttcgtaaga acggtaataa gggagccggt 660
   ggtcatagct atggcatcga tctaaagtct tatgacttag gtgacgagct tggcactgat 720
   cccattgaag attatgaaca aaactggaac actaagcatg gcagtggtgc actccgtgaa 780
   ctcactcgtg agctcaatgg aggtgcagtc actcgctatg tcgacaacaa tttctgtggc 840
   ccagatgggt accctcttga ttgcatcaaa gattttctcg cacgcgcggg caagtcaatg 900
   tgcactcttt ccgaacaact tgattacatc gagtcgaaga gaggtgtcta ctgctgccgt 960
   gaccatgagc atgaaattgc ctggttcact gagcgctctg ataagagcta cgagcaccag 1020
   acacccttcg aaattaagag tgccaagaaa tttgacactt tcaaagggga atgcccaaag 1080
   tttgtgtttc ctcttaactc aaaagtcaaa gtcattcaac cacgtgttga aaagaaaaag 1140
   actgagggtt tcatggggcg tatacgctct gtgtaccctg ttgcatctcc acaggagtgt 1200
   aacaatatgc acttgtctac cttgatgaaa tgtaatcatt gcgatgaagt ttcatggcag 1260
   acgtgcgact ttctgaaagc cacttgtgaa cattgtggca ctgaaaattt agttattgaa 1320
   ggacctacta catgtgggta cctacctact aatgctgtag tgaaaatgcc atgtcctgcc 1380
   tgtcaagacc cagagattgg acctgagcat agtgttgcag attatcacaa ccactcaaac 1440
   attgaaactc gactccgcaa gggaggtagg actagatgtt ttggaggctg tgtgtttgcc 1500
   tatgttggct gctataataa gcgtgcctac tgggttcctc gtgctagtgc tgatattggc 1560
   tcaggccata ctggcattac tggtgacaat gtggagacct tgaatgagga tctccttgag 1620
   atactgagtc gtgaacgtgt taacattaac attgttggcg attttcattt gaatgaagag 1680
   gttgccatca ttttggcatc tttctctgct tctacaagtg cctttattga cactataaag 1740
   agtcttgatt acaagtcttt caaaaccatt gttgagtcct gcggtaacta taaagttacc 1800
   aagggaaagc ccgtaaaagg tgcttggaac attggacaac agagatcagt tttaacacca 1860
   ctgtgtggtt ttccctcaca ggctgctggt gttatcagat caatttttgc gcgcacactt 1920
   gatgcagcaa accactcaat tcctgatttg caaagagcag ctgtcaccat acttgatggt 1980
   atttctgaac agtcattacg tcttgtcgac gccatggttt atacttcaga cctgctcacc 2040
   aacagtgtca ttattatggc atatgtaact ggtggtcttg tacaacagac ttctcagtgg 2100
   ttgtctaatc ttttgggcac tactgttgaa aaactcaggc ctatctttga atggattgag 2160
   gcgaaactta gtgcaggagt tgaatttctc aaggatgctt gggagattct caaatttctc 2220
   attacaggtg tttttgacat cgtcaagggt caaatacagg ttgcttcaga taacatcaag 2280
   gattgtgtaa aatgcttcat tgatgttgtt aacaaggcac tcgaaatgtg cattgatcaa 2340
   gtcactatcg ctggcgcaaa gttgcgatca ctcaacttag gtgaagtctt catcgctcaa 2400
   agcaagggac tttaccgtca gtgtatacgt ggcaaggagc agctgcaact actcatgcct 2460
   cttaaggcac caaaagaagt aacctttctt gaaggtgatt cacatgacac agtacttacc 2520
   tctgaggagg ttgttctcaa gaacggtgaa ctcgaagcac tcgagacgcc cgttgatagc 2580
   ttcacaaatg gagctatcgt tggcacacca gtctgtgtaa atggcctcat gctcttagag 2640
   attaaggaca aagaacaata ctgcgcattg tctcctggtt tactggctac aaacaatgtc 2700
   tttcgcttaa aagggggtgc accaattaaa ggtgtaacct ttggagaaga tactgtttgg 2760
   gaagttcaag gttacaagaa tgtgagaatc acatttgagc ttgatgaacg tgttgacaaa 2820
   gtgcttaatg aaaagtgctc tgtctacact gttgaatccg gtaccgaagt tactgagttt 2880
   gcatgtgttg tagcagaggc tgttgtgaag actttacaac cagtttctga tctccttacc 2940
   aacatgggta ttgatcttga tgagtggagt gtagctacat tctacttatt tgatgatgct 3000
   ggtgaagaaa acttttcatc acgtatgtat tgttcctttt accctccaga tgaggaagaa 3060
   gaggacgatg cagagtgtga ggaagaagaa attgatgaaa cctgtgaaca tgagtacggt 3120
   acagaggatg attatcaagg tctccctctg gaatttggtg cctcagctga aacagttcga 3180
   gttgaggaag aagaagagga agactggctg gatgatacta ctgagcaatc agagattgag 3240
   ccagaaccag aacctacacc tgaagaacca gttaatcagt ttactggtta tttaaaactt 3300
   actgacaatg ttgccattaa atgtgttgac atcgttaagg aggcacaaag tgctaatcct 3360
   atggtgattg taaatgctgc taacatacac ctgaaacatg gtggtggtgt agcaggtgca 3420
   ctcaacaagg caaccaatgg tgccatgcaa aaggagagtg atgattacat taagctaaat 3480
   ggccctctta cagtaggagg gtcttgtttg ctttctggac ataatcttgc taagaagtgt 3540
   ctgcatgttg ttggacctaa cctaaatgca ggtgaggaca tccagcttct taaggcagca 3600
   tatgaaaatt tcaattcaca ggacatctta cttgcaccat tgttgtcagc aggcatattt 3660
   ggtgctaaac cacttcagtc tttacaagtg tgcgtgcaga cggttcgtac acaggtttat 3720
   attgcagtca atgacaaagc tctttatgag caggttgtca tggattatct tgataacctg 3780
   aagcctagag tggaagcacc taaacaagag gagccaccaa acacagaaga ttccaaaact 3840
   gaggagaaat ctgtcgtaca gaagcctgtc gatgtgaagc caaaaattaa ggcctgcatt 3900
   gatgaggtta ccacaacact ggaagaaact aagtttctta ccaataagtt actcttgttt 3960
   gctgatatca atggtaagct ttaccatgat tctcagaaca tgcttagagg tgaagatatg 4020
   tctttccttg agaaggatgc accttacatg gtaggtgatg ttatcactag tggtgatatc 4080
   acttgtgttg taataccctc caaaaaggct ggtggcacta ctgagatgct ctcaagagct 4140
   ttgaagaaag tgccagttga tgagtatata accacgtacc ctggacaagg atgtgctggt 4200
   tatacacttg aggaagctaa gactgctctt aagaaatgca aatctgcatt ttatgtacta 4260
   ccttcagaag cacctaatgc taaggaagag attctaggaa ctgtatcctg gaatttgaga 4320
   gaaatgcttg ctcatgctga agagacaaga aaattaatgc ctatatgcat ggatgttaga 4380
   gccataatgg caaccatcca acgtaagtat aaaggaatta aaattcaaga gggcatcgtt 4440
   gactatggtg tccgattctt cttttatact agtaaagagc ctgtagcttc tattattacg 4500
   aagctgaact ctctaaatga gccgcttgtc acaatgccaa ttggttatgt gacacatggt 4560
   tttaatcttg aagaggctgc gcgctgtatg cgttctctta aagctcctgc cgtagtgtca 4620
   gtatcatcac cagatgctgt tactacatat aatggatacc tcacttcgtc atcaaagaca 4680
   tctgaggagc actttgtaga aacagtttct ttggctggct cttacagaga ttggtcctat 4740
   tcaggacagc gtacagagtt aggtgttgaa tttcttaagc gtggtgacaa aattgtgtac 4800
   cacactctgg agagccccgt cgagtttcat cttgacggtg aggttctttc acttgacaaa 4860
   ctaaagagtc tcttatccct gcgggaggtt aagactataa aagtgttcac aactgtggac 4920
   aacactaatc tccacacaca gcttgtggat atgtctatga catatggaca gcagtttggt 4980
   ccaacatact tggatggtgc tgatgttaca aaaattaaac ctcatgtaaa tcatgagggt 5040
   aagactttct ttgtactacc tagtgatgac acactacgta gtgaagcttt cgagtactac 5100
   catactcttg atgagagttt tcttggtagg tacatgtctg ctttaaacca cacaaagaaa 5160
   tggaaatttc ctcaagttgg tggtttaact tcaattaaat gggctgataa caattgttat 5220
   ttgtctagtg ttttattagc acttcaacag cttgaagtca aattcaatgc accagcactt 5280
   caagaggctt attatagagc ccgtgctggt gatgctgcta acttttgtgc actcatactc 5340
   gcttacagta ataaaactgt tggcgagctt ggtgatgtca gagaaactat gacccatctt 5400
   ctacagcatg ctaatttgga atctgcaaag cgagttctta atgtggtgtg taaacattgt 5460
   ggtcagaaaa ctactacctt aacgggtgta gaagctgtga tgtatatggg tactctatct 5520
   tatgataatc ttaagacagg tgtttccatt ccatgtgtgt gtggtcgtga tgctacacaa 5580
   tatctagtac aacaagagtc ttcttttgtt atgatgtctg caccacctgc tgagtataaa 5640
   ttacagcaag gtacattctt atgtgcgaat gagtacactg gtaactatca gtgtggtcat 5700
   tacactcata taactgctaa ggagaccctc tatcgtattg acggagctca ccttacaaag 5760
   atgtcagagt acaaaggacc agtgactgat gttttctaca aggaaacatc ttacactaca 5820
   accatcaagc ctgtgtcgta taaactcgat ggagttactt acacagagat tgaaccaaaa 5880
   ttggatgggt attataaaaa ggataatgct tactatacag agcagcctat agaccttgta 5940
   ccaactcaac cattaccaaa tgcgagtttt gataatttca aactcacatg ttctaacaca 6000
   aaatttgctg atgatttaaa tcaaatgaca ggcttcacaa agccagcttc acgagagcta 6060
   tctgtcacat tcttcccaga cttgaatggc gatgtagtgg ctattgacta tagacactat 6120
   tcagcgagtt tcaagaaagg tgctaaatta ctgcataagc caattgtttg gcacattaac 6180
   caggctacaa ccaagacaac gttcaaacca aacacttggt gtttacgttg tctttggagt 6240
   acaaagccag tagatacttc aaattcattt gaagttctgg cagtagaaga cacacaagga 6300
   atggacaatc ttgcttgtga aagtcaacaa cccacctctg aagaagtagt ggaaaatcct 6360
   accatacaga aggaagtcat agagtgtgac gtgaaaacta ccgaagttgt aggcaatgtc 6420
   atacttaaac catcagatga aggtgttaaa gtaacacaag agttaggtca tgaggatctt 6480
   atggctgctt atgtggaaaa cacaagcatt accattaaga aacctaatga gctttcacta 6540
   gccttaggtt taaaaacaat tgccactcat ggtattgctg caattaatag tgttccttgg 6600
   agtaaaattt tggcttatgt caaaccattc ttaggacaag cagcaattac aacatcaaat 6660
   tgcgctaaga gattagcaca acgtgtgttt aacaattata tgccttatgt gtttacatta 6720
   ttgttccaat tgtgtacttt tactaaaagt accaattcta gaattagagc ttcactacct 6780
   acaactattg ctaaaaatag tgttaagagt gttgctaaat tatgtttgga tgccggcatt 6840
   aattatgtga agtcacccaa attttctaaa ttgttcacaa tcgctatgtg gctattgttg 6900
   ttaagtattt gcttaggttc tctaatctgt gtaactgctg cttttggtgt actcttatct 6960
   aattttggtg ctccttctta ttgtaatggc gttagagaat tgtatcttaa ttcgtctaac 7020
   gttactacta tggatttctg tgaaggttct tttccttgca gcatttgttt aagtggatta 7080
   gactcccttg attcttatcc agctcttgaa accattcagg tgacgatttc atcgtacaag 7140
   ctagacttga caattttagg tctggccgct gagtgggttt tggcatatat gttgttcaca 7200
   aaattctttt atttattagg tctttcagct ataatgcagg tgttctttgg ctattttgct 7260
   agtcatttca tcagcaattc ttggctcatg tggtttatca ttagtattgt acaaatggca 7320
   cccgtttctg caatggttag gatgtacatc ttctttgctt ctttctacta catatggaag 7380
   agctatgttc atatcatgga tggttgcacc tcttcgactt gcatgatgtg ctataagcgc 7440
   aatcgtgcca cacgcgttga gtgtacaact attgttaatg gcatgaagag atctttctat 7500
   gtctatgcaa atggaggccg tggcttctgc aagactcaca attggaattg tctcaattgt 7560
   gacacatttt gcactggtag tacattcatt agtgatgaag ttgctcgtga tttgtcactc 7620
   cagtttaaaa gaccaatcaa ccctactgac cagtcatcgt atattgttga tagtgttgct 7680
   gtgaaaaatg gcgcgcttca cctctacttt gacaaggctg gtcaaaagac ctatgagaga 7740
   catccgctct cccattttgt caatttagac aatttgagag ctaacaacac taaaggttca 7800
   ctgcctatta atgtcatagt ttttgatggc aagtccaaat gcgacgagtc tgcttctaag 7860
   tctgcttctg tgtactacag tcagctgatg tgccaaccta ttctgttgct tgaccaagtt 7920
   cttgtatcag acgttggaga tagtactgaa gtttccgtta agatgtttga tgcttatgtc 7980
   gacacctttt cagcaacttt tagtgttcct atggaaaaac ttaaggcact tgttgctaca 8040
   gctcacagcg agttagcaaa gggtgtagct ttagatggtg tcctttctac attcgtgtca 8100
   gctgcccgac aaggtgttgt tgataccgat gttgacacaa aggatgttat tgaatgtctc 8160
   aaactttcac atcactctga cttagaagtg acaggtgaca gttgtaacaa tttcatgctc 8220
   acctataata aggttgaaaa catgacgccc agagatcttg gcgcatgtat tgactgtaat 8280
   gcaaggcata tcaatgccca agtagcaaaa agtcacaatg tttcactcat ctggaatgta 8340
   aaagactaca tgtctttatc tgaacagctg cgtaaacaaa ttcgtagtgc tgccaagaag 8400
   aacaacatac cttttagact aacttgtgct acaactagac aggttgtcaa tgtcataact 8460
   actaaaatct cactcaaggg tggtaagatt gttagtactt gttttaaact tatgcttaag 8520
   gccacattat tgtgcgttct tgctgcattg gtttgttata tcgttatgcc agtacataca 8580
   ttgtcaatcc atgatggtta cacaaatgaa atcattggtt acaaagccat tcaggatggt 8640
   gtcactcgtg acatcatttc tactgatgat tgttttgcaa ataaacatgc tggttttgac 8700
   gcatggttta gccagcgtgg tggttcatac aaaaatgaca aaagctgccc tgtagtagct 8760
   gctatcatta caagagagat tggtttcata gtgcctggct taccgggtac tgtgctgaga 8820
   gcaatcaatg gtgacttctt gcattttcta cctcgtgttt ttagtgctgt tggcaacatt 8880
   tgctacacac cttccaaact cattgagtat agtgattttg ctacctctgc ttgcgttctt 8940
   gctgctgagt gtacaatttt taaggatgct atgggcaaac ctgtgccata ttgttatgac 9000
   actaatttgc tagagggttc tatttcttat agtgagcttc gtccagacac tcgttatgtg 9060
   cttatggatg gttccatcat acagtttcct aacacttacc tggagggttc tgttagagta 9120
   gtaacaactt ttgatgctga gtactgtaga catggtacat gcgaaaggtc agaagtaggt 9180
   atttgcctat ctaccagtgg tagatgggtt cttaataatg agcattacag agctctatca 9240
   ggagttttct gtggtgttga tgcgatgaat ctcatagcta acatctttac tcctcttgtg 9300
   caacctgtgg gtgctttaga tgtgtctgct tcagtagtgg ctggtggtat tattgccata 9360
   ttggtgactt gtgctgccta ctactttatg aaattcagac gtgtttttgg tgagtacaac 9420
   catgttgttg ctgctaatgc acttttgttt ttgatgtctt tcactatact ctgtctggta 9480
   ccagcttaca gctttctgcc gggagtctac tcagtctttt acttgtactt gacattctat 9540
   ttcaccaatg atgtttcatt cttggctcac cttcaatggt ttgccatgtt ttctcctatt 9600
   gtgccttttt ggataacagc aatctatgta ttctgtattt ctctgaagca ctgccattgg 9660
   ttctttaaca actatcttag gaaaagagtc atgtttaatg gagttacatt tagtaccttc 9720
   gaggaggctg ctttgtgtac ctttttgctc aacaaggaaa tgtacctaaa attgcgtagc 9780
   gagacactgt tgccacttac acagtataac aggtatcttg ctctatataa caagtacaag 9840
   tatttcagtg gagccttaga tactaccagc tatcgtgaag cagcttgctg ccacttagca 9900
   aaggctctaa atgactttag caactcaggt gctgatgttc tctaccaacc accacagaca 9960
   tcaatcactt ctgctgttct gcagagtggt tttaggaaaa tggcattccc gtcaggcaaa 10020
   gttgaagggt gcatggtaca agtaacctgt ggaactacaa ctcttaatgg attgtggttg 10080
   gatgacacag tatactgtcc aagacatgtc atttgcacag cagaagacat gcttaatcct 10140
   aactatgaag atctgctcat tcgcaaatcc aaccatagct ttcttgttca ggctggcaat 10200
   gttcaacttc gtgttattgg ccattctatg caaaattgtc tgcttaggct taaagttgat 10260
   acttctaacc ctaagacacc caagtataaa tttgtccgta tccaacctgg tcaaacattt 10320
   tcagttctag catgctataa tggttcacca tctggtgttt atcagtgtgc catgagacct 10380
   aatcatacca ttaaaggttc tttccttaat ggatcatgtg gtagtgttgg ttttaacatt 10440
   gattatgatt gcgtgtcttt ctgctatatg catcatatgg agcttccaac aggagtacac 10500
   gctggtactg acttagaagg taaattctat ggtccatttg ttgacagaca aactgcacag 10560
   gctgcaggta cagacacaac cataacatta aatgttttgg catggctgta tgctgctgtt 10620
   atcaatggtg ataggtggtt tcttaataga ttcaccacta ctttgaatga ctttaacctt 10680
   gtggcaatga agtacaacta tgaacctttg acacaagatc atgttgacat attgggacct 10740
   ctttctgctc aaacaggaat tgccgtctta gatatgtgtg ctgctttgaa agagctgctg 10800
   cagaatggta tgaatggtcg tactatcctt ggtagcacta ttttagaaga tgagtttaca 10860
   ccatttgatg ttgttagaca atgctctggt gttaccttcc aaggtaagtt caagaaaatt 10920
   gttaagggca ctcatcattg gatgctttta actttcttga catcactatt gattcttgtt 10980
   caaagtacac agtggtcact gtttttcttt gtttacgaga atgctttctt gccatttact 11040
   cttggtatta tggcaattgc tgcatgtgct atgctgcttg ttaagcataa gcacgcattc 11100
   ttgtgcttgt ttctgttacc ttctcttgca acagttgctt actttaatat ggtctacatg 11160
   ccagctagct gggtgatgcg tatcatgaca tggcttgaat tggctgacac tagcttgtct 11220
   ggttataggc ttaaggattg tgttatgtat gcttcagctt tagttttgct tattctcatg 11280
   acagctcgca ctgtttatga tgatgctgct agacgtgttt ggacactgat gaatgtcatt 11340
   acacttgttt acaaagtcta ctatggtaat gctttagatc aagctatttc catgtgggcc 11400
   ttagttattt ctgtaacctc taactattct ggtgtcgtta cgactatcat gtttttagct 11460
   agagctatag tgtttgtgtg tgttgagtat tacccattgt tatttattac tggcaacacc 11520
   ttacagtgta tcatgcttgt ttattgtttc ttaggctatt gttgctgctg ctactttggc 11580
   cttttctgtt tactcaaccg ttacttcagg cttactcttg gtgtttatga ctacttggtc 11640
   tctacacaag aatttaggta tatgaactcc caggggcttt tgcctcctaa gagtagtatt 11700
   gatgctttca agcttaacat taagttgttg ggtattggag gtaaaccatg tatcaaggtt 11760
   gctactgtac agtctaaaat gtctgacgta aagtgcacat ctgtggtact gctctcggtt 11820
   cttcaacaac ttagagtaga gtcatcttct aaattgtggg cacaatgtgt acaactccac 11880
   aatgatattc ttcttgcaaa agacacaact gaagctttcg agaagatggt ttctcttttg 11940
   tctgttttgc tatccatgca gggtgctgta gacattaata ggttgtgcga ggaaatgctc 12000
   gataaccgtg ctactcttca ggctattgct tcagaattta gttctttacc atcatatgcc 12060
   gcttatgcca ctgcccagga ggcctatgag caggctgtag ctaatggtga ttctgaagtc 12120
   gttctcaaaa agttaaagaa atctttgaat gtggctaaat ctgagtttga ccgtgatgct 12180
   gccatgcaac gcaagttgga aaagatggca gatcaggcta tgacccaaat gtacaaacag 12240
   gcaagatctg aggacaagag ggcaaaagta actagtgcta tgcaaacaat gctcttcact 12300
   atgcttagga agcttgataa tgatgcactt aacaacatta tcaacaatgc gcgtgatggt 12360
   tgtgttccac tcaacatcat accattgact acagcagcca aactcatggt tgttgtccct 12420
   gattatggta cctacaagaa cacttgtgat ggtaacacct ttacatatgc atctgcactc 12480
   tgggaaatcc agcaagttgt tgatgcggat agcaagattg ttcaacttag tgaaattaac 12540
   atggacaatt caccaaattt ggcttggcct cttattgtta cagctctaag agccaactca 12600
   gctgttaaac tacagaataa tgaactgagt ccagtagcac tacgacagat gtcctgtgcg 12660
   gctggtacca cacaaacagc ttgtactgat gacaatgcac ttgcctacta taacaattcg 12720
   aagggaggta ggtttgtgct ggcattacta tcagaccacc aagatctcaa atgggctaga 12780
   ttccctaaga gtgatggtac aggtacaatt tacacagaac tggaaccacc ttgtaggttt 12840
   gttacagaca caccaaaagg gcctaaagtg aaatacttgt acttcatcaa aggcttaaac 12900
   aacctaaata gaggtatggt gctgggcagt ttagctgcta cagtacgtct tcaggctgga 12960
   aatgctacag aagtacctgc caattcaact gtgctttcct tctgtgcttt tgcagtagac 13020
   cctgctaaag catataagga ttacctagca agtggaggac aaccaatcac caactgtgtg 13080
   aagatgttgt gtacacacac tggtacagga caggcaatta ctgtaacacc agaagctaac 13140
   atggaccaag agtcctttgg tggtgcttca tgttgtctgt attgtagatg ccacattgac 13200
   catccaaatc ctaaaggatt ctgtgacttg aaaggtaagt acgtccaaat acctaccact 13260
   tgtgctaatg acccagtggg ttttacactt agaaacacag tctgtaccgt ctgcggaatg 13320
   tggaaaggtt atggctgtag ttgtgaccaa ctccgcgaac ccttgatgca gtctgcggat 13380
   gcatcaacgt ttttaaacgg gtttgcggtg taagtgcagc ccgtcttaca ccgtgcggca 13440
   caggcactag tactgatgtc gtctacaggg cttttgatat ttacaacgaa aaagttgctg 13500
   gttttgcaaa gttcctaaaa actaattgct gtcgcttcca ggagaaggat gaggaaggca 13560
   atttattaga ctcttacttt gtagttaaga ggcatactat gtctaactac caacatgaag 13620
   agactattta taacttggtt aaagattgtc cagcggttgc tgtccatgac tttttcaagt 13680
   ttagagtaga tggtgacatg gtaccacata tatcacgtca gcgtctaact aaatacacaa 13740
   tggctgattt agtctatgct ctacgtcatt ttgatgaggg taattgtgat acattaaaag 13800
   aaatactcgt cacatacaat tgctgtgatg atgattattt caataagaag gattggtatg 13860
   acttcgtaga gaatcctgac atcttaacgc gtatatgcta acttaggtga gcgtgtacgc 13920
   caatcattat taaagactgt acaattctgc gatgctatgc gtgatgcagg cattgtaggc 13980
   gtactgacat tagataatca ggatcttaat gggaactggt acgatttcgg tgatttcgta 14040
   caagtagcac caggctgcgg agttcctatt gtggattcat attactcatt gctgatgccc 14100
   atcctcactt tgactagggc attggctgct gagtcccata tggatgctga tctcgcaaaa 14160
   ccacttatta agtgggattt gctgaaatat gattttacgg aagagagact ttgtctcttc 14220
   gaccgttatt ttaaatattg ggaccagaca taccatccca attgtattaa ctgtttggat 14280
   gataggtgta tccttcattg tgcaaacttt aatgtgttat tttctactgt gtttccacct 14340
   acaagttttg gaccactagt aagaaaaata tttgtagatg gtgttccttt tgttgtttca 14400
   actggatacc attttcgtga gttaggagtc gtacataatc aggatgtaaa cttacatagc 14460
   tcgcgtctca gtttcaagga acttttagtg tatgctgctg atccagctat gcatgcagct 14520
   tctggcaatt tattgctaga taaacgcact acatgctttt cagtagctgc actaacaaac 14580
   aatgttgctt ttcaaactgt caaacccggt aattttaata aagactttta tgactttgct 14640
   gtgtctaaag gtttctttaa ggaaggaagt tctgttgaac taaaacactt cttctttgct 14700
   caggatggca acgctgctat cagtgattat gactattatc gttataatct gccaacaatg 14760
   tgtgatatca gacaactcct attcgtagtt gaagttgttg ataaatactt tgattgttac 14820
   gatggtggct gtattaatgc caaccaagta atcgttaaca atctggataa atcagctggt 14880
   ttcccattta ataaatgggg taaggctaga ctttattatg actcaatgag ttatgaggat 14940
   caagatgcac ttttcgcgta tactaagcgt aatgtcatcc ctactataac tcaaatgaat 15000
   cttaagtatg ccattagtgc aaagaataga gctcgcaccg tagctggtgt ctctatctgt 15060
   agtactatga caaatagaca gtttcatcag aaattattga agtcaatagc cgccactaga 15120
   ggagctactg tggtaattgg aacaagcaag ttttacggtg gctggcataa tatgttaaaa 15180
   actgtttaca gtgatgtaga aactccacac cttatgggtt gggattatcc aaaatgtgac 15240
   agagccatgc ctaacatgct taggataatg gcctctcttg ttcttgctcg caaacataac 15300
   acttgctgta acttatcaca ccgtttctac aggttagcta acgagtgtgc gcaagtatta 15360
   agtgagatgg tcatgtgtgg cggctcacta tatgttaaac caggtggaac atcatccggt 15420
   gatgctacaa ctgcttatgc taatagtgtc tttaacattt gtcaagctgt tacagccaat 15480
   gtaaatgcac ttctttcaac tgatggtaat aagatagctg acaagtatgt ccgcaatcta 15540
   caacacaggc tctatgagtg tctctataga aatagggatg ttgatcatga attcgtggat 15600
   gagttttacg cttacctgcg taaacatttc tccatgatga ttctttctga tgatgccgtt 15660
   gtgtgctata acagtaacta tgcggctcaa ggtttagtag ctagcattaa gaactttaag 15720
   gcagttcttt attatcaaaa taatgtgttc atgtctgagg caaaatgttg gactgagact 15780
   gaccttacta aaggacctca cgaattttgc tcacagcata caatgctagt taaacaagga 15840
   gatgattacg tgtacctgcc ttacccagat ccatcaagaa tattaggcgc aggctgtttt 15900
   gtcgatgata ttgtcaaaac agatggtaca cttatgattg aaaggttcgt gtcactggct 15960
   attgatgctt acccacttac aaaacatcct aatcaggagt atgctgatgt ctttcacttg 16020
   tatttacaat acattagaaa gttacatgat gagcttactg gccacatgtt ggacatgtat 16080
   tccgtaatgc taactaatga taacacctca cggtactggg aacctgagtt ttatgaggct 16140
   atgtacacac cacatacagt cttgcaggct gtaggtgctt gtgtattgtg caattcacag 16200
   acttcacttc gttgcggtgc ctgtattagg agaccattcc tatgttgcaa gtgctgctat 16260
   gaccatgtca tttcaacatc acacaaatta gtgttgtctg ttaatcccta tgtttgcaat 16320
   gccccaggtt gtgatgtcac tgatgtgaca caactgtatc taggaggtat gagctattat 16380
   tgcaagtcac ataagcctcc cattagtttt ccattatgtg ctaatggtca ggtttttggt 16440
   ttatacaaaa acacatgtgt aggcagtgac aatgtcactg acttcaatgc gatagcaaca 16500
   tgtgattgga ctaatgctgg cgattacata cttgccaaca cttgtactga gagactcaag 16560
   cttttcgcag cagaaacgct caaagccact gaggaaacat ttaagctgtc atatggtatt 16620
   gctactgtac gcgaagtact ctctgacaga gaattgcatc tttcatggga ggttggaaaa 16680
   cctagaccac cattgaacag aaactatgtc tttactggtt accgtgtaac taaaaatagt 16740
   aaagtacaga ttggagagta cacctttgaa aaaggtgact atggtgatgc tgttgtgtac 16800
   agaggtacta cgacatacaa gttgaatgtt ggtgattact ttgtgttgac atctcacact 16860
   gtaatgccac ttagtgcacc tactctagtg ccacaagagc actatgtgag aattactggc 16920
   ttgtacccaa cactcaacat ctcagatgag ttttctagca atgttgcaaa ttatcaaaag 16980
   gtcggcatgc aaaagtactc tacactccaa ggaccacctg gtactggtaa gagtcatttt 17040
   gccatcggac ttgctctcta ttacccatct gctcgcatag tgtatacggc atgctctcat 17100
   gcagctgttg atgccctatg tgaaaaggca ttaaaatatt tgcccataga taaatgtagt 17160
   agaatcatac ctgcgcgtgc gcgcgtagag tgttttgata aattcaaagt gaattcaaca 17220
   ctagaacagt atgttttctg cactgtaaat gcattgccag aaacaactgc tgacattgta 17280
   gtctttgatg aaatctctat ggctactaat tatgacttga gtgttgtcaa tgctagactt 17340
   cgtgcaaaac actacgtcta tattggcgat cctgctcaat taccagcccc ccgcacattg 17400
   ctgactaaag gcacactaga accagaatat tttaattcag tgtgcagact tatgaaaaca 17460
   ataggtccag acatgttcct tggaacttgt cgccgttgtc ctgctgaaat tgttgacact 17520
   gtgagtgctt tagtttatga caataagcta aaagcacaca aggataagtc agctcaatgc 17580
   ttcaaaatgt tctacaaagg tgttattaca catgatgttt catctgcaat caacagacct 17640
   caaataggcg ttgtaagaga atttcttaca cgcaatcctg cttggagaaa agctgttttt 17700
   atctcacctt ataattcaca gaacgctgta gcttcaaaaa tcttaggatt gcctacgcag 17760
   actgttgatt catcacaggg ttctgaatat gactatgtca tattcacaca aactactgaa 17820
   acagcacact cttgtaatgt caaccgcttc aatgtggcta tcacaagggc aaaaattggc 17880
   attttgtgca taatgtctga tagagatctt tatgacaaac tgcaatttac aagtctagaa 17940
   ataccacgtc gcaatgtggc tacattacaa gcagaaaatg taactggact ttttaaggac 18000
   tgtagtaaga tcattactgg tcttcatcct acacaggcac ctacacacct cagcgttgat 18060
   ataaagttca agactgaagg attatgtgtt gacataccag gcataccaaa ggacatgacc 18120
   taccgtagac tcatctctat gatgggtttc aaaatgaatt accaagtcaa tggttaccct 18180
   aatatgttta tcacccgcga agaagctatt cgtcacgttc gtgcgtggat tggctttgat 18240
   gtagagggct gtcatgcaac tagagatgct gtgggtacta acctacctct ccagctagga 18300
   ttttctacag gtgttaactt agtagctgta ccgactggtt atgttgacac tgaaaataac 18360
   acagaattca ccagagttaa tgcaaaacct ccaccaggtg accagtttaa acatcttata 18420
   ccactcatgt ataaaggctt gccctggaat gtagtgcgta ttaagatagt acaaatgctc 18480
   agtgatacac tgaaaggatt gtcagacaga gtcgtgttcg tcctttgggc gcatggcttt 18540
   gagcttacat caatgaagta ctttgtcaag attggacctg aaagaacgtg ttgtctgtgt 18600
   gacaaacgtg caacttgctt ttctacttca tcagatactt atgcctgctg gaatcattct 18660
   gtgggttttg actatgtcta taacccattt atgattgatg ttcagcagtg gggctttacg 18720
   ggtaaccttc agagtaacca tgaccaacat tgccaggtac atggaaatgc acatgtggct 18780
   agttgtgatg ctatcatgac tagatgttta gcagtccatg agtgctttgt taagcgcgtt 18840
   gattggtctg ttgaataccc tattatagga gatgaactga gggttaattc tgcttgcaga 18900
   aaagtacaac acatggttgt gaagtctgca ttgcttgctg ataagtttcc agttcttcat 18960
   gacattggaa atccaaaggc tatcaagtgt gtgcctcagg ctgaagtaga atggaagttc 19020
   tacgatgctc agccatgtag tgacaaagct tacaaaatag aggagctctt ctattcttat 19080
   gctacacatc acgataaatt cactgatggt gtttgtttgt tttggaattg taacgttgat 19140
   cgttacccag ccaatgcaat tgtgtgtagg tttgacacaa gagtcttgtc aaacttgaac 19200
   ttaccaggct gtgatggtgg tagtttgtat gtgaataagc atgcattcca cactccagct 19260
   ttcgataaaa gtgcatttac taatttaaag caattgcctt tcttttacta ttctgatagt 19320
   ccttgtgagt ctcatggcaa acaagtagtg tcggatattg attatgttcc actcaaatct 19380
   gctacgtgta ttacacgatg caatttaggt ggtgctgttt gcagacacca tgcaaatgag 19440
   taccgacagt acttggatgc atataatatg atgatttctg ctggatttag cctatggatt 19500
   tacaaacaat ttgatactta taacctgtgg aatacattta ccaggttaca gagtttagaa 19560
   aatgtggctt ataatgttgt taataaagga cactttgatg gacacgccgg cgaagcacct 19620
   gtttccatca ttaataatgc tgtttacaca aaggtagatg gtattgatgt ggagatcttt 19680
   gaaaataaga caacacttcc tgttaatgtt gcatttgagc tttgggctaa gcgtaacatt 19740
   aaaccagtgc cagagattaa gatactcaat aatttgggtg ttgatatcgc tgctaatact 19800
   gtaatctggg actacaaaag agaagcccca gcacatgtat ctacaatagg tgtctgcaca 19860
   atgactgaca ttgccaagaa acctactgag agtgcttgtt cttcacttac tgtcttgttt 19920
   gatggtagag tggaaggaca ggtagacctt tttagaaacg cccgtaatgg tgttttaata 19980
   acagaaggtt cagtcaaagg tctaacacct tcaaagggac cagcacaagc tagcgtcaat 20040
   ggagtcacat taattggaga atcagtaaaa acacagttta actactttaa gaaagtagac 20100
   ggcattattc aacagttgcc tgaaacctac tttactcaga gcagagactt agaggatttt 20160
   aagcccagat cacaaatgga aactgacttt ctcgagctcg ctatggatga attcatacag 20220
   cgatataagc tcgagggcta tgccttcgaa cacatcgttt atggagattt cagtcatgga 20280
   caacttggcg gtcttcattt aatgataggc ttagccaagc gctcacaaga ttcaccactt 20340
   aaattagagg attttatccc tatggacagc acagtgaaaa attacttcat aacagatgcg 20400
   caaacaggtt catcaaaatg tgtgtgttct gtgattgatc ttttacttga tgactttgtc 20460
   gagataataa agtcacaaga tttgtcagtg atttcaaaag tggtcaaggt tacaattgac 20520
   tatgctgaaa tttcattcat gctttggtgt aaggatggac atgttgaaac cttctaccca 20580
   aaactacaag caagtcaagc gtggcaacca ggtgttgcga tgcctaactt gtacaagatg 20640
   caaagaatgc ttcttgaaaa gtgtgacctt cagaattatg gtgaaaatgc tgttatacca 20700
   aaaggaataa tgatgaatgt cgcaaagtat actcaactgt gtcaatactt aaatacactt 20760
   actttagctg taccctacaa catgagagtt attcactttg gtgctggctc tgataaagga 20820
   gttgcaccag gtacagctgt gctcagacaa tggttgccaa ctggcacact acttgtcgat 20880
   tcagatctta atgacttcgt ctccgacgca gattctactt taattggaga ctgtgcaaca 20940
   gtacatacgg ctaataaatg ggaccttatt attagcgata tgtatgaccc taggaccaaa 21000
   catgtgacaa aagagaatga ctctaaagaa gggtttttca cttatctgtg tggatttata 21060
   aagcaaaaac tagccctggg tggttctata gctgtaaaga taacagagca ttcttggaat 21120
   gctgaccttt acaagcttat gggccatttc tcatggtgga cagcttttgt tacaaatgta 21180
   aatgcatcat catcggaagc atttttaatt ggggctaact atcttggcaa gccgaaggaa 21240
   caaattgatg gctataccat gcatgctaac tacattttct ggaggaacac aaatcctatc 21300
   cagttgtctt cctattcact ctttgacatg agcaaatttc ctcttaaatt aagaggaact 21360
   gctgtaatgt ctcttaagga gaatcaaatc aatgatatga tttattctct tctggaaaaa 21420
   ggtaggctta tcattagaga aaacaacaga gttgtggttt caagtgatat tcttgttaac 21480
   aactaaacga acatgtttat tttcttatta tttcttactc tcactagtgg tagtgacctt 21540
   gaccggtgca ccacttttga tgatgttcaa gctcctaatt acactcaaca tacttcatct 21600
   atgagggggg tttactatcc tgatgaaatt tttagatcag acactcttta tttaactcag 21660
   gatttatttc ttccatttta ttctaatgtt acagggtttc atactattaa tcatacgttt 21720
   ggcaaccctg tcatackttt taaggatggt atttattttg ctgccacaga gaaatcaaat 21780
   gttgtccgtg gttgggtttt tggttctacc atgaacaaca agtcacagtc ggtgattatt 21840
   attaacaatt ctactaatgt tgttatacga gcatgtaact ttgaattgtg tgacaaccct 21900
   ttctttgctg tttctaaacc catgggtaca cagacacata ctatgatatt cgataatgca 21960
   tttaattgca ctttcgagta catatctgat gccttttcgc ttgatgtttc agaaaagtca 22020
   ggtaatttta aacacttacg agagtttgtg tttaaaaata aagatgggtt tctctatgtt 22080
   tataagggct atcaacctat agatgtagtt cgtgatctac cttctggttt taacactttg 22140
   aaacctattt ttaagttgcc tcttggtatt aacattacaa attttagagc cattcttaca 22200
   gccttttcac ctgctcaaga catttggggc acgtcagctg cagcctattt tgttggctat 22260
   ttaaagccaa ctacatttat gctcaagtat gatgaaaatg gtacaatcac agatgctgtt 22320
   gattgttctc aaaatccact tgctgaactc aaatgctctg ttaagagctt tgagattgac 22380
   aaaggaattt accagacctc taatttcagg gttgttccct caggagatgt tgtgagattc 22440
   cctaatatta caaacttgtg tccttttgga gaggttttta atgctactaa attcccttct 22500
   gtctatgcat gggagagaaa aaaaatttct aattgtgttg ctgattactc tgtgctctac 22560
   aactcaacat ttttttcaac ctttaagtgc tatggcgttt ctgccactaa gttgaatgat 22620
   ctttgcttct ccaatgtcta tgcagattct tttgtagtca agggagatga tgtaagacaa 22680
   atagcgccag gacaaactgg tgttattgct gattataatt ataaattgcc agatgatttc 22740
   atgggttgtg tccttgcttg gaatactagg aacattgatg ctacttcaac tggtaattat 22800
   aattataaat ataggtatct tagacatggc aagcttaggc cctttgagag agacatatct 22860
   aatgtgcctt tctcccctga tggcaaacct tgcaccccac ctgctcttaa ttgttattgg 22920
   ccattaaatg attatggttt ttacaccact actggcattg gctaccaacc ttacagagtt 22980
   gtagtacttt cttttgaact tttaaatgca ccggccacgg tttgtggacc aaaattatcc 23040
   actgacctta ttaagaacca gtgtgtcaat tttaatttta atggactcac tggtactggt 23100
   gtgttaactc cttcttcaaa gagatttcaa ccatttcaac aatttggccg tgatgtttct 23160
   gatttcactg attccgttcg agatcctaaa acatctgaaa tattagacat ttcaccttgc 23220
   tcttttgggg gtgtaagtgt aattacacct ggaacaaatg cttcatctga agttgctgtt 23280
   ctatatcaag atgttaactg cactgatgtt tctacagcaa ttcatgcaga tcaactcaca 23340
   ccagcttggc gcatatattc tactggaaac aatgtattcc agactcaagc aggctgtctt 23400
   ataggagctg agcatgtcga cacttcttat gagtgcgaca ttcctattgg agctggcatt 23460
   tgtgctagtt accatacagt ttctttatta cgtagtacta gccaaaaatc tattgtggct 23520
   tatactatgt ctttaggtgc tgatagttca attgcttact ctaataacac cattgctata 23580
   cctactaact tttcaattag cattactaca gaagtaatgc ctgtttctat ggctaaaacc 23640
   tccgtagatt gtaatatgta catctgcgga gattctactg aatgtgctaa tttgcttctc 23700
   caatatggta gcttttgcac acaactaaat cgtgcactct caggtattgc tgctgaacag 23760
   gatcgcaaca cacgtgaagt gttcgctcaa gtcaaacaaa tgtacaaaac cccaactttg 23820
   aaatattttg gtggttttaa tttttcacaa atattacctg accctctaaa gccaactaag 23880
   aggtctttta ttgaggactt gctctttaat aaggtgacac tcgctgatgc tggcttcatg 23940
   aagcaatatg gcgaatgcct aggtgatatt aatgctagag atctcatttg tgcgcagaag 24000
   ttcaatggac ttacagtgtt gccacctctg ctcactgatg atatgattgc tgcctacact 24060
   gctgctctag ttagtggtac tgccactgct ggatggacat ttggtgctgg cgctgctctt 24120
   caaatacctt ttgctatgca aatggcatat aggttcaatg gcattggagt tacccaaaat 24180
   gttctctatg agaaccaaaa acaaatcgcc aaccaattta acaaggcgat tagtcaaatt 24240
   caagaatcac ttacaacaac atcaactgca ttgggcaagc tgcaagacgt tgttaaccag 24300
   aatgctcaag cattaaacac acttgttaaa caacttagct ctaattttgg tgcaatttca 24360
   agtgtgctaa atgatatcct ttcgcgactt gataaagtcg aggcggaggt acaaattgac 24420
   aggttaatta caggcagact tcaaagcctt caaacctatg taacacaaca actaatcagg 24480
   gctgctgaaa tcagggcttc tgctaatctt gctgctacta aaatgtctga gtgtgttctt 24540
   ggacaatcaa aaagagttga cttttgtgga aagggctacc accttatgtc cttcccacaa 24600
   gcagccccgc atggtgttgt cttcctacat gtcacgtatg tgccatccca ggagaggaac 24660
   ttcaccacag cgccagcaat ttgtcatgaa ggcaaagcat acttccctcg tgaaggtgtt 24720
   tttgtgttta atggcacttc ttggtttatt acacagagga acttcttttc tccacaaata 24780
   attactacag acaatacatt tgtctcagga aattgtgatg tcgttattgg catcattaac 24840
   aacacagttt atgatcctct gcaacctgag ctcgactcat tcaaagaaga gctggacaag 24900
   tacttcaaaa atcatacatc accagatgtt gatcttggcg acatttcagg cattaacgct 24960
   tctgtcgtca acattcaaaa agaaattgac cgcctcaatg aggtcgctaa aaatttaaat 25020
   gaatcactca ttgaccttca agaattggga aaatatgagc aatatattaa atggccttgg 25080
   tatgtttggc tcggcttcat tgctggacta attgccatcg tcatggttac aatcttgctt 25140
   tgttgcatga ctagttgttg cagttgcctc aagggtgcat gctcttgtgg ttcttgctgc 25200
   aagtttgatg aggatgactc tgagccagtt ctcaagggtg tcaaattaca ttacacataa 25260
   acgaacttat ggatttgttt atgagatttt ttactcttgg atcaattact gcacagccag 25320
   taaaaattga caatgcttct cctgcaagta ctgttcatgc tacagcaacg ataccgctac 25380
   aagcctcact ccctttcgga tggcttgtta ttggcgttgc atttcttgct gtttttcaga 25440
   gcgctaccaa aataattgcg ctcaataaaa gatggcagct agccctttat aagggcttcc 25500
   agttcatttg caatttactg ctgctatttg ttaccatcta ttcacatctt ttgcttgtcg 25560
   ctgcaggtat ggaggcgcaa tttttgtacc tctatgcctt gatatatttt ctacaatgca 25620
   tcaacgcatg tagaattatt atgagatgtt ggctttgttg gaagtgcaaa tccaagaacc 25680
   cattacttta tgatgccaac tactttgttt gctggcacac acataactat gactactgta 25740
   taccatataa cagtgtcaca gatacaattg tcgttactga aggtgacggc atttcaacac 25800
   caaaactcaa agaagactac caaattggtg gttattctga ggataggcac tcaggtgtta 25860
   aagactatgt cgttgtacat ggctatttca ccgaagttta ctaccagctt gagtctacac 25920
   aaattactac agacactggt attgaaaatg ctacattctt catctttaac aagcttgtta 25980
   aagacccacc gaatgtgcaa atacacacaa tcgacggctc ttcaggagtt gctaatccag 26040
   caatggatcc aatttatgat gagccgacga cgactactag cgtgcctttg taagcacaag 26100
   aaagtgagta cgaacttatg tactcattcg tttcggaaga aacaggtacg ttaatagtta 26160
   atagcgtact tctttttctt gctttcgtgg tattcttgct agtcacacta gccatcctta 26220
   ctgcgcttcg attgtgtgcg tactgctgca atattgttaa cgtgagttta gtaaaaccaa 26280
   cggtttacgt ctactcgcgt gttaaaaatc tgaactcttc tgaaggagtt cctgatcttc 26340
   tggtctaaac gaactaacta ttattattat tctgtttgga actttaacat tgcttatcat 26400
   ggcagacaac ggtactatta ccgttgagga gcttaaacaa ctcctggaac aatggaacct 26460
   agtaataggt ttcctattcc tagcctggat tatgttacta caatttgcct attctaatcg 26520
   gaacaggttt ttgtacataa taaagcttgt tttcctctgg ctcttgtggc cagtaacact 26580
   tgcttgtttt gtgcttgctg ctgtctacag aattaattgg gtgactggcg ggattgcgat 26640
   tgcaatggct tgtattgtag gcttgatgtg gcttagctac ttcgttgctt ccttcaggct 26700
   gtttgctcgt acccgctcaa tgtggtcatt caacccagaa acaaacattc ttctcaatgt 26760
   gcctctccgg gggacaattg tgaccagacc gctcatggaa agtgaacttg tcattggtgc 26820
   tgtgatcatt cgtggtcact tgcgaatggc cggacacccc ctagggcgct gtgacattaa 26880
   ggacctgcca aaagagatca ctgtggctac atcacgaacg ctttcttatt acaaattagg 26940
   agcgtcgcag cgtgtaggca ctgattcagg ttttgctgca tacaaccgct accgtattgg 27000
   aaactataaa ttaaatacag accacgccgg tagcaacgac aatattgctt tgctagtaca 27060
   gtaagtgaca acagatgttt catcttgttg acttccaggt tacaatagca gagatattga 27120
   ttatcattat gaggactttc aggattgcta tttggaatct tgacgttata ataagttcaa 27180
   tagtgagaca attatttaag cctctaacta agaagaatta ttcggagtta gatgatgaag 27240
   aacctatgga gttagattat ccataaaacg aacatgaaaa ttattctctt cctgacattg 27300
   attgtattta catcttgcga gctatatcac tatcaggagt gtgttagagg tacgactgta 27360
   ctactaaaag aaccttgccc atcaggaaca tacgagggca attcaccatt tcaccctctt 27420
   gctgacaata aatttgcact aacttgcact agcacacact ttgcttttgc ttgtgctgac 27480
   ggtactcgac atacctatca gctgcgtgca agatcagttt caccaaaact tttcatcaga 27540
   caagaggagg ttcaacaaga gctctactcg ccactttttc tcattgttgc tgctctagta 27600
   tttttaatac tttgcttcac cattaagaga aagacagaat gaatgagctc actttaattg 27660
   acttctattt gtgcttttta gcctttctgc tattccttgt tttaataatg cttattatat 27720
   tttggttttc actcgaaatc caggatctag aagaaccttg taccaaagtc taaacgaaca 27780
   tgaaacttct cattgttttg acttgtattt ctctatgcag ttgcatatgc actgtagtac 27840
   agcgctgtgc atctaataaa cctcatgtgc ttgaagatcc ttgtaaggta caacactagg 27900
   ggtaatactt atagcactgc ttggctttgt gctctaggaa aggttttacc ttttcataga 27960
   tggcacacta tggttcaaac atgcacacct aatgttacta tcaactgtca agatccagct 28020
   ggtggtgcgc ttatagctag gtgttggtac cttcatgaag gtcaccaaac tgctgcattt 28080
   agagacgtac ttgttgtttt aaataaacga acaaattaaa atgtctgata atggacccca 28140
   atcaaaccaa cgtagtgccc cccgcattac atttggtgga cccacagatt caactgacaa 28200
   taaccagaat ggaggacgca atggggcaag gccaaaacag cgccgacccc aaggtttacc 28260
   caataatact gcgtcttggt tcacagctct cactcagcat ggcaaggagg aacttagatt 28320
   ccctcgaggc cagggcgttc caatcaacac caatagtggt ccagatgacc aaattggcta 28380
   ctaccgaaga gctacccgac gagttcgtgg tggtgacggc aaaatgaaag agctcagccc 28440
   cagatggtac ttctattacc taggaactgg cccagaagct tcacttccct acggcgctaa 28500
   caaagaaggc atcgtatggg ttgcaactga gggagccttg aatacaccca aagaccacat 28560
   tggcacccgc aatcctaata acaatgctgc caccgtgcta caacttcctc aaggaacaac 28620
   attgccaaaa ggcttctacg cagagggaag cagaggcggc agtcaagcct cttctcgctc 28680
   ctcatcacgt agtcgcggta attcaagaaa ttcaactcct ggcagcagta ggggaaattc 28740
   tcctgctcga atggctagcg gaggtggtga aactgccctc gcgctattgc tgctagacag 28800
   attgaaccag cttgagagca aagtttctgg taaaggccaa caacaacaag gccaaactgt 28860
   cactaagaaa tctgctgctg aggcatctaa aaagcctcgc caaaaacgta ctgccacaaa 28920
   acagtacaac gtcactcaag catttgggag acgtggtcca gaacaaaccc aaggaaattt 28980
   cggggaccaa gacctaatca gacaaggaac tgattacaaa cattggccgc aaattgcaca 29040
   atttgctcca agtgcctctg cattctttgg aatgtcacgc attggcatgg aagtcacacc 29100
   ttcgggaaca tggctgactt atcatggagc cattaaattg gatgacaaag atccacaatt 29160
   caaagacaac gtcatactgc tgaacaagca cattgacgca tacaaaacat tcccaccaac 29220
   agagcctaaa aaggacaaaa agaaaaagac tgatgaagct cagcctttgc cgcagagaca 29280
   aaagaagcag cccactgtga ctcttcttcc tgcggctgac atggatgatt tctccagaca 29340
   acttcaaaat tccatgagtg gagcttctgc tgattcaact caggcataaa cactcatgat 29400
   gaccacacaa ggcagatggg ctatgtaaac gttttcgcaa ttccgtttac gatacatagt 29460
   ctactcttgt gcagaatgaa ttctcgtaac taaacagcac aagtaggttt agttaacttt 29520
   aatctcacat agcaatcttt aatcaatgtg taacattagg gaggacttga aagagccacc 29580
   acattttcat cgaggccacg cggagtacga tcgagggtac agtgaataat gctagggaga 29640
   gctgcctata tggaagagcc ctaatgtgta aaattaattt tagtagtgct atccccatgt 29700
   gattttaata gcttcttagg agaatgacaa aaaaaaaaaa aaaaaaaaaa aaagggtcgg 29760
   catggcatct ccacctcctc gcggtccgac ctgggcatcc gaaggaggac gtcgtccact 29820
   cggatggcta agggagagct cggatcgatc cgcctcgact gtgccttcta gttgccagcc 29880
   atctgttgtt tgcccctccc ccgtgccttc cttgaccctg gaaggtgcca ctcccactgt 29940
   cctttcctaa taaaatgagg aaattgcatc gcattgtctg agtaggtgtc attctattct 30000
   ggggggtggg gtggggcagg acagcaaggg ggaggattgg gaagacaata gcaggcatgc 30060
   tggggagagc tcgagctctg tacatgtccg cggaagcttg agtattctat agtgtcacct 30120
   aaatagcttg gcgtaatcat ggtcatagct gtttcctgtg tgaaattgtt atccgctcac 30180
   aattccacac aacatacgag ccggaagcat aaagtgtaaa gcctggggtg cctaatgagt 30240
   gagctaactc acattaattg cgttgcgctc actgcccgct ttccagtcgg gaaacctgtc 30300
   gtgccagctg cattaatgaa tcggccaacg cgaacccctt gcggccgccc gggccgtcga 30360
   ccaattctca tgtttgacag cttatcatcg aatttctgcc attcatccgc ttattatcac 30420
   ttattcaggc gtagcaacca ggcgtttaag ggcaccaata actgccttaa aaaaattacg 30480
   ccccgccctg ccactcatcg cagtactgtt gtaattcatt aagcattctg ccgacatgga 30540
   agccatcaca aacggcatga tgaacctgaa tcgccagcgg catcagcacc ttgtcgcctt 30600
   gcgtataata tttgcccatg gtgaaaacgg gggcgaagaa gttgtccata ttggccacgt 30660
   ttaaatcaaa actggtgaaa ctcacccagg gattggctga gacgaaaaac atattctcaa 30720
   taaacccttt agggaaatag gccaggtttt caccgtaaca cgccacatct tgcgaatata 30780
   tgtgtagaaa ctgccggaaa tcgtcgtggt attcactcca gagcgatgaa aacgtttcag 30840
   tttgctcatg gaaaacggtg taacaagggt gaacactatc ccatatcacc agctcaccgt 30900
   ctttcattgc catacggaat tccggatgag cattcatcag gcgggcaaga atgtgaataa 30960
   aggccggata aaacttgtgc ttatttttct ttacggtctt taaaaaggcc gtaatatcca 31020
   gctgaacggt ctggttatag gtacattgag caactgactg aaatgcctca aaatgttctt 31080
   tacgatgcca ttgggatata tcaacggtgg tatatccagt gatttttttc tccattttag 31140
   cttccttagc tcctgaaaat ctcgataact caaaaaatac gcccggtagt gatcttattt 31200
   cattatggtg aaagttggaa cctcttacgt gccgatcaac gtctcatttt cgccaaaagt 31260
   tggcccaggg cttcccggta tcaacaggga caccaggatt tatttattct gcgaagtgat 31320
   cttccgtcac aggtatttat tcgcgataag ctcatggagc ggcgtaaccg tcgcacagga 31380
   aggacagaga aagcgcggat ctgggaagtg acggacagaa cggtcaggac ctggattggg 31440
   gaggcggttg ccgccgctgc tgctgacggt gtgacgttct ctgttccggt cacaccacat 31500
   acgttccgcc attcctatgc gatgcacatg ctgtatgccg gtataccgct gaaagttctg 31560
   caaagcctga tgggacataa gtccatcagt tcaacggaag tctacacgaa ggtttttgcg 31620
   ctggatgtgg ctgcccggca ccgggtgcag tttgcgatgc cggagtctga tgcggttgcg 31680
   atgctgaaac aattatcctg agaataaatg ccttggcctt tatatggaaa tgtggaactg 31740
   agtggatatg ctgtttttgt ctgttaaaca gagaagctgg ctgttatcca ctgagaagcg 31800
   aacgaaacag tcgggaaaat ctcccattat cgtagagatc cgcattatta atctcaggag 31860
   cctgtgtagc gtttatagga agtagtgttc tgtcatgatg cctgcaagcg gtaacgaaaa 31920
   cgatttgaat atgccttcag gaacaataga aatcttcgtg cggtgttacg ttgaagtgga 31980
   gcggattatg tcagcaatgg acagaacaac ctaatgaaca cagaaccatg atgtggtctg 32040
   tccttttaca gccagtagtg ctcgccgcag tcgagcgaca gggcgaagcc ctcgagtgag 32100
   cgaggaagca ccagggaaca gcacttatat attctgctta cacacgatgc ctgaaaaaac 32160
   ttcccttggg gttatccact tatccacggg gatattttta taattatttt ttttatagtt 32220
   tttagatctt cttttttaga gcgccttgta ggcctttatc catgctggtt ctagagaagg 32280
   tgttgtgaca aattgccctt tcagtgtgac aaatcaccct caaatgacag tcctgtctgt 32340
   gacaaattgc ccttaaccct gtgacaaatt gccctcagaa gaagctgttt tttcacaaag 32400
   ttatccctgc ttattgactc ttttttattt agtgtgacaa tctaaaaact tgtcacactt 32460
   cacatggatc tgtcatggcg gaaacagcgg ttatcaatca caagaaacgt aaaaatagcc 32520
   cgcgaatcgt ccagtcaaac gacctcactg aggcggcata tagtctctcc cgggatcaaa 32580
   aacgtatgct gtatctgttc gttgaccaga tcagaaaatc tgatggcacc ctacaggaac 32640
   atgacggtat ctgcgagatc catgttgcta aatatgctga aatattcgga ttgacctctg 32700
   cggaagccag taaggatata cggcaggcat tgaagagttt cgcggggaag gaagtggttt 32760
   tttatcgccc tgaagaggat gccggcgatg aaaaaggcta tgaatctttt ccttggttta 32820
   tcaaacgtgc gcacagtcca tccagagggc tttacagtgt acatatcaac ccatatctca 32880
   ttcccttctt tatcgggtta cagaaccggt ttacgcagtt tcggcttagt gaaacaaaag 32940
   aaatcaccaa tccgtatgcc atgcgtttat acgaatccct gtgtcagtat cgtaagccgg 33000
   atggctcagg catcgtctct ctgaaaatcg actggatcat agagcgttac cagctgcctc 33060
   aaagttacca gcgtatgcct gacttccgcc gccgcttcct gcaggtctgt gttaatgaga 33120
   tcaacagcag aactccaatg cgcctctcat acattgagaa aaagaaaggc cgccagacga 33180
   ctcatatcgt attttccttc cgcgatatca cttccatgac gacaggatag tctgagggtt 33240
   atctgtcaca gatttgaggg tggttcgtca catttgttct gacctactga gggtaatttg 33300
   tcacagtttt gctgtttcct tcagcctgca tggattttct catacttttt gaactgtaat 33360
   ttttaaggaa gccaaatttg agggcagttt gtcacagttg atttccttct ctttcccttc 33420
   gtcatgtgac ctgatatcgg gggttagttc gtcatcattg atgagggttg attatcacag 33480
   tttattactc tgaattggct atccgcgtgt gtacctctac ctggagtttt tcccacggtg 33540
   gatatttctt cttgcgctga gcgtaagagc tatctgacag aacagttctt ctttgcttcc 33600
   tcgccagttc gctcgctatg ctcggttaca cggctgcggc gagcgctagt gataataagt 33660
   gactgaggta tgtgctcttc ttatctcctt ttgtagtgtt gctcttattt taaacaactt 33720
   tgcggttttt tgatgacttt gcgattttgt tgttgctttg cagtaaattg caagatttaa 33780
   taaaaaaacg caaagcaatg attaaaggat gttcagaatg aaactcatgg aaacacttaa 33840
   ccagtgcata aacgctggtc atgaaatgac gaaggctatc gccattgcac agtttaatga 33900
   tgacagcccg gaagcgagga aaataacccg gcgctggaga ataggtgaag cagcggattt 33960
   agttggggtt tcttctcagg ctatcagaga tgccgagaaa gcagggcgac taccgcaccc 34020
   ggatatggaa attcgaggac gggttgagca acgtgttggt tatacaattg aacaaattaa 34080
   tcatatgcgt gatgtgtttg gtacgcgatt gcgacgtgct gaagacgtat ttccaccggt 34140
   gatcggggtt gctgcccata aaggtggcgt ttacaaaacc tcagtttctg ttcatcttgc 34200
   tcaggatctg gctctgaagg ggctacgtgt tttgctcgtg gaaggtaacg acccccaggg 34260
   aacagcctca atgtatcacg gatgggtacc agatcttcat attcatgcag aagacactct 34320
   cctgcctttc tatcttgggg aaaaggacga tgtcacttat gcaataaagc ccacttgctg 34380
   gccggggctt gacattattc cttcctgtct ggctctgcac cgtattgaaa ctgagttaat 34440
   gggcaaattt gatgaaggta aactgcccac cgatccacac ctgatgctcc gactggccat 34500
   tgaaactgtt gctcatgact atgatgtcat agttattgac agcgcgccta acctgggtat 34560
   cggcacgatt aatgtcgtat gtgctgctga tgtgctgatt gttcccacgc ctgctgagtt 34620
   gtttgactac acctccgcac tgcagttttt cgatatgctt cgtgatctgc tcaagaacgt 34680
   tgatcttaaa gggttcgagc ctgatgtacg tattttgctt accaaataca gcaatagtaa 34740
   tggctctcag tccccgtgga tggaggagca aattcgggat gcctggggaa gcatggttct 34800
   aaaaaatgtt gtacgtgaaa cggatgaagt tggtaaaggt cagatccgga tgagaactgt 34860
   ttttgaacag gccattgatc aacgctcttc aactggtgcc tggagaaatg ctctttctat 34920
   ttgggaacct gtctgcaatg aaattttcga tcgtctgatt aaaccacgct gggagattag 34980
   ataatgaagc gtgcgcctgt tattccaaaa catacgctca atactcaacc ggttgaagat 35040
   acttcgttat cgacaccagc tgccccgatg gtggattcgt taattgcgcg cgtaggagta 35100
   atggctcgcg gtaatgccat tactttgcct gtatgtggtc gggatgtgaa gtttactctt 35160
   gaagtgctcc ggggtgatag tgttgagaag acctctcggg tatggtcagg taatgaacgt 35220
   gaccaggagc tgcttactga ggacgcactg gatgatctca tcccttcttt tctactgact 35280
   ggtcaacaga caccggcgtt cggtcgaaga gtatctggtg tcatagaaat tgccgatggg 35340
   agtcgccgtc gtaaagctgc tgcacttacc gaaagtgatt atcgtgttct ggttggcgag 35400
   ctggatgatg agcagatggc tgcattatcc agattgggta acgattatcg cccaacaagt 35460
   gcttatgaac gtggtcagcg ttatgcaagc cgattgcaga atgaatttgc tggaaatatt 35520
   tctgcgctgg ctgatgcgga aaatatttca cgtaagatta ttacccgctg tatcaacacc 35580
   gccaaattgc ctaaatcagt tgttgctctt ttttctcacc ccggtgaact atctgcccgg 35640
   tcaggtgatg cacttcaaaa agcctttaca gataaagagg aattacttaa gcagcaggca 35700
   tctaaccttc atgagcagaa aaaagctggg gtgatatttg aagctgaaga agttatcact 35760
   cttttaactt ctgtgcttaa aacgtcatct gcatcaagaa ctagtttaag ctcacgacat 35820
   cagtttgctc ctggagcgac agtattgtat aagggcgata aaatggtgct taacctggac 35880
   aggtctcgtg ttccaactga gtgtatagag aaaattgagg ccattcttaa ggaacttgaa 35940
   aagccagcac cctgatgcga ccacgtttta gtctacgttt atctgtcttt acttaatgtc 36000
   ctttgttaca ggccagaaag cataactggc ctgaatattc tctctgggcc cactgttcca 36060
   cttgtatcgt cggtctgata atcagactgg gaccacggtc ccactcgtat cgtcggtctg 36120
   attattagtc tgggaccacg gtcccactcg tatcgtcggt ctgattatta gtctgggacc 36180
   acggtcccac tcgtatcgtc ggtctgataa tcagactggg accacggtcc cactcgtatc 36240
   gtcggtctga ttattagtct gggaccatgg tcccactcgt atcgtcggtc tgattattag 36300
   tctgggacca cggtcccact cgtatcgtcg gtctgattat tagtctggaa ccacggtccc 36360
   actcgtatcg tcggtctgat tattagtctg ggaccacggt cccactcgta tcgtcggtct 36420
   gattattagt ctgggaccac gatcccactc gtgttgtcgg tctgattatc ggtctgggac 36480
   cacggtccca cttgtattgt cgatcagact atcagcgtga gactacgatt ccatcaatgc 36540
   ctgtcaaggg caagtattga catgtcgtcg taacctgtag aacggagtaa cctcggtgtg 36600
   cggttgtatg cctgctgtgg attgctgctg tgtcctgctt atccacaaca ttttgcgcac 36660
   ggttatgtgg acaaaatacc tggttaccca ggccgtgccg gcacgttaac cgggctgcat 36720
   ccgatgcaag tgtgtcgctg tcgacgagct cgcgagctcg gacatgaggt tgccccgtat 36780
   tcagtgtcgc tgatttgtat tgtctgaagt tgtttttacg ttaagttgat gcagatcaat 36840
   taatacgata cctgcgtcat aattgattat ttgacgtggt ttgatggcct ccacgcacgt 36900
   tgtgatatgt agatgataat cattatcact ttacgggtcc tttccggtga tccgacaggt 36960
   tacggggcgg cgacctcgcg ggttttcgct atttatgaaa attttccggt ttaaggcgtt 37020
   tccgttcttc ttcgtcataa cttaatgttt ttatttaaaa taccctctga aaagaaagga 37080
   aacgacaggt gctgaaagcg agctttttgg cctctgtcgt ttcctttctc tgtttttgtc 37140
   cgtggaatga acaatggaag tccgagctca tcgctaataa cttcgtatag catacattat 37200
   acgaagttat attcgatgcg gccgcaaggg gttcgcgtca gcgggtgttg gcgggtgtcg 37260
   gggctggctt aactatgcgg catcagagca gattgtactg agagtgcacc atatgcggtg 37320
   tgaaataccg cacagatgcg taaggagaaa ataccgcatc aggcgcctaa gttgacattg 37380
   attattgact agttattaat agtaatcaat tacggggtca ttagttcata gcccatatat 37440
   ggagttccgc gttacataac ttacggtaaa tggcccgcct ggctgaccgc ccaacgaccc 37500
   ccgcccattg acgtcaataa tgacgtatgt tcccatagta acgccaatag ggactttcca 37560
   ttgacgtcaa tgggtggagt atttacggta aactgcccac ttggcagtac atcaagtgta 37620
   tcatatgcca agtacgcccc ctattgacgt caatgacggt aaatggcccg cctggcatta 37680
   tgcccagtac atgaccttat gggactttcc tacttggcag tacatctacg tattagtcat 37740
   cgctattacc atggtgatgc ggttttggca gtacatcaat gggcgtggat agcggtttga 37800
   ctcacgggga tttccaagtc tccaccccat tgacgtcaat gggagtttgt tttggcacca 37860
   aaatcaacgg gactttccaa aatgtcgtaa caactccgcc ccattgacgc aaatgggcgg 37920
   taggcgtgta cggtgggagg tctatataag cagagctcgt ttagtgaacc t 37971

<210> 12
<211> 825
<212> DNA
<213> SARS-CoV

<400> 12
atggatttgt ttatgagatt ttttactctt ggatcaatta ctgcacagcc agtaaaaatt 60

gacaatgctt ctcctgcaag tactgttcat gctacagcaa cgataccgct acaagcctca 120

ctccctttcg gatggcttgt tattggcgtt gcatttcttg ctgtttttca gagcgctacc 180

aaaataattg cgctcaataa aagatggcag ctagcccttt ataagggctt ccagttcatt 240

tgcaatttac tgctgctatt tgttaccatc tattcacatc ttttgcttgt cgctgcaggt 300

atggaggcgc aatttttgta cctctatgcc ttgatatatt ttctacaatg catcaacgca 360

tgtagaatta ttatgagatg ttggctttgt tggaagtgca aatccaagaa cccattactt 420

tatgatgcca actactttgt ttgctggcac acacataact atgactactg tataccatat 480

aacagtgtca cagatacaat tgtcgttact gaaggtgacg gcatttcaac accaaaactc 540

aaagaagact accaaattgg tggttattct gaggataggc actcaggtgt taaagactat 600

gtcgttgtac atggctattt caccgaagtt tactaccagc ttgagtctac acaaattact 660

acagacactg gtattgaaaa tgctacattc ttcatcttta acaagcttgt taaagaccca 720

ccgaatgtgc aaatacacac aatcgacggc tcttcaggag ttgctaatcc agcaatggat 780

ccaatttatg atgagccgac gacgactact agcgtgcctt tgtaa 825

<210> 13
<211> 465
<212> DNA
<213> SARS-CoV

<400> 13
atgatgccaa ctactttgtt tgctggcaca cacataacta tgactactgt ataccatata 60

acagtgtcac agatacaatt gtcgttactg aaggtgacgg catttcaaca ccaaaactca 120

aagaagacta ccaaattggt ggttattctg aggataggca ctcaggtgtt aaagactatg 180

tcgttgtaca tggctatttc accgaagttt actaccagct tgagtctaca caaattacta 240

cagacactgg tattgaaaat gctacattct tcatctttaa caagcttgtt aaagacccac 300

cgaatgtgca aatacacaca atcgacggct cttcaggagt tgctaatcca gcaatggatc 360

caatttatga tgagccgacg acgactacta gcgtgccttt gtaagcacaa gaaagtgagt 420

acgaacttat gtactcattc gtttcggaag aaacaggtac gttaa 465

<210> 14
<211> 192
<212> DNA
<213> SARS-CoV

<400> 14
atgtttcatc ttgttgactt ccaggttaca atagcagaga tattgattat cattatgagg 60

actttcagga ttgctatttg gaatcttgac gttataataa gttcaatagt gagacaatta 120

tttaagcctc taactaagaa gaattattcg gagttagatg atgaagaacc tatggagtta 180

gattatccat aa 192

<210> 15
<211> 369
<212> DNA
<213> SARS-CoV

<400> 15
atgaaaatta ttctcttcct gacattgatt gtatttacat cttgcgagct atatcactat 60

caggagtgtg ttagaggtac gactgtacta ctaaaagaac cttgcccatc aggaacatac 120

gagggcaatt caccatttca ccctcttgct gacaataaat ttgcactaac ttgcactagc 180

acacactttg cttttgcttg tgctgacggt actcgacata cctatcagct gcgtgcaaga 240

tcagtttcac caaaactttt catcagacaa gaggaggttc aacaagagct ctactcgcca 300

ctttttctca ttgttgctgc tctagtattt ttaatacttt gcttcaccat taagagaaag 360

acagaatga 369

<210> 16
<211> 135
<212> DNA
<213> SARS-CoV

<400> 16
atgaatgagc tcactttaat tgacttctat ttgtgctttt tagcctttct gctattcctt 60

gttttaataa tgcttattat attttggttt tcactcgaaa tccaggatct agaagaacct 120

tgtaccaaag tctaa 135

<210> 17
<211> 120
<212> DNA
<213> SARS-CoV

<400> 17
atgaaacttc tcattgtttt gacttgtatt tctctatgca gttgcatatg cactgtagta 60

cagcgctgtg catctaataa acctcatgtg cttgaagatc cttgtaaggt acaacactag 120

<210>  18
<211>  255
<212>  DNA
<213>  SARS-CoV

<400>  18
atgtgcttga agatccttgt aaggtacaac actaggggta atacttatag cactgcttgg      60

ctttgtgctc taggaaaggt tttacctttt catagatggc acactatggt tcaaacatgc     120

acacctaatg ttactatcaa ctgtcaagat ccagctggtg gtgcgcttat agctaggtgt     180

tggtaccttc atgaaggtca ccaaactgct gcatttagag acgtacttgt tgttttaaat     240

aaacgaacaa attaa                                                      255


<210>  19
<211>  297
<212>  DNA
<213>  SARS-CoV

<400>  19
atggacccca atcaaaccaa cgtagtgccc cccgcattac atttggtgga cccacagatt      60

caactgacaa taaccagaat ggaggacgca atggggcaag gccaaaacag cgccgacccc     120

aaggtttacc caataatact gcgtcttggt tcacagctct cactcagcat ggcaaggagg     180

aacttagatt ccctcgaggc cagggcgttc caatcaacac caatagtggt ccagatgacc     240

aaattggcta ctaccgaaga gctacccgac gagttcgtgg tggtgacggc aaaatga        297